# FASCICULE DE BREVET EUROPEEN

(11) **EP 2 268 816 B1**
(45) Date de publication et mention de la délivrance du brevet: **06.05.2015**
(21) Numéro de dépôt: 09721663.4
(22) Date de dépôt: 18.03.2009
(51) Int. Cl.: C12N 15/62

(54) **POLYNUCLÉOTIDES ET POLYPEPTIDES CHIMÉRIQUES PERMETTANT LA SÉCRÉTION D'UN POLYPEPTIDE D'INTÉRÊT EN ASSOCIATION AVEC DES EXOSOMES ET LEUR UTILISATION POUR LA PRODUCTION DE COMPOSITIONS IMMUNOGÈNES**
POLYNUKLEOTIDE UND POLYPEPTIDCHIMÄREN ZUR FREISETZUNG EINES INTERESSIERENDEN POLYPEPTIDS IN KOMBINATION MIT EXOSOMEN UND VERWENDUNG DAVON ZUR HERSTELLUNG IMMUNOGENER ZUSAMMENSETZUNGEN
POLYNUCLEOTIDES AND CHIMAERA POLYPEPTIDES FOR RELEASING A POLYPEPTIDE OF INTEREST COMBINED WITH EXOSOMES AND USE THEREOF FOR THE PRODUCTION OF IMMUNOGENIC COMPOSITIONS

(30) Priorité: 18.03.2008 FR 0801486
(43) Date de publication de la demande: 05.01.2011
(73) Titulaire: CENTRE NATIONAL DE LA RECHERCHE SCIENTIFIQUE -CNRS-, 75794 Paris Cedex 16 (FR); UNIVERSITE MONTPELLIER II SCIENCES ET TECHNIQUES DU LANGUEDOC, F-34095 Montpellier Cédex (FR)
(72) Inventeur: MAMOUN, Robert, Zaine El Abiddine, F-34725 St André de Sangonis (FR); TRENTIN, Bernadette, Nadine, F-34725 St André de Sangonis (FR); VIDAL, Michel, F-34990 Juvignac (FR)
(74) Mandataire: Desaix, Anne
(86) Numéro de dépôt international: PCT/EP2009/053221
(87) Numéro de publication internationale: WO 2009/115561

(56) Documents cités:
- WO-A-03/016522
- WO-A-03/076603
- WO-A-2004/073319
- NOVAKOVIC SINISA ET AL: "Dileucine and YXXL motifs in the cytoplasmic tail of the bovine leukemia virus transmembrane envelope protein affect protein expression on the cell surface" JOURNAL OF VIROLOGY, vol. 78, no. 15, août 2004 (2004-08), pages 8301-8311, XP002508133 ISSN: 0022-538X
- ZEELENBERG INGRID S ET AL: "Targeting tumor antigens to secreted membrane vesicles in vivo induces efficient antitumor immune responses" CANCER RESEARCH, vol. 68, no. 4, février 2008 (2008-02), pages 1228-1235, XP002508134 ISSN: 0008-5472
- DELCAYRE ET AL: "Exosome Display technology: Applications to the development of new diagnostics and therapeutics" BLOOD CELLS, MOLECULES AND DISEASES, LAJOLLA, US, vol. 35, no. 2, 1 septembre 2005 (2005-09-01), pages 158-168, XP005067367 ISSN: 1079-9796
- KUATE ET AL: "Exosomal vaccines containing the S protein of the SARS coronavirus induce high levels of neutralizing antibodies" VIROLOGY, ACADEMIC PRESS,ORLANDO, US, vol. 362, no. 1, 25 avril 2007 (2007-04-25), pages 26-37, XP022046488 ISSN: 0042-6822
- ESTELLES ANGELES ET AL: "Exosome nanovesicles displaying G protein-coupled receptors for drug discovery." INTERNATIONAL JOURNAL OF NANOMEDICINE 2007, vol. 2, no. 4, 2007, pages 751-760, XP008099773 ISSN: 1176-9114
- DE GASSART AUDE ET AL: "Exosomal sorting of the cytoplasmic domain of bovine leukemia virus TM Env protein." CELL BIOLOGY INTERNATIONAL JAN 2009, vol. 33, no. 1, janvier 2009 (2009-01), pages 36-48, XP002530202 ISSN: 1095-8355
- Nicolas Blanchard ET AL: "TCR activation of human T cells induces the production of exosomes bearing the TCR/CD3/zeta complex.", The Journal of Immunology, vol. 168, no. 7, 1 April 2002 (2002-04-01) , pages 3235-3241, XP055057390, ISSN: 0022-1767
- ADMYRE ET AL: "B cell-derived exosomes can present allergen peptides and activate allergen-specific T cells to proliferate and produce TH2-like cytokines", JOURNAL OF ALLERGY AND CLINICAL IMMUNOLOGY, ELSEVIER, AMSTERDAM, NL, vol. 120, no. 6, 1 December 2007 (2007-12-01), pages 1418-1424, XP022383439, ISSN: 0091-6749, DOI: 10.1016/J.JACI.2007.06.040
- MALLEGOL J ET AL: "T84-Intestinal Epithelial Exosomes Bear MHC Class II/Peptide Complexes Potentiating Antigen Presentation by Dendritic Cells", GASTROENTEROLOGY, ELSEVIER, PHILADELPHIA, PA, vol. 132, no. 5, 1 May 2007 (2007-05-01), pages 1866-1876, XP026856771, ISSN: 0016-5085 [retrieved on 2007-05-05]
- ZHAO ET AL: "Natural genetic variations in bovine leukemia virus envelope gene: Possible effects of selection and escape", VIROLOGY, ELSEVIER, AMSTERDAM, NL, vol. 366, no. 1, 29 August 2007 (2007-08-29), pages 150-165, XP022232351, ISSN: 0042-6822, DOI: 10.1016/J.VIROL.2007.03.058

### SEQUENCE LISTING

<110> CENTRE NATIONAL DE LA RECHERCHE SCIENTIFIQUE
<120> Polynucléotides et polypeptides chimériques permettant la sécrétion d'un polypeptide d'intérêt en association avec des exosomes et leur utilisation pour la production de compositions immunogènes
<130> B07452A_AD/SL/CAL
<140> inconnu
   <141> 2009-03-18
<150> FR 08/01486
   <151> 2008-03-18
<160> 103
<170> PatentIn version 3.3
<210> 1
   <211> 81
   <212> DNA
   <213> Mus musculus
<220>
   <221> CDS
   <222> (1)..(81)
<220>
   <221> misc_feature
   <222> (1)..(81)
   <223> Peptide signal d'importation dans le réticulum endoplasmique de la protéine CD8 alpha
<400> 1
   atg gcc tca ccg ttg acc cgc ttt ctg tcg ctg aac ctg ctg ctg ctg      48
   Met Ala Ser Pro Leu Thr Arg Phe Leu Ser Leu Asn Leu Leu Leu Leu
   1               5                   10                  15
   ggt gag tcg att atc ctg ggg agt gga gaa gct                          81
   Gly Glu Ser Ile Ile Leu Gly Ser Gly Glu Ala
               20                  25
<210> 2
   <211> 27
   <212> PRT
   <213> Mus musculus
<400> 2
   Met Ala Ser Pro Leu Thr Arg Phe Leu Ser Leu Asn Leu Leu Leu Leu
   1               5                   10                  15
   Gly Glu Ser Ile Ile Leu Gly Ser Gly Glu Ala
               20                  25
<210> 3
   <211> 69
   <212> DNA
   <213> Bovine leukemia virus
<220>
   <221> misc_feature
   <222> (1)..(69)
   <223> Domaine transmembranaire de la protéine TM du BLV (séquence sauvage)
<220>
   <221> CDS
   <222> (1)..(69)
<400> 3
   ctc att cat tct gtt cta agc cta ttc cta tta gcc ctt ttt ttg ctc       48
   Leu Ile His Ser Val Leu Ser Leu Phe Leu Leu Ala Leu Phe Leu Leu
   1               5                   10                  15
   ttc ttg gcc ccc tgc ctg ata                                           69
   Phe Leu Ala Pro Cys Leu Ile
               20
<210> 4
   <211> 23
   <212> PRT
   <213> Bovine leukemia virus
<400> 4
   Leu Ile His Ser Val Leu Ser Leu Phe Leu Leu Ala Leu Phe Leu Leu
   1               5                   10                  15
   Phe Leu Ala Pro Cys Leu Ile
               20
<210> 5
   <211> 177
   <212> DNA
   <213> Bovine leukemia virus
<220>
   <221> misc_feature
   <222> (1)..(177)
   <223> Domaine cytoplasmique (CD) sauvage de la protéine TM
<220>
   <221> CDS
   <222> (1)..(177)
<220>
   <221> misc_feature
   <222> (42)..(43)
   <223> n = t ou a
<400> 5
   aaa tgc ttg acc tct cgc ctt tta aaa ctc ctc cgg cag gcn ccc cac     48
   Lys Cys Leu Thr Ser Arg Leu Leu Lys Leu Leu Arg Gln Ala Pro His
   1               5                   10                  15
   ttc cct gaa atc tcc ttc ccc cct aaa ccc gat tct gat tat cag gcc     96
   Phe Pro Glu Ile Ser Phe Pro Pro Lys Pro Asp Ser Asp Tyr Gln Ala
               20                  25                  30
   ttg cta cca tcc gcg cca gag atc tac tct cac ctc tcc ccc acc aaa     144
   Leu Leu Pro Ser Ala Pro Glu Ile Tyr Ser His Leu Ser Pro Thr Lys
           35                  40                  45
   ccc gat tac atc aac ctt cga ccc tgc ccc tag                         177
   Pro Asp Tyr Ile Asn Leu Arg Pro Cys Pro
                       50                  55
<210> 6
   <211> 58
   <212> PRT
   <213> Bovine leukemia virus
<400> 6
   Lys Cys Leu Thr Ser Arg Leu Leu Lys Leu Leu Arg Gln Ala Pro His
   1               5                   10                  15
   Phe Pro Glu Ile Ser Phe Pro Pro Lys Pro Asp Ser Asp Tyr Gln Ala
               20                  25                  30
   Leu Leu Pro Ser Ala Pro Glu Ile Tyr Ser His Leu Ser Pro Thr Lys
           35                  40                  45
   Pro Asp Tyr Ile Asn Leu Arg Pro Cys Pro
       50                  55
<210> 7
   <211> 135
   <212> DNA
   <213> Artificial
<220>
   <223> polynucléotide
<220>
   <221> misc_feature
   <222> (1)..(135)
   <223> Dérivé muté du CD de la protéine TM du virus de la leucémie bovine (BLV) : délétion des 13 résidus N-terminaux
<220>
   <221> CDS
   <222> (1)..(135)
<400> 7
   gca ccc cac ttc cct gaa atc tcc ttc ccc cct aaa ccc gat tct gat       48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc tcc       96
   Tyr Gln Ala Leu Leu Pro Ser Ala Pro Glu Ile Tyr Ser His Leu Ser
               20                  25                  30
   ccc acc aaa ccc gat tac atc aac ctt cga ccc tgc ccc                  135
   Pro Thr Lys Pro Asp Tyr Ile Asn Leu Arg Pro Cys Pro
           35                  40                  45
<210> 8
   <211> 45
   <212> PRT
   <213> Artificial
<220>
   <223> Synthetic Construct
<400> 8
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp Tyr Ile Asn Leu Arg Pro Cys Pro
           35                  40                  45
<210> 9
   <211> 174
   <212> DNA
   <213> Artificial
<220>
   <223> polynucléotide
<220>
   <221> misc_feature
   <222> (1)..(174)
   <223> Dérivé muté du CD de la protéine TM du BLV : substitution du résidu C du motif PCT par un résidu A
<220>
   <221> CDS
   <222> (1)..(174)
<400> 9
   aaa tgc ttg acc tct cgc ctt tta aaa ctc ctc cgg cag gca ccc cac       48
   Lys Cys Leu Thr Ser Arg Leu Leu Lys Leu Leu Arg Gln Ala Pro His
   1               5                   10                  15
   ttc cct gaa atc tcc ttc ccc cct aaa ccc gat tct gat tat cag gcc       96
   Phe Pro Glu Ile Ser Phe Pro Pro Lys Pro Asp Ser Asp Tyr Gln Ala
               20                  25                  30
   ttg cta cca tcc gcg cca gag atc tac tct cac ctc tcc ccc acc aaa      144
   Leu Leu Pro Ser Ala Pro Glu Ile Tyr Ser His Leu Ser Pro Thr Lys
           35                  40                  45
   ccc gat tac atc aac ctt cga ccg gcg ccc                              174
   Pro Asp Tyr Ile Asn Leu Arg Pro Ala Pro
       50                  55
<210> 10
   <211> 58
   <212> PRT
   <213> Artificial
<220>
   <223> Synthetic Construct
<400> 10
   Lys Cys Leu Thr Ser Arg Leu Leu Lys Leu Leu Arg Gln Ala Pro His
   1               5                   10                  15
   Phe Pro Glu Ile Ser Phe Pro Pro Lys Pro Asp Ser Asp Tyr Gln Ala
               20                  25                  30
   Leu Leu Pro Ser Ala Pro Glu Ile Tyr Ser His Leu Ser Pro Thr Lys
           35                  40                  45
   Pro Asp Tyr Ile Asn Leu Arg Pro Ala Pro
       50                  55
<210> 11
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13 résidus N-terminaux et substitution du résidu C du motif PCP par un résidu A
<400> 11
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc tcc      96
   Tyr Gln Ala Leu Leu Pro Ser Ala Pro Glu Ile Tyr Ser His Leu Ser
               20                  25                  30
   ccc acc aaa ccc gat tac atc aac ctt cga ccg gcg ccc                  135
   Pro Thr Lys Pro Asp Tyr Ile Asn Leu Arg Pro Ala Pro
           35                  40                  45
<210> 12
   <211> 45
   <212> PRT
   <213> Artificial
<220>
   <223> Synthetic Construct
<400> 12
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp Tyr Ile Asn Leu Arg Pro Ala Pro
           35                  40                  45
<210> 13
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13 résidus N-terminaux et substitution des 2 résidus P du 1er motif PXXP
<400> 13
   gca gcc cac ttc gct gag ata tcc ttc ccc cct aaa ccc gat tct gat       48
   Ala Ala His Phe Ala Glu Ile Ser Phe Pro Pro Lys Pro Asp Ser Asp
   1               5                   10                  15
   tat cag gcc ttg cta cca tcc gcg cca gag atc tac tct cac ctc tcc       96
   Tyr Gln Ala Leu Leu Pro Ser Ala Pro Glu Ile Tyr Ser His Leu Ser
               20                  25                  30
   ccc acc aaa ccc gat tac atc aac ctt cga ccc tgc ccc                   135
   Pro Thr Lys Pro Asp Tyr Ile Asn Leu Arg Pro Cys Pro
           35                  40                  45
<210> 14
   <211> 45
   <212> PRT
   <213> Artificial
<220>
   <223> Synthetic Construct
<400> 14
   Ala Ala His Phe Ala Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp Tyr Ile Asn Leu Arg Pro Cys Pro
           35                  40                  45
<210> 15
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13 résidus N-terminaux et substitution des 2 résidus P du 2ème motif PxxP
<400> 15
   gca ccc cac ttc cct gag ata tcc ttc gcc cct aaa gcc gat tct gat        48
   Ala Pro His Phe Pro Glu Ile Ser Phe Ala Pro Lys Ala Asp Ser Asp
   1               5                   10                  15
   tat cag gcc ttg cta cca tcc gcg cca gag atc tac tct cac ctc tcc        96
   Tyr Gln Ala Leu Leu Pro Ser Ala Pro Glu Ile Tyr Ser His Leu Ser
               20                  25                  30
   ccc acc aaa ccc gat tac atc aac ctt cga ccc tgc ccc                    135
   Pro Thr Lys Pro Asp Tyr Ile Asn Leu Arg Pro Cys Pro
           35                  40                  45
<210> 16
   <211> 45
   <212> PRT
   <213> Artificial
<220>
   <223> Synthetic Construct
<400> 16
   Ala Pro His Phe Pro Glu Ile Ser Phe Ala Pro Lys Ala Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp Tyr Ile Asn Leu Arg Pro Cys Pro
           35                  40                  45
<210> 17
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13 résidus N-terminaux et substitution des 2 résidus proline du 3ème motif PxxP
<400> 17
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   tat cag gcc ttg cta gca tcg gcg gca gag att tac tct cac ctc tcc      96
   Tyr Gln Ala Leu Leu Ala Ser Ala Ala Glu Ile Tyr Ser His Leu Ser
               20                  25                  30
   ccc acc aaa ccc gat tac atc aac ctt cga ccc tgc ccc                  135
   Pro Thr Lys Pro Asp Tyr Ile Asn Leu Arg Pro Cys Pro
           35                  40                  45
<210> 18
   <211> 45
   <212> PRT
   <213> Artificial
<220>
   <223> Synthetic Construct
<400> 18
   Ala Pro His Phe Pro Glu Ile Ser Phe Pro Pro Lys Pro Asp Ser Asp
   1               5                   10                  15
   Tyr Gln Ala Leu Leu Ala Ser Ala Ala Glu Ile Tyr Ser His Leu Ser
               20                  25                  30
   Pro Thr Lys Pro Asp Tyr Ile Asn Leu Arg Pro Cys Pro
           35                  40                  45
<210> 19
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13 , résidus N-terminaux et substitution du 1er résidu P du 4ème motif PxxP
<400> 19
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   tat cag gcc ttg cta cca tcc gcg cca gag ata tac tct cac ctc tcc      96
   Tyr Gln Ala Leu Leu Pro Ser Ala Pro Glu Ile Tyr Ser His Leu Ser
               20                  25                  30
   gcc acc aaa ccc gat tac atc aac ctt cga ccc tgc ccc                  135
   Ala Thr Lys Pro Asp Tyr Ile Asn Leu Arg Pro Cys Pro
           35                  40                  45
<210> 20
   <211> 45
   <212> PRT
   <213> Artificial
<220>
   <223> Synthetic Construct
<400> 20
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Ala Thr Lys Pro Asp Tyr Ile Asn Leu Arg Pro Cys Pro
           35                  40                  45
<210> 21
   <211> 135
   <212> DNA
   <213> Artificial
<220>
   <223> polynucléotide
<220>
   <221> misc_feature
   <222> (1)..(135)
   <223> Dérivé muté du CD de la protéine TM du BLV : délétion des 13 résidus N-terminaux et substitution des 2 résidus P du 4ème motif PxxP
<220>
   <221> misc_feature
   <222> (1)..(135)
   <223> Dérivé muté du CD de la protéine TM du BLV : délétion des 13 résidus N-terminaux et substitution du résidu du 4ème motif C du motif PCP par un résidu A du 4ème motif PxxP
<220>
   <221> CDS
   <222> (1)..(135)
<400> 21
   gca ccc cac ttc cct gaa atc tcc ttc ccc cct aaa ccc gat tct gat        48
   Ala Pro His Phe Pro Glu Ile Ser Phe Pro Pro Lys Pro Asp Ser Asp
   1               5                   10                  15
   tat cag gcc ttg cta cca tcc gcg cca gag ata tac tct cac ctc tcc        96
   Tyr Gln Ala Leu Leu Pro Ser Ala Pro Glu Ile Tyr Ser His Leu Ser
               20                  25                  30
   gcc acc aaa gcc gat tac atc aac ctt cga ccc tgc ccc                    135
   Ala Thr Lys Ala Asp Tyr Ile Asn Leu Arg Pro Cys Pro
           35                  40                  45
<210> 22
   <211> 45
   <212> PRT
   <213> Artificial
<220>
   <223> Synthetic Construct
<400> 22
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Ala Thr Lys Ala Asp Tyr Ile Asn Leu Arg Pro Cys Pro
           35                  40                  45
<210> 23
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13 résidus N-terminaux et substitution du résidu Y du 1er motif YxxL
<400> 23
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   gcg cag gca ttg cta cca tcg gcg cca gag atc tac tct cac ctc tcc      96
   Ala Gln Ala Leu Leu Pro Ser Ala Pro Glu Ile Tyr Ser His Leu Ser
               20                  25                  30
   ccc acc aaa ccc gat tac atc aac ctt cga ccc tgc ccc                 135
   Pro Thr Lys Pro Asp Tyr Ile Asn Leu Arg Pro Cys Pro
           35                  40                  45
<210> 24
   <211> 45
   <212> PRT
   <213> Artificial
<220>
   <223> Synthetic Construct
<400> 24
   Ala Pro His Phe Pro Glu Ile Ser Phe Pro Pro Lys Pro Asp Ser Asp
   1               5                   10                  15
   Ala Gln Ala Leu Leu Pro Ser Ala Pro Glu Ile Tyr Ser His Leu Ser
               20                  25                  30
   Pro Thr Lys Pro Asp Tyr Ile Asn Leu Arg Pro Cys Pro
           35                  40                  45
<210> 25
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13 résidus N-terminaux et substitution du résidu Y du 2ème motif YxxL
<400> 25
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   tat cag gcc ttg cta cca tcg gcg cca gag atc gca tct cac ctc tcc      96
   Tyr Gln Ala Leu Leu Pro Ser Ala Pro Glu Ile Ala Ser His Leu Ser
               20                  25                  30
   ccc acc aaa ccc gat tac atc aac ctt cga ccc tgc ccc                  135
   Pro Thr Lys Pro Asp Tyr Ile Asn Leu Arg Pro Cys Pro
           35                  40                  45
<210> 26
   <211> 45
   <212> PRT
   <213> Artificial
<220>
   <223> Synthetic Construct
<400> 26
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Ala Ser His Leu Ser
               20                  25                  30
   Pro Thr Lys Pro Asp Tyr Ile Asn Leu Arg Pro Cys Pro
           35                  40                  45
<210> 27
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : substitution du résidu Y du 3ème motif YxxL
<400> 27
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc tcc      96
   Tyr Gln Ala Leu Leu Pro Ser Ala Pro Glu Ile Tyr Ser His Leu Ser
               20                  25                  30
   ccc acc aaa ccc gat gcc atc aac ctt agg ccc tgc ccc                  135
   Pro Thr Lys Pro Asp Ala Ile Asn Leu Arg Pro Cys Pro
           35                  40                  45
<210> 28
   <211> 45
   <212> PRT
   <213> Artificial
<220>
   <223> Synthetic Construct
<400> 28
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp Ala Ile Asn Leu Arg Pro Cys Pro
           35                  40                  45
<210> 29
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : substitution des 13 résidus Y N-terminaux et substitution du résidu S se trouvant avant le 1er motif YxxL
<220>
   <221> misc_feature
   <222> (1)..(135)
   <223> Dérivé muté du CD de la protéine TM du BLV : substitution des 13 résidus N-terminaux et substitution du résidu S se trouvant avant le 1er motif YxxL
<400> 29
   gca ccc cac ttc cct gaa atc tcc ttc ccc cct aaa ccc gat gct gat      48
   Ala Pro His Phe Pro Glu Ile Ser Phe Pro Pro Lys Pro Asp Ala Asp
   1               5                   10                  15
   tat cag gcg ttg cta cca tcc gcg cca gag atc tac tct cac ctc tcc      96
   Tyr Gln Ala Leu Leu Pro Ser Ala Pro Glu Ile Tyr Ser His Leu Ser
               20                  25                  30
   ccc acc aaa ccc gat tac atc aac ctt cga ccc tgc ccc                 135
           Pro Thr Lys Pro Asp Tyr Ile Asn Leu Arg Pro Cys Pro
                   35                  40                  45
<210> 30
   <211> 45
   <212> PRT
   <213> Artificial
<220>
   <223> Synthetic Construct
<400> 30
   Ala Pro His Phe Pro Glu Ile Ser Phe Pro Pro Lys Pro Asp Ala Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp Tyr Ile Asn Leu Arg Pro Cys Pro
           35                  40                  45
<210> 31
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13 résidus N-terminaux et substitution du résidu E se trouvant avant le 2ème motif YxxL
<400> 31
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   tat cag gcc ttg cta cca tcc gcg cca gcg atc tac tct cac ctc tcc      96
   Tyr Gln Ala Leu Leu Pro Ser Ala Pro Ala Ile Tyr Ser His Leu Ser
               20                  25                  30
   ccc acc aaa ccc gat tac atc aac ctt cga ccc tgc ccc                  135
   Pro Thr Lys Pro Asp Tyr Ile Asn Leu Arg Pro Cys Pro
           35                  40                  45
<210> 32
   <211> 45
   <212> PRT
   <213> Artificial
<220>
   <223> Synthetic Construct
<400> 32
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Ala Ile Tyr Ser His Leu Ser
               20                  25                  30
   Pro Thr Lys Pro Asp Tyr Ile Asn Leu Arg Pro Cys Pro
           35                  40                  45
<210> 33
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du domaine CD de la protéine TM du BLV : délétion des 13 résidus N-terminaux et substitution du résidu D se trouvant avant le 3ème motif YxxL
<400> 33
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc tcc      96
   Tyr Gln Ala Leu Leu Pro Ser Ala Pro Glu Ile Tyr Ser His Leu Ser
               20                  25                  30
   ccc acc aag ccg gct tac atc aac ctt cga ccc tgc ccc                  135
   Pro Thr Lys Pro Ala Tyr Ile Asn Leu Arg Pro Cys Pro
           35                  40                  45
<210> 34
   <211> 45
   <212> PRT
   <213> Artificial
<220>
   <223> synthetic Construct
<400> 34
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Ala Tyr Ile Asn Leu Arg Pro Cys Pro
           35                  40                  45
<210> 35
   <211> 18
   <212> DNA
   <213> Artificial
<220>
   <223> polynucléotide
<220>
   <221> CDS
   <222> (1)..(18)
<220>
   <221> misc_feature
   <222> (1)..(18)
   <223> Dérivé muté du domaine CD de la protéine TM du BLV : délétion des 13 résidus N-terminaux et des 39 résidus C-terminaux
<400> 35
   gca ccc cac ttc cct gaa      18
   Ala Pro His Phe Pro Glu
   1               5
<210> 36
   <211> 6
   <212> PRT
   <213> Artificial
<220>
   <223> Synthetic construct
<400> 36
   Ala Pro His Phe Pro Glu
   1               5
<210> 37
   <211> 45
   <212> DNA
   <213> Artificial
<220>
   <223> polynucléotide
<220>
   <221> CDS
   <222> (1)..(45)
<220>
   <221> misc_feature
   <222> (1)..(45)
   <223> Dérivé muté du domaine CD de la protéine TM du BLV : délétion des 13 résidus N-terminaux et des 30 résidus C-terminaux
<400> 37
   gca ccc cac ttc cct gaa atc tcc ttc ccc cct aaa ccc gat tct           45
   Ala Pro His Phe Pro Glu Ile Ser Phe Pro Pro Lys Pro Asp Ser
   1               5                   10                  15
<210> 38
   <211> 15
   <212> PRT
   <213> Artificial
<220>
   <223> Synthetic Construct
<400> 38
   Ala Pro His Phe Pro Glu Ile Ser Phe Pro Pro Lys Pro Asp Ser
   1               5                   10                  15
<210> 39
   <211> 63
   <212> DNA
   <213> Artificial
<220>
   <223> polynucléotide
<220>
   <221> CDS
   <222> (1)..(63)
<220>
   <221> misc_feature
   <222> (1)..(63)
   <223> Dérivé muté du domaine CD de la protéine TM du BLV : délétion des 13 résidus N-terminaux et des 24 résidus C-terminaux
<400> 39
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   tat cag gcc ttg cta                                                  63
   Tyr Gln Ala Leu Leu
               20
<210> 40
   <211> 21
   <212> PRT
   <213> Artificial
<220>
   <223> Synthetic Construct
<400> 40
   Ala Pro His Phe Pro Glu Ile Ser Phe Pro Pro Lys Pro Asp Ser Asp
   1               5                   10                  15
   Tyr Gln Ala Leu Leu
               20
<210> 41
   <211> 78
   <212> DNA
   <213> Artificial
<220>
   <223> polynucléotide
<220>
   <221> CDS
   <222> (1)..(78)
<220>
   <221> misc_feature
   <222> (1)..(78)
   <223> Dérivé muté du domaine CD de la protéine TM du BLV : délétion des 13 résidus N-terminaux et des 19 résidus C-terminaux
<400> 41
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   tat cag gcc ttg cta cca tcc gcg cca gag                              78
   Tyr Gln Ala Leu Leu Pro Ser Ala Pro Glu
               20                  25
<210> 42
   <211> 26
   <212> PRT
   <213> Artificial
<220>
   <223> Synthetic Construct
<400> 42
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu
               20                  25
<210> 43
   <211> 93
   <212> DNA
   <213> Artificial
<220>
   <223> polynucléotide
<220>
   <221> CDS
   <222> (1)..(93)
<220>
   <221> misc_feature
   <222> (1)..(93)
   <223> Dérivé muté du domaine CD de la protéine TM du BLV : délétion des 13 résidus N-terminaux et des 14 résidus C-terminaux
<400> 43
   gca ccc cac ttc cct gaa atc tcc ttc ccc cct aaa ccc gat tct gat       48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc           93
   Tyr Gln Ala Leu Leu Pro Ser Ala Pro Glu Ile Tyr Ser His Leu
               20                  25                  30
<210> 44
   <211> 31
   <212> PRT
   <213> Artificial
<220>
   <223> Synthetic Construct
<400> 44
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu
               20                  25                  30
<210> 45
   <211> 111
   <212> DNA
   <213> Artificial
<220>
   <223> polynucléotide
<220>
   <221> CDS
   <222> (1)..(111)
<220>
   <221> misc_feature
   <222> (1)..(111)
   <223> Dérivé muté du domaine CD de la protéine TM du BLV : délétion des 13 résidus N-terminaux et des 8 résidus C-terminaux
<400> 45
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc tcc      96
   Tyr Gln Ala Leu Leu Pro Ser Ala Pro Glu Ile Tyr Ser His Leu Ser
               20                  25                  30
   ccc acc aaa ccc gat                                                  111
   Pro Thr Lys Pro Asp
           35
<210> 46
   <211> 37
   <212> PRT
   <213> Artificial
<220>
   <223> Synthetic Construct
<400> 46
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp
           35
<210> 47
   <211> 123
   <212> DNA
   <213> Artificial
<220>
   <223> polynucléotide
<220>
   <221> CDS
   <222> (1)..(123)
<220>
   <221> misc_feature
   <222> (1)..(123)
   <223> Dérivé muté du domaine CD de la protéine TM du BLV : délétion des 13 résidus N-terminaux et des 4 résidus C-terminaux
<400> 47
   gca ccc cac ttc cct gaa atc tcc ttc ccc cct aaa ccc gat tct gat       48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc tcc       96
   Tyr Gln Ala Leu Leu Pro Ser Ala Pro Glu Ile Tyr Ser His Leu Ser
               20                  25                  30
   ccc acc aaa ccc gat tac atc aac ctt                                  123
   Pro Thr Lys Pro Asp Tyr Ile Asn Leu
           35                  40
<210> 48
   <211> 41
   <212> PRT
   <213> Artificial
<220>
   <223> Synthetic Construct
<400> 48
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp Tyr Ile Asn Leu
           35                  40
<210> 49
   <211> 25
   <212> PRT
   <213> Homo sapiens
<220>
   <221> MISC_FEATURE
   <222> (1)..(24)
   <223> CD4
<400> 49
   Met Asn Arg Gly Val Pro Phe Arg His Leu Leu Leu Val Leu Gln Leu
   1               5                   10                  15
   Ala Leu Leu Pro Ala Ala Thr Gln Gly
               20                  25
<210> 50
   <211> 22
   <212> PRT
   <213> Mus musculus
<220>
   <221> MISC_FEATURE
   <222> (1)..(22)
   <223> CD4
<400> 50
   Met Cys Arg Ala Ile Ser Leu Arg Arg Leu Leu Leu Leu Leu Leu Gln
   1               5                   10                  15
   Leu Ser Gln Leu Leu Ala
               20
<210> 51
   <211> 27
   <212> PRT
   <213> Mus musculus
<220>
   <221> MISC_FEATURE
   <222> (1)..(27)
   <223> CD8 alpha
<400> 51
   Met Ala Ser Pro Leu Thr Arg Phe Leu Ser Leu Asn Leu Leu Leu Leu
   1               5                   10                  15
   Gly Glu Ser Ile Ile Leu Gly Ser Gly Glu Ala
               20                  25
<210> 52
   <211> 25
   <212> PRT
   <213> Artificial
<220>
   <223> protéine CD8 alpha bovine
<400> 52
   Met Ala Ser Leu Leu Thr Ala Leu Ile Leu Pro Leu Ala Leu Leu Leu
   1 5 10 15
   Leu Asp Ala Ala Lys Val Leu Gly Ser
   20 25
<210> 53
   <211> 21
   <212> PRT
   <213> Homo sapiens
<220>
   <221> MISC_FEATURE
   <222> (1)..(21)
   <223> CD8 alpha
<400> 53
   Met Ala Leu Pro Val Thr Ala Leu Leu Leu Pro Leu Ala Leu Leu Leu
   1 5 10 15
   His Ala Ala Arg Pro
   20
<210> 54
   <211> 26
   <212> PRT
   <213> Rattus norvegicus
<220>
   <221> MISC_FEATURE
   <222> (1)..(26)
   <223> CD8 alpha
<400> 54
   Met Ala Leu Pro Val Thr Ala Leu Leu Leu Pro Leu Ala Leu Leu Leu
   1 5 10 15
   His Ala Ala Arg Pro Ser Gln Phe Arg Val
   20 25
<210> 55
   <211> 27
   <212> PRT
   <213> Homo sapiens
<220>
   <221> MISC_FEATURE
   <222> (1)..(27)
   <223> IL1R1
<400> 55
   Met Lys Val Leu Leu Arg Leu Ile Cys Phe Ile Ala Leu Leu Ile Ser
   1               5                   10                  15
   Ser Leu Glu Ala Asp Lys Cys Lys Glu Arg Glu
               20                  25
<210> 56
   <211> 24
   <212> PRT
   <213> Homo sapiens
<220>
   <221> MISC_FEATURE
   <222> (1)..(24)
   <223> EGFR 1, HER 1
<400> 56
   Met Arg Pro Ser Gly Thr Ala Gly Ala Ala Leu Leu Ala Leu Leu Ala
   1               5                   10                  15
   Ala Leu Cys Pro Ala Ser Arg Ala
               20
<210> 57
   <211> 22
   <212> PRT
   <213> Homo sapiens
<220>
   <221> MISC_FEATURE
   <222> (1)..(22)
   <223> HER 2
<400> 57
   Met Glu Leu Ala Ala Leu Cys Arg Trp Gly Leu Leu Leu Ala Leu Leu
   1               5                   10                  15
   Pro Pro Gly Ala Ala Ser
               20
<210> 58
   <211> 19
   <212> PRT
   <213> Homo sapiens
<220>
   <221> MISC_FEATURE
   <222> (1)..(19)
   <223> HER 3
<400> 58
   Met Arg Ala Asn Asp Ala Leu Gln Val Leu Gly Leu Leu Phe Ser Leu
   1 5 10 15
   Ala Arg Gly
<210> 59
   <211> 25
   <212> PRT
   <213> Homo sapiens
<220>
   <221> MISC_FEATURE
   <222> (1)..(25)
   <223> HER 4
<400> 59
   Met Lys Pro Ala Thr Gly Leu Trp Val Trp Val Ser Leu Leu Val Ala
   1 5 10 15
   Ala Gly Thr Val Gln Pro Ser Asp Ser
   20 25
<210> 60
   <211> 20
   <212> PRT
   <213> Mus musculus
<220>
   <221> MISC_FEATURE
   <222> (1)..(20)
   <223> IL-2
<400> 60
   Met Tyr Ser Met Gln Leu Ala Ser Cys Val Thr Leu Thr Leu Val Leu
   1 5 10 15
   Leu Val Asn Ser
   20
<210> 61
   <211> 24
   <212> PRT
   <213> Mus musculus
<220>
   <221> MISC_FEATURE
   <222> (1)..(24)
   <223> IL-6
<400> 61
   Met Lys Phe Leu Ser Ala Arg Asp Phe His Pro Val Ala Phe Leu Gly
   1               5                   10                  15
   Leu Met Leu Val Thr Thr Thr Ala
               20
<210> 62
   <211> 25
   <212> PRT
   <213> Homo sapiens
<220>
   <221> MISC_FEATURE
   <222> (1)..(25)
   <223> IL-7
<400> 62
   Met Phe His Val Ser Phe Arg Tyr Ile Phe Gly Leu Pro Pro Leu Ile
   1               5                   10                  15
   Leu Val Leu Leu Pro Val Ala Ser Ser
               20                  25
<210> 63
   <211> 18
   <212> PRT
   <213> Mus musculus
<220>
   <221> MISC_FEATURE
   <222> (1)..(18)
   <223> IL-10
<400> 63
   Met Pro Gly Ser Ala Leu Leu Cys Cys Leu Leu Leu Leu Thr Gly Met
   1               5                   10                  15
   Arg Ile
<210> 64
   <211> 23
   <212> PRT
   <213> Homo sapiens
<220>
   <221> MISC_FEATURE
   <222> (1)..(23)
   <223> MIP-1-alpha chimiokine
<400> 64
   Met Gln Val Ser Thr Ala Ala Leu Ala Val Leu Leu Cys Thr Met Ala
   1 5 10 15
   Leu Cys Asn Gln Phe Ser Ala
   20
<210> 65
   <211> 15
   <212> PRT
   <213> Influenza B virus
<220>
   <221> MISC_FEATURE
   <222> (1)..(15)
   <223> Hemagglutinine virus Influenza B
<400> 65
   Met Lys Ala Ile Ile Val Leu Leu Met Val Val Thr Ser Asn Ala
   1 5 10 15
<210> 66
   <211> 16
   <212> PRT
   <213> Influenza A virus
<220>
   <221> MISC_FEATURE
   <222> (1)..(16)
   <223> Hemagglutinine virus Influenza A H1N1
<400> 66
   Met Lys Ala Lys Leu Leu Val Leu Leu Cys Ala Leu Ser Ala Thr Asp
   1 5 10 15
<210> 67
   <211> 15
   <212> PRT
   <213> Influenza A virus
<220>
   <221> MISC_FEATURE
   <222> (1)..(15)
   <223> Hemagglutinine virus Influenza A H2N2
<400> 67
   Met Ala Ile Ile Tyr Leu Ile Leu Leu Phe Thr Ala Val Arg Gly
   1 5 10 15
<210> 68
   <211> 16
   <212> PRT
   <213> Influenza A virus
<220>
   <221> MISC_FEATURE
   <222> (1)..(16)
   <223> Hemagglutinine virus Influenza A H3N2
<400> 68
   Met Lys Thr Ile Ile Ala Leu Ser Tyr Ile Phe Cys Leu Ala Leu Gly
   1               5                   10                  15
<210> 69
   <211> 16
   <212> PRT
   <213> Influenza A virus
<220>
   <221> MISC_FEATURE
   <222> (1)..(16)
   <223> Hemagglutinine virus Influenza A H4N6
<400> 69
   Met Leu Ser Ile Val Ile Leu Phe Leu Leu Ile Ala Glu Asn Ser Ser
   1               5                   10                  15
<210> 70
   <211> 16
   <212> PRT
   <213> Influenza A virus
<220>
   <221> MISC_FEATURE
   <222> (1)..(16)
   <223> Hemagglutinine virus Influenza A H5N1
<400> 70
   Met Glu Lys Ile Val Leu Leu Leu Ala Ile Ile Ser Leu Val Lys Ser
   1               5                   10                  15
<210> 71
   <211> 16
   <212> PRT
   <213> Influenza A virus
<220>
   <221> MISC_FEATURE
   <222> (1)..(16)
   <223> Hemagglutinine virus Influenza A H6N5
<400> 71
   Met Ile Ala Ile Ile Val Val Ala Ile Leu Ala Thr Ala Gly Arg Ser
   1               5                   10                  15
<210> 72
   <211> 18
   <212> PRT
   <213> Influenza A virus
<220>
   <221> MISC_FEATURE
   <222> (1)..(18)
   <223> Hemagglutinine virus Influenza A H7N7
<400> 72
   Met Asn Thr Gln Ile Leu Ile Leu Thr Leu Val Ala Ala Ile His Thr
   1 5 10 15
   Asn Ala
<210> 73
   <211> 16
   <212> PRT
   <213> Influenza A virus
<220>
   <221> MISC_FEATURE
   <222> (1)..(16)
   <223> Hemagglutinine virus Influenza A H8N4
<400> 73
   Met Glu Lys Phe Ile Ala Ile Ala Thr Leu Ala Ser Thr Asn Ala Tyr
   1 5 10 15
<210> 74
   <211> 18
   <212> PRT
   <213> Influenza A virus
<220>
   <221> MISC_FEATURE
   <222> (1)..(18)
   <223> Hemagglutinine virus Influenza A H9N2
<400> 74
   Met Glu Thr Lys Ala Ile Ile Ala Ala Leu Leu Met Val Thr Ala Ala
   1 5 10 15
   Asn Ala
<210> 75
   <211> 16
   <212> PRT
   <213> Influenza A virus
<220>
   <221> MISC_FEATURE
   <222> (1)..(16)
   <223> Hemagglutinine virus Influenza A H10N7
<400> 75
   Met Tyr Lys Val Val Val Ile Ile Ala Leu Leu Gly Ala Val Lys Gly
   1 5 10 15
<210> 76
   <211> 16
   <212> PRT
   <213> Influenza A virus
<220>
   <221> MISC_FEATURE
   <222> (1)..(16)
   <223> Hemagglutinine virus Influenza A H11N6
<400> 76
   Met Glu Lys Thr Leu Leu Phe Ala Ala Ile Phe Leu Cys Val Lys Ala
   1 5 10 15
<210> 77
   <211> 17
   <212> PRT
   <213> Influenza A virus
<220>
   <221> MISC_FEATURE
   <222> (1)..(17)
   <223> Hemagglutinine virus Influenza A H12N5
<400> 77
   Met Glu Lys Phe Ile Ile Leu Ser Thr Val Leu Ala Ala Ser Phe Ala
   1 5 10 15
   Tyr
<210> 78
   <211> 18
   <212> PRT
   <213> Influenza A virus
<220>
   <221> MISC_FEATURE
   <222> (1)..(18)
   <223> Hemagglutinine virus Influenza A H13N6
<400> 78
   Met Ala Leu Asn Val Ile Ala Thr Leu Thr Leu Ile Ser Val Cys Val
   1 5 10 15
   His Ala
<210> 79
   <211> 246
   <212> DNA
   <213> Artificial
<220>
   <223> polynucléotide
<220>
   <221> misc_feature
   <222> (1)..(246)
   <223> Construction X2
<220>
   <221> CDS
   <222> (1)..(246)
<400> 79
   ctc att cat tct gtt cta agc cta ttc cta tta gcc ctt ttt ttg ctc       48
   Leu Ile His Ser Val Leu Ser Leu Phe Leu Leu Ala Leu Phe Leu Leu
   1               5                   10                  15
   ttc ttg gcc ccc tgc ctg ata aaa tgc ttg acc tct cgc ctt tta aaa       96
   Phe Leu Ala Pro Cys Leu Ile Lys Cys Leu Thr Ser Arg Leu Leu Lys
               20                  25                  30
   ctc ctc cgg cag gct ccc cac ttc cct gaa atc tcc ttc ccc cct aaa      144
   Leu Leu Arg Gln Ala Pro His Phe Pro Glu Ile Ser Phe Pro Pro Lys
           35                  40                  45
   ccc gat tct gat tat cag gcc ttg cta cca tcc gcg cca gag atc tac      192
   Pro Asp Ser Asp Tyr Gln Ala Leu Leu Pro Ser Ala Pro Glu Ile Tyr
       50                  55                  60
   tct cac ctc tcc ccc acc aaa ccc gat tac atc aac ctt cga ccc tgc      240
   Ser His Leu Ser Pro Thr Lys Pro Asp Tyr Ile Asn Leu Arg Pro Cys
   65                  70                  75                  80
   ccc tag                                                              246
   Pro
<210> 80
   <211> 81
   <212> PRT
   <213> Artificial
<220>
   <223> Synthetic Construct
<400> 80
   Leu Ile His Ser Val Leu Ser Leu Phe Leu Leu Ala Leu Phe Leu Leu
   1               5                   10                  15
   Phe Leu Ala Pro Cys Leu Ile Lys Cys Leu Thr Ser Arg Leu Leu Lys
               20                  25                  30
   Leu Leu Arg Gln Ala Pro His Phe Pro Glu Ile Ser Phe Pro Pro Lys
           35                  40                  45
   Pro Asp Ser Asp Tyr Gln Ala Leu Leu Pro Ser Ala Pro Glu Ile Tyr
       50                  55                  60
   Ser His Leu Ser Pro Thr Lys Pro Asp Tyr Ile Asn Leu Arg Pro Cys
   65                  70                  75                  80
   Pro
<210> 81
   <211> 246
   <212> DNA
   <213> Artificial
<220>
   <223> polynucléotide
<220>
   <221> misc_feature
   <222> (1)..(246)
   <223> Construction X3
<220>
   <221> CDS
   <222> (1)..(246)
<400> 81
   att tac atc tgg gca ccc ttg gcc gga atc tgc gtg gcc ctt ctg cta      48
   Ile Tyr Ile Trp Ala Pro Leu Ala Gly Ile Cys Val Ala Leu Leu Leu
   1               5                   10                  15
   agc ttg atc ccc tgc ctg ata aaa tgc ctg acc tct cgc ctt tta aaa      96
   Ser Leu Ile Pro Cys Leu Ile Lys Cys Leu Thr Ser Arg Leu Leu Lys
               20                  25                  30
   ctc ctc cgg cag gct ccc cac ttc cct gaa atc tcc ttc ccc cct aaa     144
   Leu Leu Arg Gln Ala Pro His Phe Pro Glu Ile Ser Phe Pro Pro Lys
           35                  40                  45
   ccc gat tct gat tat cag gcc ttg cta cca tcc gcg cca gag atc tac     192
   Pro Asp Ser Asp Tyr Gln Ala Leu Leu Pro Ser Ala Pro Glu Ile Tyr
       50                  55                  60
   tct cac ctc tcc ccc acc aaa ccc gat tac atc aac ctt cga ccc tgc     240
   Ser His Leu Ser Pro Thr Lys Pro Asp Tyr Ile Asn Leu Arg Pro Cys
   65                  70                  75                  80
   ccc tag                                                             246
   Pro
<210> 82
   <211> 81
   <212> PRT
   <213> Artificial
<220>
   <223> Synthetic Construct
<400> 82
   Ile Tyr Ile Trp Ala Pro Leu Ala Gly Ile Cys Val Ala Leu Leu Leu
   1               5                   10                  15
   Ser Leu Ile Pro Cys Leu Ile Lys Cys Leu Thr Ser Arg Leu Leu Lys
               20                  25                  30
   Leu Leu Arg Gln Ala Pro His Phe Pro Glu Ile Ser Phe Pro Pro Lys
           35                  40                  45
   Pro Asp Ser Asp Tyr Gln Ala Leu Leu Pro Ser Ala Pro Glu Ile Tyr
       50                  55                  60
   Ser His Leu Ser Pro Thr Lys Pro Asp Tyr Ile Asn Leu Arg Pro Cys
   65                  70                  75                  80
   Pro
<210> 83
   <211> 225
   <212> DNA
   <213> Artificial
<220>
   <223> polynucléotide
<220>
   <221> misc_feature
   <222> (1)..(225)
   <223> Construction X4
<220>
   <221> CDS
   <222> (1)..(225)
<400> 83
   att tac atc tgg gca ccc ttg gcc gga atc tgc gtg gcc ctt ctg ctg       48
   Ile Tyr Ile Trp Ala Pro Leu Ala Gly Ile Cys Val Ala Leu Leu Leu
   1               5                   10                  15
   tcc ttg atc atc act ctc atc tgc tac cac agg tct aga gct ccc cac       96
   Ser Leu Ile Ile Thr Leu Ile Cys Tyr His Arg Ser Arg Ala Pro His
               20                  25                  30
   ttc cct gaa atc tcc ttc ccc cct aaa ccc gat tct gat tat cag gcc      144
   Phe Pro Glu Ile Ser Phe Pro Pro Lys Pro Asp Ser Asp Tyr Gln Ala
           35                  40                  45
   ttg cta cca tcc gcg cca gag atc tac tct cac ctc tcc ccc acc aaa      192
   Leu Leu Pro Ser Ala Pro Glu Ile Tyr Ser His Leu Ser Pro Thr Lys
       50                  55                  60
   ccc gat tac atc aac ctt cga ccc tgc ccc tag                        225
   Pro Asp Tyr Ile Asn Leu Arg Pro Cys Pro
   65                  70
<210> 84
   <211> 74
   <212> PRT
   <213> Artificial
<220>
   <223> Synthetic Construct
<400> 84
   Ile Tyr Ile Trp Ala Pro Leu Ala Gly Ile Cys Val Ala Leu Leu Leu
   1               5                   10                  15
   Ser Leu Ile Ile Thr Leu Ile Cys Tyr His Arg Ser Arg Ala Pro His
               20                  25                  30
   Phe Pro Glu Ile Ser Phe Pro Pro Lys Pro Asp Ser Asp Tyr Gln Ala
           35                  40                  45
   Leu Leu Pro Ser Ala Pro Glu Ile Tyr Ser His Leu Ser Pro Thr Lys
       50                  55                  60
   Pro Asp Tyr Ile Asn Leu Arg Pro Cys Pro
   65                  70
<210> 85
   <211> 947
   <212> DNA
   <213> Artificial
<220>
   <223> polynucléotide
<220>
   <221> misc_feature
   <222> (1)..(947)
   <223> chimère CD8 alpha de souris / domaine CD de TM du BLV
<220>
   <221> CDS
   <222> (28)..(831)
<400> 85
   ggatcccttg ctggtggaga gcacacc atg gcc tca ccg ttg acc cgc ttt ctg      54
                                 Met Ala Ser Pro Leu Thr Arg Phe Leu
                                 1               5
   tcg ctg aac ctg ctg ctg ctg ggt gag tcg att atc ctg ggg agt gga       102
   Ser Leu Asn Leu Leu Leu Leu Gly Glu Ser Ile Ile Leu Gly Ser Gly
   10                  15                  20                  25
   gaa gct aag cca cag gca ccc gaa ctc cga atc ttt cca aag aaa atg       150
   Glu Ala Lys Pro Gln Ala Pro Glu Leu Arg Ile Phe Pro Lys Lys Met
                   30                  35                  40
   gac gcc gaa ctt ggt cag aag gtg gac ctg gta tgt gaa gtg ttg ggg      198
   Asp Ala Glu Leu Gly Gln Lys Val Asp Leu Val Cys Glu Val Leu Gly
               45                  50                  55
   tcc gtt tcg caa gga tgc tct tgg ctc ttc cag aac tcc agc tcc aaa      246
   Ser Val Ser Gln Gly Cys Ser Trp Leu Phe Gln Asn Ser Ser Ser Lys
           60                  65                  70
   ctc ccc cag ccc acc ttc gtt gtc tat atg gct tca tcc cac aac aag      294
   Leu Pro Gln Pro Thr Phe Val Val Tyr Met Ala Ser Ser His Asn Lys
       75                  80                  85
   ata acg tgg gac gag aag ctg aat tcg tcg aaa ctg ttt tct gcc atg      342
   Ile Thr Trp Asp Glu Lys Leu Asn Ser Ser Lys Leu Phe Ser Ala Met
   90                  95                  100                 105
   agg gac acg aat aat aag tac gtt ctc acc ctg aac aag ttc agc aag      390
   Arg Asp Thr Asn Asn Lys Tyr Val Leu Thr Leu Asn Lys Phe Ser Lys
                   110                 115                 120
   gaa aac gaa ggc tac tat ttc tgc tca gtc atc agc aac tcg gtg atg      438
   Glu Asn Glu Gly Tyr Tyr Phe Cys Ser Val Ile Ser Asn Ser Val Met
               125                 130                 135
   tac ttc agt tct gtc gtg cca gtc ctt cag aaa gtg aac tct act act      486
   Tyr Phe Ser Ser Val Val Pro Val Leu Gln Lys Val Asn Ser Thr Thr
           140                 145                 150
   acc aag cca gtg ctg cga act ccc tca cct gtg cac cct acc ggg aca      534
   Thr Lys Pro Val Leu Arg Thr Pro Ser Pro Val His Pro Thr Gly Thr
       155                 160                 165
   tct cag ccc cag aga cca gaa gat tgt cgg ccc cgt ggc tca gtg aag      582
   Ser Gln Pro Gln Arg Pro Glu Asp Cys Arg Pro Arg Gly Ser Val Lys
   170                 175                 180                 185
   ggg acc gga ttg gac ttc gcc tgt gat att tac atc tgg gca ccc ttg      630
   Gly Thr Gly Leu Asp Phe Ala Cys Asp Ile Tyr Ile Trp Ala Pro Leu
                   190                 195                 200
   gcc gga atc tgc gtg gcc ctt ctg ctg tcc ttg atc atc act ctc atc      678
   Ala Gly Ile Cys Val Ala Leu Leu Leu Ser Leu Ile Ile Thr Leu Ile
               205                 210                 215
   tgc tac cac agg tct aga gct ccc cac ttc cct gaa atc tcc ttc ccc      726
   Cys Tyr His Arg Ser Arg Ala Pro His Phe Pro Glu Ile Ser Phe Pro
           220                 225                 230
   cct aaa ccc gat tct gat tat cag gcc ttg cta cca tcc gcg cca gag      774
   Pro Lys Pro Asp Ser Asp Tyr Gln Ala Leu Leu Pro Ser Ala Pro Glu
       235                 240                 245
   atc tac tct cac ctc tcc ccc acc aaa ccc gat tac atc aac ctt cga      822
   Ile Tyr Ser His Leu Ser Pro Thr Lys Pro Asp Tyr Ile Asn Leu Arg
   250                 255                 260                 265
   ccc tgc ccc taggaccccc atgtttcacg caccctcagg ctgtggtggg              871
   Pro Cys Pro
   gcactggctt agtggaatag tcagtgtacc atcacaagcc tcttcttgct gccagcaccg    931
   agttcgaagc ggccgc                                                    947
<210> 86
   <211> 268
   <212> PRT
   <213> Artificial
<220>
   <223> Synthetic Construct
<400> 86
   Met Ala Ser Pro Leu Thr Arg Phe Leu Ser Leu Asn Leu Leu Leu Leu
   1               5                   10                  15
   Gly Glu Ser Ile Ile Leu Gly Ser Gly Glu Ala Lys Pro Gln Ala Pro
               20                  25                  30
   Glu Leu Arg Ile Phe Pro Lys Lys Met Asp Ala Glu Leu Gly Gln Lys
           35                  40                  45
   Val Asp Leu Val Cys Glu Val Leu Gly Ser Val Ser Gln Gly Cys Ser
       50                  55                  60
   Trp Leu Phe Gln Asn Ser Ser Ser Lys Leu Pro Gln Pro Thr Phe Val
   65                  70                  75                  80
   Val Tyr Met Ala Ser Ser His Asn Lys Ile Thr Trp Asp Glu Lys Leu
                   85                  90                  95
   Asn Ser Ser Lys Leu Phe Ser Ala Met Arg Asp Thr Asn Asn Lys Tyr
               100                 105                 110
   Val Leu Thr Leu Asn Lys Phe Ser Lys Glu Asn Glu Gly Tyr Tyr Phe
           115                 120                 125
   Cys Ser Val Ile Ser Asn Ser Val Met Tyr Phe Ser Ser Val Val Pro
       130                 135                 140
   Val Leu Gln Lys Val Asn Ser Thr Thr Thr Lys Pro Val Leu Arg Thr
   145                 150                 155                 160
   Pro Ser Pro Val His Pro Thr Gly Thr Ser Gln Pro Gln Arg Pro Glu
                   165                 170                 175
   Asp Cys Arg Pro Arg Gly Ser Val Lys Gly Thr Gly Leu Asp Phe Ala
               180                 185                 190
   Cys Asp Ile Tyr Ile Trp Ala Pro Leu Ala Gly Ile Cys Val Ala Leu
           195                 200                 205
   Leu Leu Ser Leu Ile Ile Thr Leu Ile Cys Tyr His Arg Ser Arg Ala
       210                 215                 220
   Pro His Phe Pro Glu Ile Ser Phe Pro Pro Lys Pro Asp Ser Asp Tyr
   225                 230                 235                 240
   Gln Ala Leu Leu Pro Ser Ala Pro Glu Ile Tyr Ser His Leu Ser Pro
                   245                 250                 255
   Thr Lys Pro Asp Tyr Ile Asn Leu Arg Pro Cys Pro
               260                 265
<210> 87
   <211> 12
   <212> DNA
   <213> Artificial
<220>
   <223> oligonucleotide
<220>
   <221> CDS
   <222> (1)..(12)
<400> 87
   cca tcc gcg cca                                   12
   Pro Ser Ala Pro
   1
<210> 88
   <211> 4
   <212> PRT
   <213> Artificial
<220>
   <223> Synthetic Construct
<400> 88
   Pro Ser Ala Pro
   1
<210> 89
   <211> 4
   <212> PRT
   <213> Artificial
<220>
   <223> polypeptide
<400> 89
   Pro Thr Ala Pro
   1
<210> 90
   <211> 12
   <212> DNA
   <213> Artificial
<220>
   <223> oligonucleotide
<220>
   <221> CDS
   <222> (1)..(12)
<400> 90
   tac atc aac ctt                                              12
   Tyr Ile Asn Leu
   1
<210> 91
   <211> 4
   <212> PRT
   <213> Artificial
<220>
   <223> Synthetic Construct
<400> 91
   Tyr Ile Asn Leu
   1
<210> 92
   <211> 15
   <212> DNA
   <213> Artificial
<220>
   <223> oligonucleotide
<220>
   <221> CDS
   <222> (1)..(15)
<400> 92
   gat tac atc aac ctt                                          15
   Asp Tyr Ile Asn Leu
   1               5
<210> 93
   <211> 5
   <212> PRT
   <213> Artificial
<220>
   <223> Synthetic Construct
<400> 93
   Asp Tyr Ile Asn Leu
   1               5
<210> 94
   <211> 60
   <212> DNA
   <213> Artificial
<220>
   <223> oligonucleotide
<220>
   <221> CDS
   <222> (1)..(60)
<220>
   <221> misc_feature
   <222> (1)..(60)
   <223> Dérivé muté du CD de la protéine TM du BLV
<400> 94
   cca tcc gcg cca gag atc tac tct cac ctc tcc ccc acc aaa ccc gat       48
   Pro Ser Ala Pro Glu Ile Tyr Ser His Leu Ser Pro Thr Lys Pro Asp
   1               5                   10                  15
   tac atc aac ctt                                                       60
   Tyr Ile Asn Leu
               20
<210> 95
   <211> 20
   <212> PRT
   <213> Artificial
<220>
   <223> Synthetic Construct
<400> 95
   Pro Ser Ala Pro Glu Ile Tyr Ser His Leu Ser Pro Thr Lys Pro Asp
   1               5                   10                  15
   Tyr Ile Asn Leu
               20
<210> 96
   <211> 12
   <212> DNA
   <213> Artificial
<220>
   <223> Polynucleotide
<220>
   <221> CDS
   <222> (1)..(12)
<400> 96
   tac tct cac ctc                                                       12
   Tyr Ser His Leu
   1
<210> 97
   <211> 4
   <212> PRT
   <213> Artificial
<220>
   <223> Synthetic Construct
<400> 97
   Tyr Ser His Leu
   1
<210> 98
   <211> 40
   <212> DNA
   <213> Artificial
<220>
   <223> Amorce S vers A, sens
<400> 98
   ccctaaaccc gatgctgatt atcaggcgtt gctaccatcc 40
<210> 99
   <211> 40
   <212> DNA
   <213> Artificial
<220>
   <223> Amorce S vers A, anti-sens
<400> 99
   cgcggatggt agcaacgcct gataatcagc atcgggttta 40
<210> 100
   <211> 25
   <212> DNA
   <213> Artificial
<220>
   <223> Amorce D vers A, sens
<400> 100
   ccaccaagcc ggcatacatc aacct 25
<210> 101
   <211> 27
   <212> DNA
   <213> Artificial
<220>
   <223> Amorce D vers A, anti-sens
<400> 101
   tcgaaggttg atgtatgccg gcttggt 27
<210> 102
   <211> 25
   <212> DNA
   <213> Artificial
<220>
   <223> amorce E vers A, sens
<400> 102
   gctaccatcc gcgccagcga tctac 25
<210> 103
   <211> 23
   <212> DNA
   <213> Artificial
<220>
   <223> Amorce E vers A, anti-sens
<400> 103
   gtagatcgct ggcgcggatg gta 23

## Description

La demande concerne des polynucléotides et polypeptides chimériques permettant la sécrétion d'un polypeptide d'intérêt en association avec des exosomes, et leur utilisation pour la production de compositions immunogènes à base d'ADN ou d'exosomes ou pour le criblage d'interactions protéiques.

La demande concerne un polypeptide chimérique capable d'être sécrété en association avec des exosomes lorsqu'il est exprimé dans des cellules eucaryotes appropriées, ledit polypeptide chimérique comprenant plusieurs domaines polypeptidiques.

La demande ainsi que l'invention concernent également un polypeptide constitué par l'un de ces domaines, permettant la sécrétion, en association avec des exosomes, d'un peptide ou polypeptide d'intérêt auquel il est associé.

La demande ainsi que l'invention concernent également une vésicule membranaire, en particulier un exosome, qui comporte un polypeptide de la demande ou l'invention, une composition immunogène à base de tels exosomes et un procédé de production de tels exosomes.

La demande ainsi que l'invention concernent également un polynucléotide codant pour un polypeptide de la demande ou l'invention et une composition immunogène le comprenant, en particulier un vaccin à ADN le comprenant.

La demande ainsi que l'invention concernent également la mise en oeuvre des propriétés des vésicules membranaires et des compositions immunogènes de la demande ou l'invention pour la prophylaxie et/ou le traitement d'une infection par un agent pathogène, un organisme pathogène, un antigène tumoral ou un antigène cytoplasmique, en particulier pour éliciter ou favoriser *in vivo,* chez un hôte (humain ou non humain), une réponse humorale et/ou cellulaire contre un virus, une bactérie, un parasite ou une tumeur.

La demande ainsi que l'invention concernent également l'utilisation de vésicules membranaires de la demande ou l'invention pour produire des anticorps dirigés contre le peptide ou le polypeptide d'intérêt ou dans le cadre d'un procédé de criblage de molécules interagissant avec le peptide ou le polypeptide d'intérêt.

Les exosomes se présentent sous la forme de petites sphères limitées par une bicouche lipidique. Ces vésicules membranaires sont naturellement sécrétées par différents types de cellules, en particulier par les cellules épithéliales, des cellules tumorales et certaines cellules du système immunitaire, (mastocytes, lymphocytes T et B, cellules dendritiques, notamment cellules de Langerhans). Les exosomes se distinguent d'autres vésicules membranaires sécrétées par les cellules notamment par leur petite taille (de 50 à 100 nm de diamètre) et par leur composition en protéines membranaires (des molécules d'adhésion, de transport, de transduction de signal et des molécules du complexe majeur d'histocompatibilité entre autres).

Les exosomes pourraient notamment correspondre à des vésicules internes des endosomes multivésiculaires (notamment des endosomes tardifs) sécrétées par la cellule lors de la fusion de ces endosomes avec la membrane plasmique ; les endosomes multivésiculaires sont généralement impliqués dans le transport de molécules dans les compartiments lysosomiques (voie de dégradation des protéines) mais, dans certaines cellules telles que les réticulocytes et certaines cellules présentatrices d'antigènes, ils pourraient être dirigés vers la membrane plasmique, avec laquelle ils fusionneraient pour libérer des exosomes dans le milieu extracellulaire.

Des études antérieures ont démontré que les exosomes sont capables d'induire des réponses immunes humorales et/ou cellulaires (Delcayre *et al.,* 2002). Les exosomes sont considérés comme des vecteurs d'antigènes capables de stimuler directement *in vitro* des lymphocytes T de manière antigène spécifique. En effet, les exosomes sécrétés par les cellules dendritiques expriment des molécules du complexe majeur d'histocompatibilité (CMH) de classe I et II. Les complexes peptides/CMH fonctionnels portés par les exosomes seraient transférés d'une cellule dendritique à une autre qui n'a jamais vu l'antigène dont dérivent les peptides associés aux molécules du CMH. En stimulant de proche en proche les cellules dendritiques naïves, les exosomes sécrétés et présentant à leur surface un peptide antigénique contribueraient à l'amplification de la réponse T CD4 et T CD8 spécifique (Delcayre et Le Pecq, 2006). Par exemple, chargés avec des peptides tumoraux et injectés à des souris, ces exosomes sont capables de promouvoir une forte réponse immunitaire et de faire régresser des tumeurs solides pré-établies (Zitvogel et al., 1998).

Les exosomes sont capables de présenter des antigènes exogènes soit sous la forme de protéines entières natives, soit sous la forme de peptides associés aux molécules du CMH I et II (Colino et Snapper 2006). La présentation d'antigène à la surface d'exosomes est similaire à une présentation à la membrane d'une cellule ou d'un virus enveloppé. Toutefois, les exosomes n'étant ni vivants ni infectieux, ils présentent l'avantage de pouvoir être manipulés comme un produit ordinaire et sans précautions de confinement, contrairement à un virus. Les exosomes peuvent donc être utilisés comme présentateurs de peptides ou de polypeptides antigéniques à des fins d'immunisation. Cette technique dite « exosome display » ne requiert pas nécessairement la présentation directe de l'antigène par le CMH (Chaput et al., 2004). Néanmoins le développement de cette technique de vaccination inédite suppose de disposer d'un « outil » moléculaire efficace permettant le ciblage des protéines antigéniques sur les exosomes. Or, à ce jour, un tel « outil » n'a pas été décrit.

L'étude des rétrovirus, et plus particulièrement du virus d'immunodéficience humaine (VIH), a révélé leur capacité à détourner la machinerie cellulaire de biogenèse des endosomes multivésiculaires afin de bourgeonner à la membrane plasmique (Pornillos *et al.,* 2002). Ces virus peuvent également utiliser cette machinerie au niveau de la membrane endosomale, son site normal de fonctionnement. En effet, dans les macrophages, le VIH bourgeonne au niveau de la membrane des endosomes multivésiculaires, qui accumulent donc une importante quantité de particules virales (Raposo et al., 2002). Cette découverte, associée à l'étude comparative des exosomes et des particules de VIH a conduit à l'hypothèse des « exosomes chevaux de Troie » (Trojan Exosomes ; Gould et al., 2003). Dans cette hypothèse, les virus utiliseraient la voie préexistante de biogenèse des exosomes, pour la formation de particules virales.

Par ailleurs, le site d'assemblage des virus pourrait avoir une conséquence significative sur la pathogénicité et le développement de l'infection. Dans le cas du VIH, on sait actuellement que les macrophages infectés constituent un important réservoir infectieux. Ces cellules apparaissent, en effet, moins susceptibles à l'apoptose normalement induite par la présence de particules virales (Herbein et al., 2002) ; leur production virale est moins sensible aux drogues antivirales et les virus semblent capables de conserver leur pouvoir infectieux durant de longues périodes. Le fait que le VIH bourgeonne et s'accumule dans les endosomes multivésiculaires de ces cellules pourrait donc avoir d'importantes conséquences sur ce type d'infection.

Les rétrovirus sont des virus enveloppés possédant un génome à ARN et dont le cycle de réplication est très particulier puisqu'il fait appel à une transcriptase inverse, qui transcrit le génome ARN viral en ADN bicaténaire, et une intégrase virale, permettant l'insertion de l'ADN double brin dans le génome de la cellule infectée.

L'enveloppe des rétrovirus est constituée de la protéine d'enveloppe externe (SU), responsable de la liaison au récepteur de la cellule cible, et la glycoprotéine trans-membranaire (TM), responsable de la fusion entre les membranes virales et cellulaires.

Les glycoprotéines d'enveloppe SU et TM sont issues du clivage de la protéine précurseur mature nommée ENV. L'expression du gène *env* aboutit à la synthèse des glycoprotéines d'enveloppe des rétrovirus, sous forme d'un précurseur protéique qui transite par le Golgi avant d'atteindre la portion de membrane (endosomale ou plasmique), qui servira d'enveloppe virale lors du bourgeonnement des virions. Lors de ce trajet, ce précurseur oligomérique est glycosylé et clivé en glycoprotéines de surface (SU) et transmembranaire (TM). Les protéines SU et TM demeurent associées et sont ancrées dans la membrane cellulaire par l'intermédiaire d'une hélice hydrophobe transmembranaire de la protéine TM.

Les glycoprotéines d'enveloppe (SU et TM) jouent un rôle majeur dans le cycle de multiplication rétrovirale. L'initiation de l'infection se fait par fixation et fusion de la particule virale à la membrane plasmique cellulaire. Cette fixation est engendrée par la liaison de SU à un récepteur spécifique présent sur la surface de la cellule cible.

La glycoprotéine TM des rétrovirus présente de multiples facettes dues à l'association de ses domaines externe, transmembranaire et cytoplasmique (CD™), qui en font la seule protéine des rétrovirus permettant une communication de part et d'autre des membranes du virus et de la cellule infectée (Cann et al., 1992; Delamarre et al., 1996).

La protéine TM est impliquée dans le phénomène de pénétration du virus dans la cellule cible, en provoquant la fusion entre les membranes virales et cellulaires. Elle serait aussi impliquée dans la transmission des informations entre milieux extra et intracellulaire. Il existe une dépendance structurelle mais aussi fonctionnelle entre les domaines interne et externe, qui se traduit par un transfert d'informations entre ces domaines. Ainsi, le domaine cytoplasmique peut moduler l'activité fusionnante de la protéine TM, activité pourtant portée par la région externe de la molécule. Dans d'autres cas, un signal externe peut être transduit via le CD™ pour déclencher une activation de la cellule. Ceci révèle que le CD™ interagit plus ou moins directement avec des protéines de la machinerie de transduction de signaux de la cellule.

Lors de son cheminement intracellulaire, la protéine TM est susceptible d'influer sur le tri, le devenir, le ciblage et le bourgeonnement des particules virales, par interactions avec le cytosquelette, ainsi qu'avec les machineries d'ubiquitination et de bourgeonnement (Cann et al., 1992 ; Delamarre et al., 1996 ; Straub et Levy, 1999)

Une étude antérieure suggère que le domaine cytoplasmique de la protéine TM de deux rétrovirus, le virus de la leucémie bovine (BLV, pour « Bovine Leukemia Virus ») et le VIH, permet d'induire l'adressage et la sécrétion d'une protéine recombinante dans les exosomes (De Gassart et al., 2004). Des cellules de la lignée K562 (une lignée cellulaire érythroleucémique d'origine humaine), qui sécrètent des exosomes de façon constitutive, ont été transfectées avec des vecteurs rétroviraux permettant d'exprimer, en système eucaryote, deux types de protéine chimère : (i) une chimère comportant le domaine extracellulaire de la protéine CD8 murine et les domaines transmembranaire et cytoplasmique de la glycoprotéine TM du BLV (chimère TM-BLV/CD8), et (ii) une chimère comportant les domaines extracellulaire et transmembranaire de la protéine CD8 murine et le domaine cytoplasmique de la protéine TM du VIH (chimère TM-HIV/CD8). Les deux chimères sont exprimées à la fois dans les cellules K562 transfectées et dans les exosomes sécrétés par ces cellules. En particulier, l'expression de la protéine chimère TM-BLV/CD8 dans les cellules de la lignée K562 disparaît rapidement après transit dans le réseau trans-golgien et les compartiments endosomaux tardifs pour se retrouver dans les exosomes sécrétés par ces cellules. Il semble que la chimère comportant le domaine cytoplasmique de la protéine TM du BLV soit plus fortement adressée vers les exosomes que la chimère comportant le domaine cytoplasmique de la protéine TM du VIH.

Cependant, comme le prouvent les résultats présentés dans la partie exemple de la demande, des exosomes portant la construction chimère TM-BLV/CD8 décrite par De Gassart et al. ne sont produits efficacement que dans les cellules de la lignée K562, et sont peu ou pas produits efficacement dans d'autres types cellulaires tels que les cellules de la lignée HEK293.

La demande ainsi que l'invention concernent des polypeptides particuliers qui se distinguent notamment de ceux décrits dans l'art antérieur et, en particulier de ceux décrits dans De Gassart et al, en ce qu'ils permettent d'adresser de façon beaucoup plus efficace un peptide ou un polypeptide d'intérêt vers des exosomes produits par les cellules de la lignée HEK293 et ainsi, d'amplifier très fortement (de 10 à 100 fois) la production d'exosomes comportant ledit peptide ou polypeptide d'intérêt. Par ailleurs, contrairement aux constructions décrites dans De Gassart et al, qui ne peuvent être utilisées efficacement que dans des cellules de la lignée K562, ces polypeptides peuvent être adressés à la membrane d'exosomes et sécrétés en association avec des vésicules membranaires, en particulier des exosomes produit(e)s par différents types cellulaires, en particulier des cellules HEK293 ou des lymphocytes T ou des lymphocytes B, et pas uniquement par des cellules de la lignée K562.

Ainsi, les moyens de la demande ainsi que ceux de l'invention permettent notamment de produire des quantités importantes de vésicules membranaires, en particulier d'exosomes, utilisables en immunisation et notamment en vaccination. De telles vésicules membranaires (notamment exosomes) pourraient également être produites *in vivo* par un hôte humain ou non humain (en particulier un mammifère humain ou non humain ou un oiseau), que l'on souhaite immuniser, et en particulier vacciner, en administrant, à cet hôte, une composition immunogène dont le principe actif est un polynucléotide codant pour un polypeptide de la demande ou l'invention, en particulier une composition immunogène à base d'ADN, et plus particulièrement un vaccin à ADN, ou une composition immunogène dont le principe actif consiste en des vésicules membranaires (en particulier des exosomes) comportant un polypeptide de la demande ou l'invention.

Un premier objet de la demande est un polypeptide chimérique caractérisé en ce qu'il comprend ou consiste en les domaines suivants:
- (i) un peptide ou polypeptide d'intérét ;
- (ii) un domaine membranaire ayant la capacité de s'ancrer dans la bicouche lipidique d'une membrane cellulaire; et
- (iii) un domaine cytoplasmique (CD) d'une protéine membranaire, permettant, dans des cellules eucaryotes, l'adressage dudit polypeptide chimérique vers les vésicules membranaires, en particulier vers les vésicules formant des exosomes, et/ou vers le(s) compartiment(s) cellulaire(s) impliqué(s) dans la formation des vésicules membranaires, et en particulier des vésicules formant les exosomes, ou un dérivé muté de ce domaine CD, ce dérivé muté étant défini par la substitution, la délétion et/ou l'insertion d'un ou plusieurs résidu(s) dans la séquence du domaine CD de référence et ce dérivé muté conservant la susdite capacité d'adressage du domaine CD, le domaine CD ou son dérivé muté comprenant au moins un motif YxxL, dans lequel Y représente un résidu tyrosine, x représente un résidu quelconque et L représente un résidu leucine ; et
   étant entendu que lorsque le domaine CD et un domaine membranaire dudit polypeptide chimérique sont dérivés d'une même protéine, alors au moins le domaine CD, son dérivé muté ou le domaine membranaire dérivé de la même protéine que le domaine CD est un dérivé muté par substitution et/ou délétion d'un ou plusieurs résidu(s) dans la séquence du domaine original.

Ledit polypeptide chimérique est en particulier capable d'être sécrété en association avec des vésicules membranaires, en particulier avec des exosomes, lorsqu'il est exprimé dans des cellules eucaryotes appropriées.

L'invention est relative à une vésicule membranaire comportant un polypeptide chimérique qui comprend ou consiste en les domaines suivants :
(i) un peptide ou polypeptide d'intérêt ;
(ii) un domaine membranaire ayant la capacité de s'ancrer dans la bicouche lipidique d'une membrane cellulaire ; et
(iii) le domaine cytoplasmique (CD) de la protéine membranaire (TM) du virus de la leucémie bovine (BLV) ou un dérivé muté de ce domaine CD,
   ledit dérivé muté ayant une capacité d'adressage dudit polypeptide chimérique vers des vésicules membranaires de cellules eucaryotes et/ou vers le(s) compartiment(s) cellulaire(s) impliqué(s) dans la formation de ces vésicules membranaires, et
   la séquence dudit dérivé muté :
   - étant définie par la substitution, la délétion et/ou l'insertion d'un ou plusieurs résidu(s) dans la séquence dudit domaine CD, et
   - étant identique à au moins 60% à la séquence de SEQ ID NO : 8, sous réserve que la séquence dudit dérivé muté comprenne au moins un motif PxxP et au moins un motif YxxL, dans lequel P représente un résidu proline, Y représente un résidu tyrosine, x représente un résidu quelconque
et L représente un résidu leucine,
et sous réserve que, lorsque ledit domaine (iii) comprend ou consiste en le domaine CD de la protéine TM du BLV, ledit domaine (ii) ne comprend pas et ne consiste pas en le domaine membranaire de la protéine TM du BLV.

Les moyens de la demande ainsi que ceux de l'invention concernent tout polypeptide chimérique dans lequel il existe au moins un domaine membranaire positionné entre le peptide ou polypeptide d'intérêt et le domaine CD ou son dérivé muté.

Selon un mode de réalisation préféré, les domaines (i) à (iii) sont positionnés successivement dans l'ordre suivant, de l'extrémité N-terminale vers l'extrémité C-terminale dans le polypeptide chimérique de la demande ou de la vésicule de l'invention: peptide ou polypeptide d'intérêt - domaine membranaire - domaine CD ou son dérivé muté.

Alternativement, les domaines (i) à (iii) sont positionnés par exemple dans l'ordre suivant : domaine CD ou son dérivé muté - domaine membranaire - peptide ou polypeptide d'intérêt.

Le terme « chimérique » désigne ici un polypeptide qui associe plusieurs domaines, d'au moins deux types différents par leur fonction et/ou par leur localisation cellulaire, au moins deux de ces domaines provenant de molécules distinctes, en particulier provenant soit de différentes protéines d'une même espèce ou d'espèces différentes soit d'une même protéine de différentes espèces. Par « domaine » d'une protéine ou d'un polypeptide on entend une région ayant une propriété fonctionnelle pour ladite protéine ou ledit polypeptide. Ainsi, le polypeptide chimérique de la demande ou de la vésicule de l'invention comporte au moins deux types différents de domaines qui sont définis par leur différence de localisation cellulaire, lorsqu'ils sont contenus dans la protéine chimérique: un ou plusieurs domaine(s) membranaire(s), et un domaine cytoplasmique particulier.

Le polypeptide chimérique de la demande ou de la vésicule de l'invention comprend nécessairement au moins un domaine membranaire mais il peut en comprendre plusieurs ; le peptide ou polypeptide d'intérêt pouvant comporter zéro, un ou plusieurs domaines membranaires (voir ci-dessous), le polypeptide chimérique de la demande ou de la vésicule de l'invention comporte un ou plusieurs domaines membranaires. Lorsque ledit polypeptide chimérique ne comporte qu'un seul domaine membranaire, celui-ci peut être ou non dérivé de la même entité, en particulier de la même protéine que le peptide ou polypeptide d'intérêt du polypeptide chimérique de la demande ou de la vésicule de l'invention.

Un polypeptide chimérique de la demande ou de la vésicule de l'invention peut être le produit d'expression d'un polynucléotide recombinant et peut être exprimé par voie recombinante dans une cellule hôte. Ledit polypeptide chimérique est donc par exemple un polypeptide de fusion.

L'expression « vésicule membranaire » désigne au sens de la demande ou invention toute vésicule composée d'une bicouche lipidique renfermant une fraction cytosolique telle que produite par des cellules eucaryotes. Cette expression inclut notamment les vésicules sécrétées dans l'espace extracellulaire, c'est-à-dire les exosomes.

Par « exosomes », on entend, dans la demande ou invention, nanovésicules des membranes cellulaires telles que définies ci-dessus. Ces exosomes peuvent être purifiés à partir des surnageants de culture de cellules par centrifugation différentielle, par ultra-filtration ou par adsorption sur un support ou par tout autre procédé, comme illustré dans les exemples.

L'expression « sécrété en association avec des vésicules membranaires, en particulier avec des exosomes » signifie, dans la demande ou invention, qu'un polypeptide chimérique de la demande ou de la vésicule de l'invention et/ou au moins un de ses produits de dégradation est sécrété dans l'espace extracellulaire, non pas sous forme soluble, mais sous une forme ancrée dans la membrane des vésicules membranaires, en particulier des exosomes. Cet ancrage se fait par l'intermédiaire du ou des domaine(s) membranaire(s) dudit polypeptide chimérique ou du domaine membranaire d'une molécule du CMH (de classe I ou II) à laquelle un produit de dégradation dudit polypeptide chimérique est associé. Dans un polypeptide ainsi ancré dans la membrane d'une vésicule membranaire (en particulier d'un exosome), le peptide ou un polypeptide d'intérêt peut se trouver exposé (en totalité ou en partie) à l'extérieur de ladite vésicule membranaire, inclus (en totalité ou en partie) dans la membrane de ladite vésicule membranaire (c'est le cas lorsque ledit polypeptide ou peptide d'intérêt comporte un ou plusieurs domaines membranaires) et/ou inclus (en totalité ou en partie) dans la fraction cytosolique de ladite vésicule membranaire.

Selon un mode de réalisation particulier, le polypeptide chimérique de la demande ou de la vésicule de l'invention est produit avec et est intégré dans les exosomes avant leur sortie de la cellule.

La sécrétion d'un peptide ou d'un polypeptide en association avec des vésicules membranaires (en particulier des exosomes) requiert notamment (1) l'adressage dudit peptide ou d'un polypeptide au(x) lieu(x) de formation des vésicules membranaires et en particulier des exosomes, et (2) le bourgeonnement vésiculaire à partir de la membrane dans laquelle ledit peptide ou polypeptide est ancré.

Le terme « adressage » encore appelé « tri », « aiguillage » ou « routage intracellulaire » fait référence, dans la demande ou invention, au processus qui permet à un polypeptide, dont la synthèse débute dans le cytosol, d'atteindre les compartiments impliqués dans le bourgeonnement de vésicules membranaires, en particulier des vésicules formant les exosomes et/ou d'atteindre les vésicules membranaires, en particulier les vésicules formant les exosomes.

L'adressage d'un peptide ou d'un polypeptide au(x) lieu(x) de formation des vésicules membranaires et en particulier au(x) lieu(x) de formation des exosomes peut nécessiter notamment que ledit peptide ou polypeptide comprenne un peptide signal d'importation dans le réticulum endoplasmique, comme explicité ci-après, afin que ledit peptide ou polypeptide puisse être inséré dans une membrane cellulaire, la fonction d'ancrage dans la membrane étant assurée par le domaine membranaire.

Le terme « sécrétion » désigne, dans le cadre de la demande ou de l'invention, le processus par lequel des vésicules membranaires, alors appelées « exosomes », sont sécrétées, c'est-à-dire relarguées dans l'espace extracellulaire à partir d'une ou plusieurs cellule(s). Ce processus peut notamment se produire lorsque des endosomes multivésiculaires fusionnent avec la membrane plasmique d'une cellule, relarguant ainsi les vésicules membranaires qu'ils contiennent à l'extérieur de la cellule.

Comme illustré dans les exemples ci-après, un test permettant de mettre en évidence les propriétés d'adressage et de sécrétion d'un polypeptide chimérique de la demande ou de la vésicule de l'invention consiste à vérifier que ledit polypeptide chimérique et/ou ses produits de dégradation se retrouvent bien associés avec des vésicules membranaires (en particulier des exosomes) lorsque ledit polypeptide est exprimé dans des cellules eucaryotes appropriées.

Une cellule eucaryote « appropriée» est avantageusement une cellule eucaryote comportant des vésicules internes de sécrétion, cultivable, capable d'exocytose, modifiable génétiquement et, préférentiellement, dont les vésicules internes peuvent être sécrétées sous l'effet d'une stimulation externe. Il s'agit en particulier d'une cellule de mammifère et plus particulièrement d'une cellule d'origine humaine ou une cellule ayant pour origine un mammifère non humain. Il peut également s'agir de cultures primaires ou de lignées immortalisées. De telles cellules eucaryotes « appropriées » incluent notamment les cellules eucaryotes naturellement capables de produire des exosomes, en particulier les mastocytes, lymphocytes T et B et les cellules dendritiques (par exemple les cellules de Langerhans) ou des cellules dérivées de ces types cellulaires, ainsi que les cellules eucaryotes ou les lignées cellulaires eucaryotes modifiées par génie génétique de façon à les rendre capables de sécréter des exosomes.

Le terme « résidu », tel qu'utilisé dans la demande ou invention, fait référence à un résidu d'acide aminé. Ces résidus sont indiqués en utilisant le code abrégé à une lettre (par exemple, Y pour un résidu tyrosine, L pour un résidu leucine et P pour un résidu proline).

Le terme « bicouche lipidique », désigne la structure de base de la membrane plasmique et de toute membrane biologique, c'est-à-dire tout assemblage de lipides amphiphile en un double feuillet (ou double couche) séparant une cellule ou une vésicule de son environnement et délimitant le cytoplasme d'une cellulaire ou d'une vésicule, ou délimitant les organites à l'intérieur du cytoplasme. Ce terme englobe donc toute membrane de la cellule, c'est-à-dire à la fois la membrane plasmique et les membranes des différents compartiments intracellulaires, en particulier celles du réticulum endoplasmique, celles de l'appareil de Golgi, ou encore celle des vésicules membranaires, par exemple celle des exosomes ou des endosomes.

Un « domaine membranaire », désigne, dans la demande ou invention, tout domaine capable d'interagir avec une bicouche lipidique et en particulier capable de s'ancrer - et ainsi ancrer un polypeptide le comprenant - dans une bicouche lipidique et en particulier dans une membrane cellulaire et dans la membrane d'un exosome. Selon un mode de réalisation particulier, ce domaine membranaire est capable de se lier d'une part à un premier domaine (par exemple un peptide ou un polypeptide d'intérêt) et d'autre part à un deuxième domaine (par exemple un domaine cytoplasmique). Il peut par exemple comporter 10 à 50 résidus, de préférence 15 à 40 résidus et plus préférablement 20 à 30 résidus.

Selon un mode de réalisation particulier, ledit domaine membranaire est un domaine transmembranaire, c'est-à-dire un domaine membranaire traversant entièrement la bicouche lipidique d'une membrane cellulaire. Un domaine transmembranaire est généralement agencé en hélice α hydrophobes, les protéines transmembranaires à traversées multiples pouvant contenir plusieurs, en particulier 2, 3, 4, 5, 6, 7 8, 9 ou 10, voire 20 ou plus de 20 hélices α hydrophobes. Il peut également être agencé en feuillet β. Un ou plusieurs domaines transmembranaires peuvent également adopter une structure en tonneau β transmembranaire, généralement composée de 8 à 22 brins β.

Selon un autre mode de réalisation particulier, ledit domaine membranaire peut ne pas traverser entièrement la bicouche lipidique d'une membrane cellulaire. C'est par exemple le cas du domaine membranaire d'une protéine membranaire en contact avec un seul des compartiments définis par ladite membrane. Ledit domaine membranaire peut alors être par exemple une hélice hydrophobe parallèle au plan de la membrane ou une boucle hydrophobe.

Ledit domaine membranaire peut être dérivé d'une ou de plusieurs protéine(s) membranaire(s), en particulier d'une ou plusieurs protéine(s) transmembranaire(s).

Par « protéine membranaire » on entend toute chaîne polypeptidique comportant un ou plusieurs domaine(s) membranaire(s) tel(s) que défini(s) ci-dessus.

L'expression « protéine transmembranaire » telle qu'utilisée dans la demande ou invention désigne toute chaîne polypeptidique qui traverse au moins une fois entièrement une membrane cellulaire, en particulier la membrane plasmique d'une cellule. Cette protéine, qui est en contact avec les deux compartiments définis par la membrane cellulaire qu'elle traverse (par exemple avec l'espace extracellulaire et le cytosol) comporte donc trois types de domaines, chacun en contact avec un environnement de composition différente (par exemple, lorsque la membrane cellulaire traversée est la membrane plasmique), le(s) ectodomaine(s) (ou domaine(s) extracellulaire(s)), le(s) domaine(s) transmembranaire(s), et le(s) domaine(s) cytoplasmique(s) sont respectivement en contact avec le milieu extracellulaire, les lipides de la membrane plasmique, et le cytosol). Selon un mode de réalisation particulier, la protéine transmembranaire traverse une fois une membrane cellulaire. Selon un autre mode de réalisation particulier, la protéine transmembranaire traverse plusieurs fois, en particulier 2, 3, 4, 5, 6, 7 8, 9 ou 10 fois, voire 20 fois ou plus une membrane cellulaire.

Lesdites protéines membranaires ou transmembranaires peuvent être notamment choisies parmi : des protéines humaines, des protéines d'un animal non humain, des protéines d'un organisme pathogène ou d'un agent pathogène, en particulier des protéines virales, bactériennes, ou des protéines exprimées par un parasite ou une cellule tumorale.

Le ou au moins un des domaine(s) membranaire(s) du polypeptide chimérique de la demande ou de la vésicule de l'invention peut être en particulier celui d'une seule et même protéine membranaire ou être un dérivé muté de ce domaine. Ledit dérivé muté est défini par la substitution, la délétion et/ou l'insertion d'un ou plusieurs résidu(s) dans la séquence du domaine membranaire de référence et conserve la capacité de ce domaine de référence à s'ancrer dans la bicouche lipidique d'une membrane cellulaire. Ledit dérivé muté peut par exemple être obtenu en remplaçant une partie de la séquence du domaine de référence par une séquence dérivée du domaine membranaire d'une autre protéine membranaire.

Le terme « issu d'une protéine particulière » indique, dans la demande ou invention que le peptide ou polypeptide d'intérêt comprend ou consiste en cette protéine particulière, ou en un fragment de ladite protéine particulière.

Un « dérivé muté » d'un domaine membranaire ou cytoplasmique au sens de la demande ou invention fait référence à tout polypeptide ou peptide modifié par rapport au domaine membranaire ou cytoplasmique original ou de référence, sous réserve que ce dérivé muté conserve la fonction d'insertion, d'ancrage dans une bicouche lipidique normalement attachée au domaine membranaire de référence ou la capacité d'adressage normalement attachée au domaine cytoplasmique de référence. Un tel dérivé muté peut donc correspondre par exemple à un fragment composé de résidus contigus dudit domaine membranaire ou cytoplasmique (ce dérivé étant obtenu notamment par délétion et éventuellement substitution d'un ou plusieurs résidu(s) dans la séquence originale) ou, au contraire à un polypeptide de taille plus importante que le domaine membranaire ou cytoplasmique original, en particulier un polypeptide comprenant le domaine membranaire ou cytoplasmique original (ce dérivé étant obtenu notamment par insertion d'un ou plusieurs résidu(s) dans la séquence originale).

Selon un mode de réalisation particulier, le « dérivé muté du domaine membranaire » au sens de la demande ou invention diffère de la séquence originale du domaine membranaire par la substitution d'au moins un résidu, de préférence un, deux, trois, quatre, cinq, voire plus de cinq résidu(s), consécutifs ou non, dans la séquence du domaine membranaire original, ces substitutions pouvant être conservatives, semi-conservatives ou non conservatives, et/ou par la délétion et/ou l'insertion d'au moins un résidu, de préférence un, deux, trois, quatre, cinq, voire plus de cinq résidus, consécutifs ou non, dans la séquence du domaine original. La séquence dudit dérivé muté du domaine membranaire peut présenter au moins 60 ou 70%, en particulier au moins 80%, 90% ou 95%, de similarité ou d'identité avec le domaine membranaire de la protéine membranaire dont elle dérive, par référence à la séquence complète dudit domaine membranaire.

Par « pourcentage d'identité », on entend, dans la demande ou invention, le nombre de résidus identiques rapporté au nombre total de résidus du peptide ou polypeptide étudié. Le « pourcentage de similarité », défini le nombre de résidus identiques ou chimiquement similaires rapporté au nombre total de résidus du peptide ou polypeptide étudié. Le pourcentage d'identité ou de similarité est déterminé en alignant les deux séquences à comparer et en utilisant l'algorithme de Needleman et Wunsch, qui permet un alignement global entre deux séquences. Le pourcentage de similarité ou d'identité est alors calculé sur la totalité de la longueur de ces deux séquences.

Par domaine cytoplasmique (CD), on entend, dans la demande ou invention, un domaine cytoplasmique particulier capable d'être adressé vers les vésicules membranaires, en particulier vers les vésicules formant des exosomes, ou vers le(s) compartiment(s) cellulaire(s) impliqué(s) dans la formation des vésicules membranaires, et en particulier des vésicules formant les exosomes dans des cellules eucaryotes; ce domaine peut ainsi être sécrété dans l'espace extracellulaire en association avec des exosomes, lorsqu'il est exprimé dans des cellules eucaryotes appropriées. Ainsi, ce domaine permet, lorsqu'il est intégré à un polypeptide chimérique comprenant un peptide ou un polypeptide d'intérêt, d'adresser ledit polypeptide chimérique vers les vésicules membranaires et/ou vers leurs(s) lieu(x) de formation et en particulier d'adresser ledit polypeptide chimérique à la membrane de vésicules membranaires, de façon à ce que ce ledit polypeptide puisse être sécrété en association avec des vésicules membranaires (en particulier des exosomes) par une cellule, en particulier une cellule eucaryote appropriée.

Ce domaine CD comprend au moins un motif YxxL, dans lequel x représente un résidu quelconque. Il peut en particulier comprendre un, deux ou trois motifs YxxL.

Ledit motif ou un des motifs YxxL du domaine CD peut être, par exemple, le motif YINL (SEQ ID NO : 91) ou YSHL (SEQ ID NO : 97). Selon un mode de réalisation particulier, le domaine CD comprend un motif DYxxL, dans lequel x représente un résidu quelconque. A titre d'exemple illustré dans la partie expérimentale, on citera le motif DYINL (SEQ ID NO : 93).

Alternativement ou de façon complémentaire, le domaine CD comprend au moins un motif équivalent à un motif YxxL, par exemple un motif YxxF, dans lequel x représente un résidu quelconque. Par exemple, le récepteur de la transférine, qui est une protéine cellulaire, comporte un tel domaine.

Alternativement ou de façon complémentaire, le domaine CD comprend au moins un motif équivalent à un motif DYxxL, par exemple un motif DYxxF, dans lequel x représente un résidu quelconque.

La description de la demande ou invention, faite par référence au domaine YxxL en général ou tel qu'illustré par des séquences particulières, vaut également pour les domaines DYxxL ou DYxxF, définis en termes généraux ou ayant des séquences spécifiques dérivées des exemples donnés de motifs.

Selon un mode de réalisation préféré, ledit domaine CD comprend en outre au moins un, en particulier un, deux, trois ou quatre motif(s) PxxP, dans lequel x représente un résidu quelconque. Selon un mode de réalisation particulier, le motif PxxP ou un des motifs PxxP du domaine CD est le motif PSAP (SEQ ID NO : 88) ou PTAP (SEQ ID NO : 89).

La description générale ou spécifique des polypeptides chimériques de la demande ou de la vésicule de l'invention, faisant mention d'un ou plusieurs motifs YxxL, s'applique donc également aux polypeptides chimériques qui comprennent en outre un motif PxxP.

Selon un mode de réalisation particulier, le domaine CD comprend au moins un motif YxxL ou DYxxL (par exemple le motif YINL, YSHL ou DYINL) et un motif PxxP (par exemple le motif PSAP ou PTAP).

En particulier, le domaine CD consiste en une séquence ayant un motif PxxP et un motif YxxL (ou YxxF, DYxxL ou DYxxF). Dans un mode de réalisation particulier de la demande ou l'invention, le motif PxxP du domaine CD est PSAP et le motif YxxL est YINL ou YSHL.

Dans un mode de réalisation particulier, le motif YxxL du domaine CD (par exemple le motif YINL ou YSHL), ou un des motifs est YxxL, localisé en position C-terminale par rapport au motif PxxP (par exemple le motif PSAP).

Dans un mode de réalisation particulier, le motif YxxL du domaine CD (par exemple le motif YINL ou YSHL) est localisé en position N-terminale par rapport au motif PxxP (par exemple le motif PSAP).

Parmi les protéines ayant un domaine CD comportant au moins un motif YxxL, on trouve notamment des protéines cellulaires et des protéines virales. Ces protéines virales sont notamment des protéines de virus enveloppés, en particulier les glycoprotéines TM de virus enveloppés tels que définis ci-dessus, ou des protéines d'herpèsvirus, par exemple la protéine LMP2-A, du virus d'Epstein-Barr qui comporte au moins deux motifs YxxL.

Selon un mode de réalisation particulier, le domaine CD est celui d'une protéine TM d'un rétrovirus. Le terme « rétrovirus » couvre ici tout rétrovirus. Ce rétrovirus peut être notamment choisi parmi les deux sous-familles de rétrovirus suivantes, établies sur la base de critères de pathogénicité :
- les Oncovirinae (Oncovirus), qui sont des virus transformants et qui incluent notamment le BLV, qui induit, chez les bovins, une prolifération des cellules B pouvant engendrer une leucémie, et le virus de la leucémie-lymphome à cellules T (ou HTLV-1, pour human T cell leukemia virus) ;
- les lentivirinae (Lentivirus), qui sont des virus cytopathogènes, et qui incluent notamment les virus de l'immunodéficience humaine VIH-1 et VIH-2.

A titre d'exemple, le domaine CD peut être celui d'une protéine TM d'un rétrovirus choisi parmi le virus de la leucémie bovine (BLV), un virus d'immunodéficience humaine (VIH), en particulier un VIH de type 1 ou un VIH de type 2, le virus de la leucémie-lymphome à cellules T (HTLV), en particulier le HTLV-1 ou le HTLV-2, et le virus de singe Mason-Pfizer (MPMV).

Selon un mode de réalisation particulier, le domaine CD est celui de la protéine TM du BLV. Ce domaine CD du BLV, qui est composé de 58 résidus, a pour séquence SEQ ID NO : 6. Le domaine (iii) du polypeptide chimérique de la demande ou de la vésicule de l'invention correspond alors soit au domaine de séquence SEQ ID NO : 6, soit à un dérivé muté du domaine de séquence SEQ ID NO: 6.

Dans le cas particulier où le domaine (iii) comprend ou consiste en le domaine CD la protéine TM du BLV, de séquence SEQ ID NO: 6, le(s) domaine(s) membranaire(s) dudit polypeptide chimérique de la demande ou de la vésicule de l'invention est(sont) dépourvu(s) du domaine membranaire de la protéine TM du BLV, qui a pour séquence SEQ ID NO: 4. Un domaine membranaire du polypeptide chimérique de la demande ou de la vésicule de l'invention peut en revanche correspondre à un dérivé muté du domaine de séquence SEQ ID NO : 4, ledit dérivé muté ne comprenant pas la séquence SEQ ID NO : 4. Un tel dérivé muté peut être obtenu, par délétion et éventuellement substitution d'un ou plusieurs résidu(s) dans la séquence SEQ ID NO : 4, comme illustré dans les exemples.

Le « dérivé muté du domaine CD » au sens de la demande ou invention comprend au moins un motif YxxL, dans lequel x représente un résidu quelconque. Il peut en particulier comprendre un, deux ou trois motif(s) YxxL. Ce dérivé muté peut en outre comprendre au moins un, en particulier un, deux, trois ou quatre motif(s) PxxP, dans lequel x représente un résidu quelconque. Ce dérivé muté diffère du domaine cytoplasmique original par la substitution d'au moins un résidu, de préférence un, deux, trois, quatre, cinq, voire plus de cinq résidus, consécutifs ou non, dans la séquence du domaine cytoplasmique original, ces substitutions pouvant être conservatives, semi-conservatives ou non conservatives, et/ou par la délétion et/ou l'insertion d'au moins un résidu, de préférence un, deux, trois, quatre, cinq, voire plus de cinq résidus, consécutifs ou non, dans la séquence du domaine cytoplasmique original.

Dans un mode de réalisation particulier de la demande ou l'invention, le dérivé muté du domaine CD est un fragment de domaine CD natif constitué essentiellement par une séquence d'acides aminés s'étendant du motif PSAP jusqu'au motif YxxL (par exemple YINL ou YSHL) qui, dans un mode de réalisation particulier, le suit en partie C-terminale. Le cas échéant, un ou plusieurs résidus d'acide aminé naturellement présent dans le domaine CD, entre ces deux motifs, est substitué ou délété.

Selon un mode de réalisation particulier, le dérivé muté du domaine CD comprend au moins un motif YxxF ou DYxxL (par exemple le motif YINL, YSHL ou DYINL) et un motif PxxP (par exemple le motif PSAP ou PTAP), dans lequel x représente un résidu quelconque.

Selon un mode de réalisation particulier, le domaine (iii) du polypeptide chimérique de la demande ou de la vésicule de l'invention, en particulier le dérivé muté du domaine CD, est dépourvu de la séquence KCLTSRLLKLLRQ. Ainsi, le dérivé muté du domaine CD peut différer du domaine CD original en ce que sa séquence est dépourvue de la séquence : KCLTSRLLKLLRQ.

En outre, ou alternativement, la séquence du dérivé muté du domaine CD peut présenter au moins 60 ou 70%, en particulier au moins 80%, 90% ou 95%, de similarité ou d'identité avec la séquence du domaine CD original dépourvue de l'enchaînement KCLTSRLLKLLRQ.

Il peut également être préférable que le domaine (iii) du polypeptide chimérique de la demande ou de la vésicule de l'invention, et en particulier le dérivé muté du domaine CD soit dépourvu de la séquence PC et/ou de la séquence CP. Selon un mode de réalisation particulier, ledit domaine (iii), en particulier le dérivé muté du domaine CD est notamment dépourvu de la séquence PCP. Ainsi, si le domaine CD original contient une séquence PCP, son dérivé muté peut être obtenu notamment en délétant, dans la séquence du domaine CD original, le résidu cystéine de ladite séquence PCP, ou en le substituant par un autre résidu, de préférence par un résidu non palmitoylable, par exemple un résidu alanine.

Selon un mode de réalisation particulier, le domaine CD ou son dérivé est dépourvu à la fois de la séquence PCP et de l'enchaînement KCLTSRLLKLLRQ.

Lorsque le ou un des domaine(s) membranaire(s) et cytoplasmique(s) dérivent d'une même protéine, notamment lorsqu'ils dérivent de la protéine TM du BLV, il peut être préférable d'utiliser, en tant que domaine (iii), un dérivé muté du domaine CD dépourvu de la séquence PCP et/ou dépourvu de la séquence KCLTSRLLKLLRQ.

Dans le cas particulier où un des domaines membranaires du polypeptide chimérique de la demande ou de la vésicule de l'invention comprend ou consiste en le domaine transmembranaire de la protéine TM du BLV, de séquence SEQ ID NO : 4, le domaine CD ou son dérivé muté est dépourvu du domaine CD de la protéine TM du BLV, de séquence SEQ ID NO : 6. Le domaine CD ou son dérivé muté peut en revanche correspondre à, ou comprendre un dérivé muté du domaine de séquence SEQ ID NO : 6. Comme illustré dans les exemples, un tel dérivé muté peut être obtenu, par exemple, en délétant des résidus situés dans la partie N-terminale de la séquence SEQ ID NO : 6, en particulier en délétant la séquence KCLTSRLLKLLRQ et/ou en délétant la séquence PCP dans la séquence SEQ ID NO : 6, ou en substituant par exemple le résidu cystéine de la séquence PCP un autre résidu, de préférence par un résidu non palmitoylable, par exemple un résidu alanine.

A titre d'exemple, le dérivé muté du domaine CD la protéine TM du BLV peut comprendre ou consister en la séquence SEQ ID NO : 8, qui correspond à la séquence SEQ ID NO.6 dans laquelle les 13 résidus N-terminaux ont été délétés.

A titre d'exemple également, la séquence du dérivé muté de la protéine TM du BLV peut différer de la séquence du domaine CD la protéine TM du BLV par la substitution d'au moins un résidu, de préférence un, deux, trois, quatre, cinq, voire plus de cinq résidu(s), consécutifs ou non, et/ou par la délétion et/ou l'insertion d'au moins un résidu, de préférence un, deux, trois, quatre, cinq, voire plus de cinq résidu(s), consécutifs ou non dans la séquence dudit domaine correspondant à la séquence SEQ ID NO : 8.

Encore à titre d'exemple, la séquence du dérivé muté du domaine CD de la protéine TM du BLV (domaine CD de séquence SEQ ID NO.6) peut présenter au moins 60 ou 70%, en particulier au moins 80%, 90% ou 95%, de similarité ou d'identité avec la séquence SEQ ID NO : 8. De préférence, la séquence dudit dérivé muté conserve notamment le motif YINL, YSHL (ou, le cas échant, DYINL) et le motif PSAP de la séquence SEQ ID NO : 8.

La séquence dudit dérivé muté du domaine CD de séquence SEQ ID NO.6 est, de préférence, dépourvue de motif PC (proline - cystéine) et CP (cystéine - proline) et, plus préférablement dépourvue de motif PCP (proline - cystéine - proline). Par exemple, il peut comprendre ou consister en la séquence SEQ ID NO : 10 ou SEQ ID NO : 12.

Selon un mode de réalisation particulier, le domaine CD ou son dérivé muté, en particulier le dérivé muté du domaine CD de séquence SEQ ID NO : 6, comprend ou consiste en une séquence choisie parmi les séquences suivantes:
PxxPxxxxPxxPxSxYxxLxPxxPExYxxLxPxxPDYxxL ;
PxxPxₙPxxPxₙSxYxxLXₙPxxPExₙYxxLxₙPxxPDYxxL ;
PxxPxxxxPxxPxSxYxxLxPxxPExYxxLxPxxPDYxxLxxxx ; et
PxxPxₙPxxPxₙSxYxxLXₙPxxPExₙYxxLxₙPxxPDYxxLxxxx ;
dans lesquelles x et xₙ représentent respectivement un résidu quelconque et un ou plusieurs résidu(s) quelconque(s).

Selon un mode de réalisation particulier, le domaine CD ou son dérivé muté, en particulier le dérivé muté du domaine CD de séquence SEQ ID NO.6, comprend ou consiste en une séquence choisie parmi les séquences suivantes:
PxxPxxxxxxxxxxxxYxxL;
PxxPxxxxxxxxxxxDYxxL ;
PxxPxxYxxxxxxxxxYxxL ;
PxxPxxYxxxxxxxxDYxxL ;
PxxPExYxxLxPxxPDYxxL ;
PxxPxₙYxxL ;
PxxPxₙDYxxL ;
PxxPxₙYxₙYxxL;
PxxPxₙYxₙDYxxL ;
PxxPEXₙYxxLxₙPxxPDYxxL ;
PxxPxxxxPxxPxxxYxxLXPxxPEXYxxLXPxxPDYxxL ;
PxxPxₙPxxPxₙYxxLxₙPxxPEXₙYxxLxₙPxxPDYxxL ;
PxxPxxxxPxxPxxxYxxLxPxxPExYxxLxPxxPDYxxLxxxx ; et
PxxPxₙPxxPxₙYxxLXₙPxxPEXₙYxxLxₙPxxPDYxxLxxxx.
dans lesquelles x et xₙ représentent respectivement un résidu quelconque et un ou plusieurs résidu(s) quelconque(s).

Selon un mode de réalisation particulier de l'invention, la séquence dudit domaine CD comprend, ou la séquence dudit dérivé muté de domaine CD comprend ou consiste en une séquence choisie parmi les séquences suivantes:
PxxPxxxxPxxPxxxYxxLxPxxPExYxxLxPxxPDYxxL ;
PxxPxₙPxxPxₙYxxLXₙPxxPEXₙYxxLxₙPxxPDYxxL ;
PxxPxxxxPxxPxxxYxxLxPxxPExYxxLxPxxPDYxxLxxxx ; et
PxxPxₙPxxPXₙYxxLxₙPxxPEXₙYxxLxₙPxxPDYxxLxxxx,
dans lesquelles x et xₙ représentent respectivement un résidu quelconque et un ou plusieurs résidu(s) quelconque(s).

En particulier n est supérieur ou égal à 1 et inférieur à 50. n peut avoir en particulier toute valeur entre 1 et 20.

A titre d'exemple, le motif PxxP qui, dans un mode de réalisation particulier, se trouve en position N-terminale dans les séquences indiquées ci-dessus, peut être le motif PSAP ou PTAP.

Alternativement ou de façon complémentaire, le motif YxxL qui, dans un mode de réalisation particulier, se trouve en position C-terminale dans les séquences indiquées ci-dessus, peut être par exemple le motif YINL ou YSHL.

Dans un mode de réalisation particulier de la demande ou l'invention, lorsque le domaine CD ou son dérivé muté comprend l'une des séquences ci-dessus, les résidus d'acides aminés consécutifs ajoutés en amont ou en aval de cette séquence ne forment pas un motif PxxP ni un motif YxxL, YxxF, DYxxL ou DYxxF.

Selon un mode de réalisation particulier, ledit dérivé muté du domaine CD de séquence SEQ ID NO.6 comporte 6 à 100 résidus, en particulier 20 à 80, 30 à 70 ou 40 à 60, par exemple 40, 41, 42, 43, 44, 45, 46, 47, 48, 49 ou 50 résidus.

A titre d'exemple, la séquence du dérivé muté du domaine CD peut comprendre ou consister en la séquence SEQ ID NO. 30, SEQ ID NO. 42, SEQ ID NO. 44 ou SEQ ID NO. 95 ou présenter au moins 60 ou 70%, en particulier au moins 80%, 90% ou 95%, de similarité ou d'identité avec la séquence SEQ ID NO : 30, SEQ ID NO : 42, SEQ ID NO : 44 ou SEQ ID NO: 95 par référence respectivement à la séquence SEQ ID NO : 30, SEQ ID NO : 42, SEQ ID NO : 44 ou SEQ ID NO : 95 complète.

Par « peptide ou polypeptide d'intérêt » on entend tout un enchaînement de plusieurs (au moins deux) résidus successifs, formant la structure d'un peptide ou d'un polypeptide. Un peptide désigne une chaîne de 2 à 20 résidus successifs (en particulier 2, 3, 4, 5, 6, 7, 8, 9, 10, 11, 12, 13, 14, 15, 16, 17, 18, 19 ou 20 résidus), en particulier une chaîne de 5 à 10, de 10 à 15 ou de 15 à 20 résidus successifs. Un polypeptide, qui désigne également une protéine ou un fragment d'une protéine, est un enchaînement de plus de 20 (au moins 21) résidus successifs, en particulier une chaîne de 21 à 1000 résidus successifs, de préférence de 21 à 500, 21 à 250 ou 21 à 150 résidus successifs, par exemple 21 à 50, 50 à 100, 100 à 150 résidus successifs. Ledit peptide ou polypeptide d'intérêt peut être en particulier comprendre ou consister en un ou plusieurs domaines d'une protéine soluble, membranaire, transmembranaire et/ou multimérique.

Selon un mode de réalisation particulier, ledit peptide ou polypeptide d'intérêt comprend ou consiste en un ou plusieurs domaine(s) d'une protéine extracellulaire ou un ou des fragment(s) d'un ou plusieurs de ce(s) domaine(s).

Selon un mode de réalisation particulier, ledit peptide ou polypeptide d'intérêt comprend ou consiste en un ou plusieurs ectodomaine(s) et/ou un ou plusieurs domaine(s) membranaire(s) et/ou un ou plusieurs domaines(s) cytoplasmique(s) d'une protéine membranaire, en particulier d'une protéine transmembranaire, ou un ou plusieurs fragment(s) d'un ou plusieurs de ce(s) domaine(s).

Selon un mode de réalisation particulier, ledit peptide ou polypeptide d'intérêt comprend ou consiste en un ou plusieurs domaine(s) d'une protéine cytosolique ou un ou des fragment(s) de ce(s) domaine(s).

Par « fragment » d'un domaine on entend, dans la demande ou invention, une portion composée d'au moins 6 résidus contigus dudit domaine, et en particulier une portion présentant au moins 50%, de préférence au moins 60%, 70%, 80%, ou au moins 90%, voire 100% d'identité avec la séquence complète dudit domaine. Quoi qu'il en soit, un fragment est de taille inférieure à la taille de la protéine dont il dérive.

Selon un mode de réalisation particulier, ledit peptide ou polypeptide d'intérêt est antigénique, c'est-à-dire qu'il est capable d'éliciter une réponse immunitaire dirigée contre ledit peptide ou polypeptide d'intérêt. Ledit peptide ou polypeptide d'intérêt peut en particulier comprendre ou consister en un ou plusieurs épitope(s) d'une protéine.

Selon un mode de réalisation particulier, ledit peptide ou polypeptide d'intérêt provient d'un organisme pathogène, par exemple un virus, une bactérie ou un parasite, ou d'un agent pathogène, par exemple une cellule tumorale, une toxine.... Tout composant peptidique d'un tel organisme ou agent pathogène peut être utilisé, qu'il s'agisse ou non d'une protéine de structure. Ce peut être notamment un antigène de pathogène, et en particulier un antigène viral, bactérien ou un antigène provenant d'un parasite.

Ledit peptide ou polypeptide d'intérêt peut également provenir d'un antigène tumoral, d'un antigène cytoplasmique, d'une protéine transmembranaire, notamment d'une intégrine ou d'un co-récepteur ou d'une protéine intervenant dans des interactions (en particulier les protéines ICAM, CD4, CD8), d'un récepteur de ligand, notamment d'un récepteur à un seul domaine membranaire, par exemple un récepteur de cytokine (en particulier les récepteurs de la famille HER répondant à l'EGF, par exemple le récepteur EGF-R1), notamment d'un récepteur à multiples domaines membranaires, par exemple un récepteur à sept domaines transmembranaires (en particulier le récepteur CXCR4 du VIH ou un récepteur de l'acide gamma amino butyrique (GABA)), ou d'un ligand de récepteur, notamment d'une cytokine ou d'un fragment de ceux-ci.

En fonction du type de peptide ou de polypeptide d'intérêt choisi, les moyens de la demande ou de l'invention pourront être utilisés *in vivo,* notamment en immunisation, en particulier en vaccination, et/ou *in vitro,* par exemple pour cribler des molécules interagissant avec ledit peptide ou polypeptide d'intérêt (comme décrit ci-après).

Selon un mode de réalisation particulier, ledit peptide ou polypeptide d'intérêt provient d'une protéine présente à la surface d'un virus, par exemple une protéine responsable de la fixation d'une particule virale à un récepteur situé sur une cellule cible et/ou responsable de la fusion de l'enveloppe virale ou de la membrane plasmique d'une cellule infectée avec la membrane plasmique d'une cellule cible, ou un fragment d'une telle protéine.

A titre d'exemple, on peut utiliser, en tant que domaine (i), une protéine de l'enveloppe d'un virus enveloppé ou un fragment de cette protéine. Ce virus enveloppé peut être notamment choisi parmi les familles suivantes :
- les Poxviridae, en particulier ceux du genre Orthopoxvirus, qui comprend notamment le virus de la variole et le virus de la vaccine ;
- les Herpesviridae, en particulier ceux du genre Herpesvirus, qui comprend notamment les Herpesvirus de types 1 et 2, le virus de la Varicelle, le virus d'Epstein Barr, le Cytomégalovirus, et les Herpesvirus de types 6, 7, 8 ;
- les Hepadnaviridae, qui comprennent notamment le virus de l'hépatite B ;
- les Orthomyxoviridae, en particulier ceux du genre virus influenza A, B ou C, qui comprend notamment le virus de la grippe aviaire H5N1 ;
- les Paramyxoviridae, en particulier ceux du genre Paramyxovirus, qui comprend notamment les virus parainfluenzae et le virus des oreillons, ceux du genre Morbillivirus, qui comprend notamment les virus de la rougeole, et ceux du genre Pneumovirus, qui comprend notamment le virus respiratoire syncytial ;
- les Rhabdoviridae, en particulier ceux du genre Lyssavirus, qui comprend notamment le virus de la rage ;
- les Filoviridae, qui comprennent notamment le virus de Marburg et le virus Ebola ;
- les Togaviridae, en particulier ceux du genre Flavivirus, qui comprend notamment le virus de la fièvre jaune et le virus de l'hépatite C (HCV), ceux du genre Alphavirus et ceux du genre Rubivirus, qui comprend notamment le virus de la rubéole ;
- les Coronaviridae, en particulier ceux du genre Coronavirus, qui comprend notamment des virus responsables d'infections respiratoires et digestives telles que le syndrome respiratoire aigu sévère (SRAS);
- les Arenaviridae, en particulier ceux du genre Arenavirus, qui comprend notamment le virus de Lassa ;
- les Bunyaviridae, en particulier ceux du genre Bunyavirus, Hantavirus, Phlebovirus ; et
- les Retroviridae, en particulier ceux du genre Lentivirus qui comprend notamment les virus d'immunodéficience humaine (VIH).

Encore à titre d'exemple, le peptide ou polypeptide d'intérêt peut être issu d'une protéine de l'enveloppe d'un virus de la grippe, en particulier de l'hémagglutinine (HA) d'un virus de la grippe, et plus particulièrement de la protéine HA du virus de la grippe aviaire H5N1. Ce peut être l'ectodomaine de cette protéine ou un fragment de cet ectodomaine ou un fragment comprenant ou consistant en un ou plusieurs épitope(s) de cet ectodomaine.

D'autres polypeptides antigéniques de ces virus peuvent naturellement être utilisés, tels que la protéine ou un fragment de la polyprotéine GAG du VIH, la protéine ou un fragment de capside du poliovirus ou d'un papillomavirus.

A titre d'exemple également, le peptide ou polypeptide d'intérêt peut être issu d'une protéine de l'enveloppe externe de l'un de ces virus. Il peut s'agir de l'enveloppe de l'un de ces virus. Il peut s'agir de l'enveloppe d'un virus d'un coronavirus, en particulier de la protéine Spike (S) d'un coronavirus, et plus particulièrement la protéine Spike d'un coronavirus du syndrome respiratoire aigu sévère (coronavirus du SRAS ou SARS-CoV en anglais). Ce peptide ou polypeptide d'intérêt peut en particulier comprendre ou consister en l'ectodomaine de cette protéine ou un fragment comprenant ou consistant en un ou plusieurs épitope(s) de cet ectodomaine.

Selon un mode de réalisation préféré, ledit peptide ou polypeptide d'intérêt se trouve dans sa conformation native dans le polypeptide de la demande ou l'invention.

Selon un mode de réalisation particulier, le polypeptide chimérique de la demande ou de la vésicule de l'invention comprend en outre au moins une molécule de liaison (ou linker). Le terme « linker » désigne tout élément permettant de lier deux domaines successifs. Celui-ci peut-être de longueur et de nature variable.

Selon un mode de réalisation particulier, au moins deux domaines successifs du polypeptide de la demande ou de la vésicule de l'invention sont liés de façon covalente, par exemple par l'intermédiaire de liaisons peptidiques. Ainsi, selon un mode de réalisation particulier, ledit linker est un polypeptide ou un peptide. Ce linker polypeptidique peut consister en une séquence de 2 à 50 résidus, de préférence de 2 à 30 résidus, par exemple 2 à 5, 5 à 10, 10 à 20 ou 20 à 30 résidus successifs.

Selon un mode de réalisation particulier, la séquence nucléotidique codant pour ledit linker comprend ou consiste en un site de restriction. Par « site de restriction », on entend une séquence nucléotidique particulière reconnue par une enzyme de restriction de type Il comme un site de coupure dans la molécule d'ADN. Ainsi, ce linker peut-être, par exemple, le linker de séquence RSR, codé par la séquence nucléotidique AGGTCTAGA, qui comprend le site de restriction de l'enzyme de restriction Xbal (séquence TCTAGA).

Selon un mode de réalisation particulier, le polypeptide chimérique de la demande ou de la vésicule de l'invention comporte un (ou plusieurs) peptide signal d'importation dans le réticulum endoplasmique.

Par « peptide signal d'importation dans le réticulum endoplasmique», on entend une petite séquence polypeptidique continue d'environ 5 à environ 60 résidus, en particulier de 15 à 60 résidus et plus particulièrement de 15 à 30 résidus, qui permet le passage d'une protéine le comportant à travers la membrane du réticulum endoplasmique, le passage de la protéine pouvant être complet ou être partiel, l'arrêt du passage de la protéine dépendant de la présence d'un autre signal (ou d'autres signaux) additionnel(s). Les peptides signaux pour une même destination étant interchangeables d'une protéine à une autre, tout peptide signal permettant l'adressage d'une protéine au réticulum endoplasmique peut-être utilisé dans le cadre de la demande ou invention.

A titre d'exemple, on peut utiliser en tant que peptide signal d'importation dans le réticulum endoplasmique un peptide de 27 résidus de séquence SEQ ID NO: 2.

A titre d'exemple également, on pourrait également utiliser en tant que peptide signal d'importation dans le réticulum endoplasmique le peptide signal d'une protéine membranaire telle que les protéines CD4, CD8 et hémagglutinine (HA), le peptide signal d'un récepteur de cytokine tel que IL1 R1, EGFR1 (HER1), HER2, HER3 ou HER4, ou encore, le peptide signal d'une protéine sécrétée, par exemple, celui d'une cytokine. Ainsi, on peut utiliser un peptide signal choisi parmi : celui de la protéine CD4 humaine (peptide de séquence SEQ ID NO : 49) ou CD4 de souris (peptide de séquence SEQ ID NO : 50), celui de la protéine CD8 alpha de souris (peptide de séquence SEQ ID NO: 51), CD8 alpha bovine (peptide de séquence SEQ ID NO: 52), CD8 alpha humaine (peptide de séquence SEQ ID NO : 53), ou CD8 alpha de rat (peptide de séquence SEQ ID NO : 54), celui des récepteurs IL1 R1 humain (peptide de séquence SEQ ID NO : 55), EGFR1 (HER1) humain (peptide de séquence SEQ ID NO: 56), HER2 humain (peptide de séquence SEQ ID NO: 57), HER3 humain (peptide de séquence SEQ ID NO : 58) ou HER4 humain (peptide de séquence SEQ ID NO : 59), ou celui des cytokines IL-2 de souris (peptide de séquence SEQ ID NO : 60), IL-6 de souris (peptide de séquence SEQ ID NO : 61), IL-7 humaine (peptide de séquence SEQ ID NO : 62), IL-10 de souris (peptide de séquence SEQ ID NO : 63), ou MIP-1-alpha chimiokine humaine (peptide de séquence SEQ ID NO : 64), celui de l'hémagglutinine du virus Influenza B (peptide de séquence SEQ ID NO : 65), celui de l' hémagglutinine des virus Influenza A H1N1 (peptide de séquence SEQ ID NO : 66), Influenza A H2N2 (peptide de séquence SEQ ID NO : 67), Influenza A H3N2 (peptide de séquence SEQ ID NO : 68), Influenza A H4N6 (peptide de séquence SEQ ID NO : 69), Influenza A H5N1 (peptide de séquence SEQ ID NO: 70), Influenza A H6N5 (peptide de séquence SEQ ID NO: 71), Influenza A H7N7 (peptide de séquence SEQ ID NO : 72), Influenza A H8N4 (peptide de séquence SEQ ID NO : 73), Influenza A H9N2 (peptide de séquence SEQ ID NO : 74), Influenza A H10N7 (peptide de séquence SEQ ID NO : 75), Influenza A H11 N6 (peptide de séquence SEQ ID NO : 76), Influenza A H12N5 (peptide de séquence SEQ ID NO: 77), ou Influenza A H13N6 (peptide de séquence SEQ ID NO : 78).

Selon un autre mode de réalisation particulier, ledit peptide signal d'importation dans le réticulum endoplasmique peut faire partie du peptide ou polypeptide d'intérêt du polypeptide chimérique de la demande ou de la vésicule de l'invention. C'est par exemple le cas lorsque ledit peptide ou polypeptide d'intérêt est une protéine membranaire ou une cytokine.

Indépendamment ou en combinaison avec le mode de réalisation ci-dessus, ledit peptide signal d'importation dans le réticulum endoplasmique peut faire partie d'un des domaines membranaires du polypeptide chimérique de la demande ou de la vésicule de l'invention. Cela peut être par exemple le cas lorsque le domaine membranaire ou un des domaines membranaires dudit polypeptide est dérivé d'un récepteur à sept domaines transmembranaires ; il est en effet connu dans l'état de la technique que pour certains récepteurs à sept domaines transmembranaires tels que le récepteur CXCR4, le domaine transmembranaire en position N-terminale fait office de signal d'importation dans le réticulum endoplasmique.

Alternativement, selon un autre mode de réalisation, ledit peptide signal d'importation ne fait partie d'aucun des trois domaines (i), (ii) et (iii), et est, par conséquent, ajouté en plus des trois domaines (i), (ii) et (iii) dudit polypeptide chimérique. Il peut alors être placé à différentes localisations dans la séquence linéaire du polypeptide chimérique de la demande ou de la vésicule de l'invention, mais se trouve généralement à une extrémité dudit polypeptide et est de préférence en position N-terminale dans ledit polypeptide.

Si un deuxième peptide signal d'importation dans le réticulum endoplasmique est ajouté dans le polypeptide chimérique de la demande ou de la vésicule de l'invention, il permet le cas échéant d'augmenter le ciblage membranaire.

Sous une forme mature, en particulier lorsqu'il est ancré dans la membrane d'une vésicule membranaire, par exemple d'un exosome, le polypeptide chimérique de la demande ou de la vésicule de l'invention ne comporte généralement pas ou plus de peptide signal N- ou C- terminal d'importation dans le réticulum endoplasmique; après qu'il a rempli sa fonction, le peptide signal N-ou C- terminal peut être est séparé du polypeptide par un clivage protéolytique, par exemple dans le réticulum endoplasmique ou dans le Golgi.

Selon un mode de réalisation préféré, ledit peptide ou polypeptide d'intérêt se trouve dans sa conformation native dans le polypeptide de la demande ou de la vésicule de l'invention.

Selon un mode de réalisation particulier, ledit polypeptide chimérique est multimérique et en particulier est sous la forme d'un dimère ou d'un trimère. Ceci peut être le cas, en particulier, lorsque le peptide ou le polypeptide d'intérêt provient d'une protéine qui, sous sa forme native, se dimérise ou se trimérise.

Selon un mode de réalisation particulier, le polypeptide chimérique comprend en outre une séquence tag, qui permet de le purifier. Par exemple, ladite séquence tag peut comprendre ou consister en une pluralité de résidus histidine liés consécutivement, en particulier une séquence de 6 résidus histidine consécutifs.

Selon un mode de réalisation particulier, le polypeptide chimérique comprend en outre un marqueur permettant de détecter le polypeptide chimérique par ELISA ou en Western Blot. De préférence, ledit marqueur est un épitope reconnu par un anticorps monoclonal spécifique, par exemple un épitope myc.

La demande ainsi que l'invention concernent tout particulièrement une population de polypeptides chimériques en particulier de polypeptides chimériques essentiellement glycosylés au stade terminal, c'est-à-dire sous forme mature, par suite de leur ciblage et association aux exosomes. Une telle population est avantageusement dépourvue de polypeptides glycosylés à des stades précoces ou intermédiaires de glycosylation.

Dans un mode de réalisation particulier de la demande ou l'invention, dans les polypeptides chimériques, le peptide ou polypeptide d'intérêt a avantageusement sa conformation native (en particulier est sous forme de multimère, par exemple sous forme de dimère ou de trimère) lorsqu'il est associé aux exosomes.

La demande est également relative à un polypeptide permettant la sécrétion d'un peptide ou polypeptide d'intérêt auquel il est associé, en association avec des vésicules membranaires (en particulier des exosomes) lorsqu'il est exprimé dans une cellule eucaryote, caractérisé en ce qu'il comprend ou consiste en un dérivé muté du domaine CD de la protéine TM d'un rétrovirus, ce dérivé muté étant défini par la substitution, la délétion et/ou l'insertion d'un ou plusieurs résidu(s) dans la séquence du domaine CD de référence et comprenant au moins un motif YxxL, dans lequel Y représente un résidu tyrosine, x représente un résidu quelconque et L représente un résidu leucine et ce dérivé muté conservant la capacité dudit domaine CD à adresser un peptide ou polypeptide d'intérêt vers les vésicules membranaires, en particulier vers les vésicules formant des exosomes, et/ou vers le(s) compartiment(s) cellulaire(s) impliqué(s) dans la formation des vésicules membranaires, et en particulier des vésicules formant les exosomes.

L'invention a également pour objet un polypeptide permettant la sécrétion d'un peptide ou polypeptide d'intérêt auquel il est associé, en association avec des vésicules membranaires, en particulier avec des exosomes, lorsqu'il est exprimé dans une cellule eucaryote, caractérisé en ce qu'il comprend ou consiste en un dérivé muté du domaine cytoplasmique (CD) de la protéine transmembranaire (TM) du virus de la leucémie bovine (BLV), de séquence SEQ ID NO : 6, où ledit dérivé muté
a) est défini par la substitution, la délétion et/ou l'insertion d'un ou plusieurs résidu(s) dans la séquence du domaine cytoplasmique (CD) de la protéine transmembranaire (TM) du virus de la leucémie bovine (BLV) de séquence SEQ ID NO: 6,
b) comprend au moins un motif PxxP et au moins un motif YxxL, dans lesquels P représente un résidu proline, Y représente un résidu tyrosine, x représente un résidu quelconque, et L représente un résidu leucine ;
c) conserve la capacité du domaine CD de la protéine TM du BLV à adresser le peptide ou polypeptide d'intérêt auquel il est associé vers les vésicules membranaires ;
d) comprend une séquence qui présente au moins 60% d'identité avec la séquence de SEQ ID NO : 8 complète ; et
e) a subi une délétion, ou une substitution par un résidu alanine, du résidu cystéine de la séquence PCP contenue dans la séquence de SEQ ID NO : 6.

Les définitions données ci-dessus dans le cadre du polypeptide chimérique de la demande ou de la vésicule de l'invention s'appliquent également par analogie à ce polypeptide.

Selon un mode de réalisation particulier, ledit dérivé muté est un dérivé muté du domaine CD de la protéine TM du BLV (domaine CD de séquence SEQ ID NO : 6). Ce dérivé muté est tel que défini dans les différents aspects de la demande ou invention concernant le polypeptide chimérique. Ainsi, ledit dérivé muté, qui est généralement dépourvu de séquence KCLTSRLLKLLRQ et/ou dépourvu de peptide PCP, peut comprendre ou consister en une séquences choisie parmi les séquences suivantes:
PxxPxxxxxxxxxxxxYxxL ;
PxxPxxxxxxxxxxxDYxxL ;
PxxPxxYxxxxxxxxxYxxL ;
PxxPxxYxxxxxxxxDYxxL ;
PxxPExYxxLxPxxPDYxxL ;
PxxPxₙYxxL ;
PxxPxₙDYxxL ;
PxxPXₙYXₙYxxL ;
PxxPxₙYxₙDYxxL ;
PxxPExₙYxxLxₙPxxPDYxxL ;
PxxPxxxxPxxPxxxYxxLxPxxPExYxxLxPxxPDYxxL ;
PxxPXₙPxxPXₙYxxLXₙPxxPEXₙYxxLxₙPxxPDYxxL ;
PxxPxxxxPxxPxxxYxxLxPxxPExYxxLxPxxPDYxxLxxxx ;
PxxPxₙPxxPxₙYxxLxₙPxxPEXₙYxxLxₙPxxPDYxxLxxxx.
PxxPExxxPxKPDxDYxxLxPxxPExYxxLxPxxPDYxxLR ;
PxxPExₙPxKPDxₙDYxxLxₙPxxPEXₙYxxLxₙPxxPDYxxLR ;
PxxPExxxPxKPDxDYxxLxPxxPExYxxLxPxxPDYxxLRxxxx et
PxxPExₙPxKPDxₙDYxxLxₙPxxPExₙYxxLxₙPxxPDYxxLRxxxx,
dans lesquelles x et xₙ représentent respectivement un résidu quelconque et un ou plusieurs résidu (s) quelconque(s).

En particulier n est supérieur ou égal à 1 et inférieur à 50. n peut avoir en particulier toute valeur entre 1 et 20.

A titre d'exemple, le motif PxxP qui, dans un mode de réalisation particulier, se trouve en position N-terminale dans les séquences indiquées ci-dessus, peut être le motif PSAP ou PTAP.

Alternativement ou de façon complémentaire, le motif YxxL qui, dans un mode de réalisation particulier, se trouve en position C-terminale dans les séquences indiquées ci-dessus, peut être par exemple le motif YINL ou YSHL.

En particulier, ce dérivé muté de CD comprend exclusivement les motifs PxxP et YxxL ou DYxxL contenus dans les séquences décrites ci-dessus.

Selon un mode de réalisation préféré, le dérivé muté du domaine CD du polypeptide de la demande ou l'invention comprend au moins un motif YxxL ou DYxxL (par exemple le motif YINL, YSHL ou DYINL) et un motif PxxP (par exemple le motif PSAP ou PTAP) dans lequel x représente un résidu quelconque.

A titre d'exemple, le dérivé muté du polypeptide de la demande ou l'invention peut avoir pour séquence SEQ ID NO : 30, SEQ ID NO : 42, SEQ ID NO : 44 ou SEQ ID NO : 95 ou présenter au moins 60 ou 70%, en particulier au moins 80%, 90% ou 95%, de similarité ou d'identité avec la séquence SEQ ID NO : 30, SEQ ID NO : 42, SEQ ID NO : 44 ou SEQ ID NO: 95 par référence respectivement à la séquence SEQ ID NO : 30, SEQ ID NO : 42, SEQ ID NO : 44 ou SEQ ID NO : 95 complète.
Plus précisément, le dérivé muté du polypeptide de la demande ou l'invention peut avoir pour séquence SEQ ID NO : 8 ou présenter au moins 60 ou 70%, en particulier au moins 80%, 90% ou 95%, de similarité ou d'identité avec la séquence SEQ ID NO : 8 par référence à la séquence SEQ ID NO : 8 complète. Par exemple, la séquence dudit dérivé muté peut comprendre ou consister en la séquence SEQ ID NO : 10 ou SEQ ID NO : 12. De préférence, la séquence dudit dérivé muté conserve notamment le motif YINL ou YSHL (ou le cas échant DYINL) et le motif PSAP de la séquence SEQ ID NO : 8.

Selon un mode de réalisation particulier, le polypeptide de la demande ou l'invention est en outre associé ou fusionné à un peptide ou un polypeptide d'intérêt.

La demande ainsi que l'invention ont également pour objet l'utilisation d'un polypeptide chimérique de la demande ou d'une vésicule de l'invention ou d'un polypeptide de la demande ou l'invention tels que définis dans la demande ou invention, pour adresser (*in vivo* ou *in vitro*) un peptide ou polypeptide d'intérêt vers les vésicules membranaires, en particulier vers les vésicules formant des exosomes, et/ou vers le(s) compartiment(s) cellulaire(s) impliqué(s) dans la formation des vésicules membranaires, et en particulier des vésicules formant les exosomes, et pour permettre ainsi la sécrétion, par des cellules eucaryotes appropriées, dudit peptide ou polypeptide d'intérêt en association avec lesdites vésicules membranaires.

La demande ainsi que l'invention ont également pour objet une vésicule membranaire, et plus précisément un exosome, qui comporte un polypeptide chimérique de la demande ou de la vésicule de l'invention, ou un polypeptide de la demande ou l'invention tels que définis ci-dessus, et/ou un ou plusieurs produit(s) de dégradation dudit polypeptide chimérique ou dudit polypeptide, ce(s) produit(s) de dégradation étant le cas échéant associé(s) à une molécule du complexe majeur d'histocompatibilité (CMH) de type I et/ou de type II.

Le polypeptide chimérique de la demande ou de la vésicule de l'invention, ou le polypeptide de la demande ou l'invention, et/ou leur(s) produit(s) de dégradation est(sont) ancré(s) dans la membrane de ladite vésicule membranaire par l'intermédiaire de leur ou d'un de leur(s) domaine(s) membranaire(s) ou par l'intermédiaire du domaine membranaire de la molécule du CMH (de classe I ou II) à laquelle il est(sont) associé(s).

Selon un mode de réalisation particulier, ledit produit de dégradation comprend ou consiste en un fragment du peptide ou polypeptide d'intérêt, en particulier en un fragment comprenant ou consistant en un ou plusieurs épitope(s) dudit peptide ou polypeptide d'intérêt.

Selon un mode de réalisation particulier, le peptide ou le polypeptide d'intérêt ou un de ses fragments est exposé, en partie ou en totalité, à la surface, à l'extérieur de la vésicule membranaire. Ainsi, cette vésicule membranaire peut être utilisée notamment pour produire ou sélectionner *in vivo* ou *in vitro* des cellules procaryotes ou eucaryotes ou des virus (par exemple des phages) ou des ribosomes interagissant directement ou indirectement avec ledit peptide ou polypeptide d'intérêt ou leur fragment. Cette vésicule membranaire peut aussi être utilisée pour produire *in vivo* ou *in vitro* des anticorps monoclonaux ou polyclonaux, vaccinants ou non vaccinants, dirigés contre ledit peptide ou polypeptide d'intérêt ou leur fragment. De tels anticorps peuvent être utilisés notamment en diagnostic ou pour étudier des interactions protéiques, en particulier pour réaliser des criblages à haut débit de molécules telles que des drogues ou des cytokines capables d'interagir avec le peptide ou le polypeptide d'intérêt ou leur fragment. En outre, cette vésicule membranaire peut être utilisée *in vivo,* en immunisation, pour éliciter ou favoriser, chez un hôte (humain ou non humain), une réponse humorale et/ou cellulaire contre une tumeur, ou contre le virus, la bactérie ou le parasite dont le peptide ou le polypeptide d'intérêt dérive.

La réponse immune élicitée ou favorisée pour la vésicule membranaire de la demande ou l'invention, en particulier par un exosome de la demande ou l'invention, peut être, selon la nature des polypeptides associés aux exosomes, une réponse tolérogène ou de défense. Une réponse tolérogène peut permettre, par exemple, à l'hôte de lutter contre l'asthme ou de supporter une greffe.

Selon un mode de réalisation préféré, ledit peptide ou polypeptide d'intérêt ou son fragment est exposé (en partie ou en totalité) dans sa conformation native à la surface de la vésicule membranaire.

Selon un mode de réalisation particulier, le peptide ou le polypeptide d'intérêt ou son fragment est inclus en partie ou en totalité dans la membrane de la vésicule membranaire de la demande ou l'invention, et/ou inclus en partie ou en totalité dans la fraction cytosolique de ladite vésicule membranaire.

Une composition pharmaceutique ou une composition immunogène, dont le principe actif comprend une ou plusieurs vésicule(s) membranaire(s), en particulier un ou plusieurs exosome(s) tel(s) que défini(s) dans la demande ou invention, ou encore un ou plusieurs polypeptide(s) chimérique(s) de la demande ou de la vésicule de l'invention, un ou plusieurs polypeptide(s) de la demande ou l'invention tels que définis ci-dessus fait également partie de la demande et l'invention. Ladite composition comprend en outre un ou plusieurs véhicule(s), diluant(s), et/ou adjuvant(s) ou une de leurs combinaisons. Dans le cas d'une administration injectable, on peut notamment choisir une formulation dans une solution aqueuse, non aqueuse ou isotonique.

Le terme « véhicule » désigne, dans la demande ou invention, tout support (c'est-à-dire tout ce qui peut transporter au moins un principe actif) qui n'altère pas l'efficacité de l'activité biologique des substances actives de la demande ou l'invention. De nombreux véhicules sont connus dans l'état de la technique. Les véhicules utilisés peuvent être, par exemple, de l'eau, une solution saline, de l'albumine sérique, une solution Ringer, le polyéthylène glycol, des solvants miscibles dans l'eau, des sucres, des lieurs, des excipients, des pigments, des huiles végétales ou minérales, des polymères solubles dans l'eau, des agents tensioactifs, des agents épaississants ou gélifiants, des agents cosmétiques, des agents de solubilisation, des agents de stabilisation, des agents conservateurs, des agents alcalinisants ou acidifiants ou une de leurs combinaisons.

Le terme « diluant » signifie, dans la demande ou invention, un agent de dilution, et inclut les diluants solubles et les diluants insolubles. On utilise généralement un diluant insoluble quand le principe actif est soluble et un diluant soluble quand le principe actif est insoluble. Un principe actif « insoluble » peut être totalement insoluble en milieu aqueux ou avoir une solubilité limitée (c'est à dire une solubilité inférieure à 10mg/ml dans 250 ml d'eau à un pH de 1,0 à 7,5) en milieu aqueux. Des exemples de diluants insolubles incluent la cellulose microcristalline, la cellulose microcristalline silicifiée, l'hydroxyméthylcellulose, le phosphate de dicalcium, le carbonate de calcium, le sulfate de calcium, le carbonate de magnésium, le phosphate de tricalcium, etc. Des exemples de diluants solubles incluent le mannitol, le glucose, le sorbitol, le maltose, les dextrates, les dextrines, le dextrose, etc.

Le terme « adjuvant » désigne, dans la demande ou invention, un produit qui, ajouté au contenu à une composition immunogène, en particulier à un vaccin, accroît l'intensité de la réaction immunitaire induite chez l'hôte (humain ou non humain) auquel ladite composition est administrée. Un adjuvant peut notamment accroître la quantité d'anticorps spécifiques que ledit mammifère est capable de produire après l'administration de ladite composition et accroît donc l'efficacité de la l'immunisation. Les adjuvants sont notamment utiles quand l'antigène utilisé seul ne provoque qu'une réaction immunitaire trop faible pour procurer une bonne protection, pour réduire la quantité d'antigène à administrer à un hôte, ou encore pour faciliter certains modes d'administration de ladite composition, par exemple dans le cas d'une administration au niveau des muqueuses. Les adjuvants susceptibles d'être utilisés dans le cadre de la demande ou l'invention sont en particulier des saponines, du phosphate d'aluminium (alum), des peptidoglycanes, des carbohydrates, des peptides, par exemple le muramyl dipeptide (N-acétylmuramyl-L-alanyl-D-isoglutamine, MDP), des émulsions huile/eau, des polysaccharides, des cytokines, des hormones, l'hémocyanine de patelle, des adjuvants de la famille des dinucléotides CpG non méthylés, des adjuvants de la famille des poly IC, des adjuvants de la famille du monophosphoryl lipide A et des acides nucléiques, en particulier des ADN bactériens ou des ADN codant pour une protéine ayant un effet adjuvant, par exemple un facteur de croissance ou une cytokine, plus particulièrement le GM-CSF ou l'IL4.

Selon un mode de réalisation préféré, ledit ou lesdits véhicule(s), diluant(s), et/ou adjuvant(s) ou leur combinaison sont des substances ou une combinaison de substances pharmaceutiquement acceptables, c'est à dire appropriée(s) pour une administration à un hôte (par exemple un humain, un mammifère non humain ou un oiseau) à des fins thérapeutiques ou prophylactiques. Une telle substance ou combinaison de substance est donc préférentiellement non toxique pour l'hôte auquel elle est administrée.

La demande ainsi que l'invention ont également pour objet un polynucléotide caractérisé en ce qu'il code pour un polypeptide chimérique de la demande ou de la vésicule de l'invention, ou pour un polypeptide selon la demande ou invention, selon le code génétique universel, et en tenant compte de la dégénérescence de ce code. Le terme « polynucléotide » couvre toute molécule d'ADN ou d'ARN (mono ou bicaténaire). Ce polynucléotide peut être nu ou, alternativement, il peut être inséré dans un vecteur de clonage ou d'expression, de préférence un vecteur approprié pour l'expression dans des cellules eucaryotes. Ce vecteur est, de préférence un plasmide. Ledit polynucléotide peut être notamment assemblé par PCR. Il comporte de préférence 2000 à 50000 nucléotides.

Le terme « code » ne signifie pas nécessairement que ledit polynucléotide ne comprend que la partie codante. Ledit polynucléotide peut en effet comprendre en outre des séquences de régulation de l'expression et notamment comprendre un promoteur, par exemple un promoteur eucaryote

A titre d'exemple, ledit polynucléotide comprend en tant que séquence codant pour le domaine (iii) du polypeptide chimérique de la demande ou de la vésicule de l'invention une séquence comprenant ou consistant en une séquence choisie parmi les séquences SEQ ID NO : 5, SEQ ID NO : 7, SEQ ID NO : 9, SEQ ID NO : 11, SEQ ID NO : 47 et SEQ ID NO : 94.

Selon un mode de réalisation préféré, ledit polynucléotide comprend une séquence codant pour un signal d'importation dans le réticulum endoplasmique. Par exemple, cette séquence peut être la séquence SEQ ID NO : 1 ou une séquence codant pour un peptide de séquence SEQ ID NO : 49 à SEQ ID NO : 78. Comme indiqué précédemment, le peptide signal codé par cette séquence, lorsqu'il se trouve en position N- ou C-terminale dans le polypeptide codé par ledit polypeptide, peut être clivé lors d'une étape de maturation du polypeptide chimérique, qui a généralement lieu dans le réticulum endoplasmique ou dans le Golgi.

Ledit polynucléotide peut être placé sous le contrôle d'éléments de régulation, de clonage ou d'expression.

Ainsi, selon un mode de réalisation particulier, ledit polynucléotide est inséré dans un vecteur de clonage ou d'expression, de préférence un vecteur d'expression et plus préférablement un vecteur approprié pour l'expression dans des cellules eucaryotes. Ledit vecteur est de préférence un plasmide.

Ledit polynucléotide est en général placé sous le contrôle d'un promoteur eucaryote, de préférence un promoteur eucaryote fort tel qu'un promoteur viral, par exemple le promoteur d'un virus choisi parmi : le virus SV40, le virus du sarcome de Rous, le virus des leucémies murines (MuLV), le virus des leucémies à cellules T de l'homme adulte (HTLV-I), le virus de la leucémie bovine (BLV), le cytomégalovirus, un promoteur hybride dérivé de ces promoteurs viraux ou un promoteur viral contenant des séquences modifiées.

Ledit polynucléotide peut en outre comporter une séquence kozak, en particulier la séquence nucléotidique ACCATGG, dans laquelle la séquence ATG correspond au codon d'initiation de la séquence codante.

Par ailleurs, ledit polynucléotide peut en outre comporter un intron.

Selon un mode de réalisation particulier, ledit polynucléotide comprend au moins un linker nucléotidique codant pour un linker polypeptidique tel que défini ci-dessus. Ce linker nucléotidique peut en particulier comprendre ou consister en un site de restriction. Par « site de restriction », on entend une séquence nucléotidique particulière reconnue par une enzyme de restriction de type Il comme un site de coupure dans le polynucléotide. A titre d'exemple, ce linker nucléotidique peut consister en la séquence nucléotidique AGGTCTAGA, qui comprend le site de restriction de l'enzyme de restriction Xbal (séquence TCTAGA).

Selon un mode de réalisation particulier, la séquence dudit polynucléotide est optimisée pour une utilisation chez un hôte (par exemple un hôte eucaryote), en particulier un être humain, un mammifère non humain et/ou un oiseau.

La demande ainsi que l'invention ont également pour objet un vecteur de clonage ou d'expression, qui est, de préférence un plasmide, caractérisé en ce qu'il comprend un insert polynucléotidique constitué par un polynucléotide de la demande ou l'invention, sous le contrôle d'éléments de régulation, de clonage ou d'expression.

La demande ainsi que l'invention ont également pour objet une culture cellulaire sélectionnée parmi le groupe comprenant les cultures cellulaires de bactéries, les cultures cellulaires primaires de cellules eucaryotes animales et les lignées cellulaires, ladite culture cellulaire contenant un polynucléotide de la demande ou l'invention, un vecteur de clonage ou d'expression de la demande ou l'invention, ou un polypeptide chimérique de la demande ou de la vésicule de l'invention.

La demande ainsi que l'invention ont également pour objet une composition immunogène comprenant un acide nucléique, en particulier un ADN, caractérisé en ce qu'il comprend ou consiste en un polynucléotide de la demande ou l'invention, et un support, un diluant ou un véhicule pharmaceutiquement acceptable.

Selon un mode de réalisation particulier, ladite composition immunogène comprend en outre un adjuvant tel que défini dans la demande ou l'invention.

Selon un mode de réalisation particulier, ledit acide nucléique est un ADN. Les compositions immunogènes à base d'ADN ciblent *in vivo* des cellules de l'hôte à immuniser, en particulier les cellules dendritiques (notamment les cellules de Langerhans), qui sont d'excellents producteurs d'exosomes vaccinants. Ainsi, cette composition immunogène peut permettre d'induire la production, par les propres cellules d'un hôte immunisé avec ladite composition immunogène, d'exosomes porteurs du peptide ou du polypeptide d'intérêt ou d'un fragment de celui-ci.

Selon un mode de réalisation particulier, ladite composition immunogène est un vaccin, en particulier un vaccin à ADN.

La demande ainsi que l'invention ont également pour objet une cellule recombinante productrice d'exosomes, en particulier une cellule du système immunitaire et plus particulièrement une cellule du système immunitaire choisie parmi les mastocytes, les lymphocytes, en particulier les lymphocytes B et T, et les cellules dendritiques, en particulier les cellules de Langerhans, caractérisée en ce qu'elle est recombinée avec un ou plusieurs polynucléotide(s) de la demande ou l'invention, un vecteur de clonage ou d'expression de la demande ou l'invention ou qu'elle a absorbé une vésicule membranaire de la demande ou l'invention.

En outre, la demande ainsi que l'invention concernent également un polypeptide chimérique de la demande ou de la vésicule de l'invention, un polypeptide de la demande ou l'invention, une ou plusieurs vésicule(s) membranaire(s) de la demande ou l'invention (en particulier un ou plusieurs exosome(s) de la demande ou l'invention), un polynucléotide de la demande ou l'invention ou une composition immunogène de la demande ou l'invention pour une utilisation comme médicament, en particulier pour une utilisation pour la prophylaxie et/ou le traitement d'une infection bactérienne, virale, parasitaire, ou d'une tumeur. Ils sont notamment destinés à une utilisation en immunisation, en particulier pour éliciter ou favoriser (c'est-à-dire notamment amplifier) *in vivo,* chez un hôte (humain ou non humain) une réponse humorale et/ou cellulaire contre la tumeur, le virus, la bactérie ou le parasite dont le peptide ou polypeptide d'intérêt dérive. Ils peuvent être notamment utilisés *in vivo* pour éliciter ou amplifier une réponse T CD4 et/ou T CD8 spécifique dirigée contre le peptide ou le polypeptide d'intérêt et/ou pour produire des anticorps polyclonaux et/ou monoclonaux dirigés contre le peptide ou le polypeptide d'intérêt, en particulier des anticorps dirigés contre un peptide ou un polypeptide comprenant ou consistant en un ou plusieurs épitopes conformationnels.

La demande ainsi que l'invention ont également pour objet l'utilisation d'un polypeptide de la demande ou l'invention (en particulier un polypeptide chimérique de la demande ou de la vésicule de l'invention), d'une ou plusieurs vésicule(s) membranaire(s) de la demande ou l'invention (en particulier un ou plusieurs exosome(s) de la demande ou l'invention), d'un polynucléotide de la demande ou l'invention ou d'une composition immunogène de la demande ou l'invention pour la fabrication d'un médicament destiné à la prophylaxie et/ou au traitement d'une tumeur ou d'une infection par un organisme pathogène ou par un agent pathogène, notamment une infection bactérienne, virale ou parasitaire.

Suite à l'administration d'une composition immunogène, dont le principe actif est un polynucléotide de la demande ou l'invention ou une ou plusieurs vésicule(s) membranaire(s) de la demande ou l'invention, à un hôte (humain ou non humain), les cellules productrices d'exosomes de cet hôte, en particulier les cellules dendritiques, vont produire des exosomes, comportant un polypeptide chimérique de la demande ou de la vésicule de l'invention, ou un polypeptide de la demande ou l'invention, et/ou un produit de dégradation de ces derniers polypeptides, ce produit de dégradation pouvant s'associer naturellement à une molécule du complexe majeur d'histocompatibilité. Ces vésicules membranaires, en particulier celles sur la surface desquelles le peptide ou le polypeptide d'intérêt ou un fragment est exposé (en partie ou en totalité) vont permettre d'éliciter ou favoriser une réponse immune dirigée contre le peptide ou le polypeptide d'intérêt.

Un « hôte » au sens de la demande ou invention désigne un humain ou un animal non humain.

Le terme « animal non humain » tel qu'utilisé dans la demande ou invention inclut tout mammifère non humain, notamment un rongeur (en particulier une souris, un rat, un hamster ou un lapin), un singe, un camélidé, un chat, un chien, un cheval, un mulet, un bovin, un ovin, un porcin, et inclut également un oiseau, en particulier un poulet.

L'expression « infection » telle qu'utilisée dans la demande ou invention signifie que ledit hôte (humain ou non humain) a été exposé à un organisme pathogène ou un agent pathogène en particulier à un virus enveloppé tel que défini dans la demande ou l'invention. En particulier une telle infection est capable d'évoluer vers des signes cliniques de pathologies induites ou accompagnant ladite infection. Le terme « infection » englobe donc également tout signe clinique, symptôme ou maladie apparaissant chez un hôte (humain ou non humain) suite à son exposition à un organisme pathogène ou un agent pathogène. Par exemple, une « infection virale » ou une « infection bactérienne » au sens de la demande ou invention inclut à la fois les phases les plus précoces de la contamination virale, bactérienne ou parasitaire, les phases intermédiaires, et les phases les plus tardives de la contamination, ainsi que les pathologies diverses qui sont la conséquence de la contamination d'un hôte par un virus, par des bactéries ou par un parasite, il inclut aussi la présence de tout ou partie de génome d'un organisme pathogène.

Le terme « prophylaxie » désigne tout degré de retardement dans le moment d'apparition de signes cliniques ou de symptômes de l'infection ou de l'apparition d'une tumeur, ainsi que tout degré d'inhibition de la sévérité des signes cliniques ou symptômes de l'infection ou de la tumeur, y compris, mais sans limitation à, la prévention totale de l'infection ou d'un cancer. Ceci nécessite que les polypeptides de la demande ou l'invention, le polynucléotide de la demande ou l'invention, les vésicules membranaires ou les compositions immunogènes de la demande ou l'invention soit administrés à l'hôte susceptible d'être exposé à un organisme pathogène ou un agent pathogène et/ou susceptible de développer une tumeur avant l'apparition de tout signe clinique ou symptôme de la maladie. L'administration prophylactique peut avoir lieu avant que ledit hôte ne soit exposé à l'organisme ou à l'agent pathogène responsable de l'infection, ou au moment de l'exposition. Une telle administration prophylactique sert à empêcher, et/ou réduire la sévérité de n'importe quelle infection subséquente. La prophylaxie au sens de la demande ou invention couvre également la prévention totale d'une infection ou d'un cancer.

Par « traitement », on entend l'effet thérapeutique que produisent sur un hôte le polypeptide chimérique, le polypeptide, le polynucléotide, les vésicules membranaires ou une des compositions immunogènes de la demande ou l'invention lorsqu'ils sont administrés audit hôte au moment de l'exposition à un organisme ou un agent pathogène, après l'exposition ou après l'apparition de signes cliniques ou de symptômes de l'infection ou après l'apparition d'une tumeur. Lorsque les substances actives de la demande ou l'invention sont administrées à un hôte après la contamination par un virus, ils peuvent être administrés pendant la phase de primo-infection, pendant la phase asymptomatique ou encore après l'apparition de signes cliniques ou de symptômes de la maladie.

Le terme « traitement » inclut tout effet curatif obtenu grâce à une substance active de la demande ou l'invention, ainsi que l'amélioration des signes cliniques ou des symptômes observés chez l'hôte (humain ou non humain), de même que l'amélioration de la condition de l'hôte. Ainsi, le terme « traitement » couvre notamment le ralentissement, la diminution, l'interruption, ainsi que l'arrêt d'une infection virale, bactérienne ou parasitaire ou de la croissance de la tumeur et/ou des conséquences néfastes de l'infection ou de l'apparition de la tumeur; un traitement n'exige pas nécessairement l'élimination complète de tous les signes cliniques de l'infection ou de la tumeur et de tous les symptômes de la maladie, ni même l'élimination complète du virus, des bactéries, des parasites ou des cellules tumorales.

Les substances actives de la demande ou l'invention peuvent donc être administrées à un hôte qui présente des risques d'être exposé à un organisme ou un agent pathogène et de développer une infection ou une tumeur (prophylaxie) ou après l'exposition de l'hôte à un organisme ou un agent pathogène, en particulier après la manifestation des premiers signes cliniques ou symptômes de la maladie, par exemple après que des protéines ou anticorps spécifiques d'un virus, de bactéries, de parasites ou d'une tumeur ont été détectés dans le sang de l'hôte (traitement).

La demande ainsi que l'invention ont également pour objet une méthode pour prévenir et/ou traiter une infection virale, bactérienne ou parasitaire ou une tumeur, ladite méthode comprenant au moins une étape d'administration *in vivo,* à un hôte le nécessitant, du polypeptide chimérique de la demande ou de la vésicule de l'invention, ou du polypeptide de la demande ou l'invention, d'une ou plusieurs vésicule(s) membranaire(s) de la demande ou l'invention (en particulier un ou plusieurs exosome(s) de la demande ou l'invention), d'un polynucléotide de la demande ou l'invention ou d'une composition immunogène de la demande ou l'invention. Ladite méthode de traitement est, en particulier, appropriée pour et destinée à éliciter ou favoriser, *in vivo,* chez ledit hôte, une réponse humorale et/ou cellulaire contre la tumeur, le virus, la bactérie ou le parasite dont le peptide ou polypeptide d'intérêt dérive.

Les termes « administration » et « administrer » tels qu'utilisés dans la demande ou invention incluent toute administration, quelle que soit la voie d'administration choisie.

Les voies d'administration et les posologies varient en fonction d'une variété de paramètres, par exemple en fonction de l'état de l'hôte, du type d'infection et de la sévérité de l'infection à traiter ou de l'importance de la tumeur.

Le polypeptide chimérique de la demande ou de la vésicule de l'invention, ou le polypeptide de la demande ou l'invention, de même qu'une ou plusieurs vésicule(s) membranaire(s) de la demande ou l'invention (en particulier un ou plusieurs exosome(s) de la demande ou l'invention), le polynucléotide de la demande ou l'invention ou une composition immunogène de la demande ou l'invention sont susceptibles d'être administrés à un hôte humain ou non humain sous forme sèche, solide (en particulier cachet, poudre, gélule, pilule, granule, suppositoire, capsule polymère ou comprimé, et plus précisément comprimé à libération accélérée, comprimé gastrorésistant ou comprimé à libération prolongée), sous forme gélatineuse ou sous la forme d'une solution ou d'une suspension liquide (en particulier sirop, solution injectable, infusible ou buvable, nébulisats, microvésicules, liposomes) ou sous la forme de patch. Ces composés peuvent également se présenter sous la forme de doses sous forme sèche (poudre, lyophilisat, etc.) à reconstituer au moment de l'utilisation en utilisant un diluant approprié. Par ailleurs, ils peuvent être conditionnés pour une administration sous la forme d'une dose unique (monodose) ou multiple (multidose).

Afin d'augmenter les effets bénéfiques des compositions immunogènes de la demande ou l'invention, il est en effet envisageable de procéder à une administration sous la forme de plusieurs administrations successives, répétées à une ou plusieurs occasions, après un intervalle de temps particulier. Ils peuvent en outre être administrés avec un second agent thérapeutique, en particulier un agent antiviral, antibactérien, antiparasitaire ou antitumoral.

Dans le cadre d'une utilisation en vaccination, il peut également être préférable d'immuniser un hôte, dans un premier temps avec une composition immunogène à base d'un polynucléotide de la demande ou l'invention, en particulier avec un vaccin à ADN de la demande ou l'invention, puis, dans un deuxième temps, avec les vésicules membranaires de la demande ou l'invention, une composition immunogène à base desdites vésicules ou une composition immunogène dont le principe actif est un polypeptide de la demande ou l'invention, un polypeptide chimérique de la demande ou l'invention ou un ou plusieurs fragment(s) de ceux-ci (ledit polypeptide, polypeptide chimérique ou leur(s) fragment(s) pouvant être obtenus notamment par purification ou par synthèse chimique).

Alternativement, il peut également être préférable d'immuniser un hôte, dans un premier temps avec les vésicules membranaires de la demande ou l'invention ou une composition immunogène à base desdites vésicules, puis, dans un deuxième temps, avec une composition immunogène à base d'un polynucléotide de la demande ou l'invention, en particulier avec un vaccin à ADN de la demande ou l'invention.

Les substances actives de la demande ou l'invention peuvent être formulées pour une administration par voie entérale, parentérale (intraveineuse, intramusculaire ou sous-cutanée), transcutanée (ou transdermique ou percutanée), cutanée, orale, mucosale, en particulier transmuqueuse-buccale, nasale, ophtalmique, otologique (dans l'oreille), vaginale, rectale, ou encore les voies intragastrique, intracardiaque, intrapéritonéale, intrapulmonaire ou intratrachéale.

Les compositions immunogènes à base d'un polynucléotide de la demande ou l'invention et en particulier les vaccins à ADN de la demande ou l'invention sont de préférence administré(e)s à un hôte par voie intramusculaire ou sous-cutanée, en utilisant soit une aiguille et une seringue, soit un injecteur dépourvu d'aiguille, en particulier un pistolet à air comprimé capable de propulser, à l'intérieur de cellules d'un hôte, des microbilles d'or, de tungstène ou de platine chargées d'ADN (pistolet « biolistique » ou pistolet à gène), par exemple le pistolet « Helios® Gene Gun System» de la société BioRad.

La quantité de principe actif administrée à un hôte humain ou non humain est une quantité thérapeutiquement efficace, c'est-à-dire une quantité active, suffisante, à des posologies et pendant des périodes de temps nécessaires, pour obtenir un effet significatif et en particulier apporter un bénéfice significatif à l'hôte dans le cadre d'une administration pour la prophylaxie ou le traitement tel que défini dans la demande ou invention. Une quantité thérapeutiquement efficace est également une quantité pour laquelle les effets bénéfiques l'emportent sur un quelconque effet toxique ou néfaste du ou des principe(s) actif(s). Une telle quantité peut correspondre à une quantité suffisante pour inhiber la réplication virale, la prolifération bactérienne ou la croissance d'une tumeur de façon significative ou pour faire disparaître, réduire, ou améliorer toute infection existante provoquée l'agent ou l'organisme pathogène. La quantité thérapeutiquement efficace varie en fonction de facteurs tels que l'état d'infection, l'âge, le sexe ou le poids de l'hôte. Les régimes posologiques peuvent être ajustés de façon à obtenir un effet thérapeutique optimum.

La demande ainsi que l'invention ont également pour objet un procédé d'obtention *in vivo* de vésicules membranaires, en particulier d'exosomes, caractérisé en ce qu'il comprend une étape d'administration d'un polypeptide de la demande ou l'invention (en particulier un polypeptide chimérique dé la demande ou l'invention), d'une ou plusieurs vésicule(s) membranaire(s) de la demande ou l'invention (en particulier un ou plusieurs exosome(s) de la demande ou l'invention), d'un polynucléotide de la demande ou l'invention ou d'une composition immunogène de la demande ou l'invention, à un hôte (humain ou non humain). Ce procédé peut être notamment mis en oeuvre dans le cadre d'une méthode pour la prophylaxie et/ou le traitement d'une tumeur ou d'une infection par un organisme pathogène ou par un agent pathogène, notamment une infection bactérienne, virale ou parasitaire.

La demande ainsi que l'invention concernent en outre un procédé de production *in vitro* de vésicules membranaires et en particulier d'exosomes comportant un peptide ou un polypeptide d'intérêt et/ou un produit de dégradation de ce peptide ou un polypeptide d'intérêt, ce produit de dégradation pouvant le cas échéant s'associer à une molécule du complexe majeur d'histocompatibilité. Ledit procédé comprend les étapes suivantes :
a) l'introduction, dans une cellule productrice d'exosomes, d'un ou plusieurs polynucléotide(s) de la demande ou l'invention, qui code(nt) pour un polypeptide comprenant ledit peptide ou polypeptide d'intérêt, ou la mise en contact d'une cellule productrice d'exosomes avec une ou plusieurs vésicule(s) membranaire(s) de la demande ou l'invention, qui comporte(nt) un polypeptide comprenant ledit peptide ou polypeptide d'intérêt et/ou un produit de dégradation de ce polypeptide, ou avec une composition à base de telles vésicules;
b) la culture de ladite cellule productrice d'exosomes;
c) la récupération des vésicules membranaires et en particulier des exosomes produit(e)s par ladite cellule productrice d'exosomes.

Selon un mode de réalisation particulier, la cellule productrice d'exosomes est une cellule de la lignée HEK293, ou une lignée dérivée, ou une cellule du système immunitaire. En particulier, la cellule du système immunitaire peut être choisie parmi les mastocytes, les lymphocytes, en particulier les lymphocytes T et B, et les cellules dendritiques. La cellule du système immunitaire est de préférence une cellule dendritique, par exemple une cellule de Langerhans.

Selon un mode de réalisation particulier, l'étape a) consiste en la mise en contact d'une ou plusieurs vésicule(s) membranaire(s) de la demande ou l'invention, en particulier d'un ou plusieurs exosome(s) de la demande ou l'invention, avec une cellule dendritique.

Selon un mode de réalisation particulier, ledit procédé comprend en outre une étape intermédiaire entre les étapes a) et b), au cours de laquelle la cellule est sélectionnée et/ou stimulée pour induire et/ou augmenter la sécrétion des exosomes ou pour obtenir des exosomes présentant des qualités définies, en particulier pour induire une spécificité dans la composition des exosomes en certaines protéines cellulaires, par exemple la protéine ICAM.

Selon un mode de réalisation particulier, l'introduction, d'un ou plusieurs polynucléotide(s) de la demande ou l'invention dans une cellule productrice d'exosomes à l'étape a) est réalisée par transfection ou par transduction.

Selon un mode de réalisation particulier, l'étape c) est réalisée en purifiant les vésicules membranaires et en particulier les exosomes à partir du surnageant de culture de la cellule productrice d'exosomes par centrifugation différentielle, par ultrafiltration, par adsorption sur un support, ou par tout autre procédé.

Les vésicules membranaires et en particulier les exosomes obtenus par ce procédé font également partie de la demande ainsi que de l'invention.

La demande ainsi que l'invention ont également pour objet l'utilisation d'une ou plusieurs vésicule(s) membranaire(s) de la demande ou l'invention, ou d'une composition immunogène à base de vésicule(s) membranaire(s) de la demande ou l'invention pour produire des d'anticorps dirigés contre le peptide ou polypeptide d'intérêt, ces anticorps étant destinés à une utilisation en diagnostic ou en recherche.

La demande ainsi que l'invention ont également pour objet un procédé de préparation d'un sérum polyclonal dirigé contre un ou plusieurs peptide(s) ou polypeptide(s) antigénique(s) d'intérêt exprimé(s) à la surface de vésicules membranaires, en particulier d'exosomes, ledit procédé comprenant les étapes suivantes :
a) l'administration, le cas échéant répétée, à un animal non humain, de vésicules membranaires de la demande ou l'invention, d'une composition immunogène de la demande ou l'invention ou d'un polynucléotide de la demande ou l'invention, associés ou non à un adjuvant; et
b) la récupération des anticorps formés, capables de reconnaître le ou les peptide(s) ou polypeptide(s) antigénique(s) d'intérêt.

Selon un mode de réalisation particulier, l'étape a) est suivie d'une étape de sacrifice de l'animal non humain.

La demande ainsi que l'invention ont également pour objet deux procédés de préparation d'anticorps monoclonaux dirigés contre un ou plusieurs peptide(s) ou polypeptide(s) antigénique(s) exprimé(s) à la surface de vésicules membranaires, en particulier d'exosomes. Le premier procédé comprend les étapes suivantes:
a) la fusion, avec des cellules de myélome, de cellules spléniques préalablement obtenues chez un hôte (humain ou non humain), par exemple une souris Balb/c, auquel on a administré des vésicules membranaires de la demande ou l'invention, une composition immunogène de la demande ou l'invention ou un polynucléotide de la demande ou l'invention, le cas échéant en association avec un adjuvant et le cas échéant par administration répétée;
b) la culture et la sélection des hybridomes dans des conditions permettant la production d'anticorps;
c) la récupération des anticorps monoclonaux dirigés contre les peptide(s) ou polypeptide(s) antigénique(s) d'intérêt.

Le deuxième procédé de préparation d'anticorps monoclonaux comprend les étapes suivantes:
a) l'immortalisation de cellules productrices d'anticorps par exemple des lymphocytes ou des lymphoblastes, à partir de cellules hématopoïétiques, plus particulièrement de cellules du sang, préalablement obtenues chez un hôte (humain ou non humain), par exemple une souris Balb/c, auquel on a administré des vésicules membranaires de la demande ou l'invention une composition immunogène de la demande ou l'invention ou un polynucléotide de la demande ou l'invention, le cas échéant en association avec un adjuvant et le cas échéant par administration répétée;
b) la culture et la sélection des cellules immortalisées dans des conditions permettant la production d'anticorps;
c) la récupération des anticorps monoclonaux dirigés contre les peptide(s) ou polypeptide(s) antigénique(s) d'intérêt.

L'immortalisation des cellules productrices d'anticorps à l'étape a) peut être réalisée notamment par infection de ces cellules avec un virus immortalisant. Ce virus immortalisant peut être un virus herpétique, par exemple le virus d'Epstein-Barr. Cette immortalisation peut également être effectuée par modification du génome des cellules productrices d'anticorps avec un composant immortalisant. Ce composant immortalisant peut être un composant viral, par exemple d'un virus herpétique, ou un gène cellulaire, par exemple le gène de la télomérase.

L'animal non humain auquel on a administré des vésicules membranaires, une composition immunogène ou un polynucléotide de la demande ou l'invention dans le cadre du procédé de préparation d'un sérum polyclonal ou des procédés de préparation d'anticorps monoclonaux peut être notamment un rongeur (en particulier une souris, par exemple une souris Balb/c, un rat, un hamster ou un lapin), un oiseau, en particulier un poulet, ou un mulet.

Selon un mode de réalisation particulier des deux procédés de préparation d'anticorps monoclonaux de la demande ou l'invention, les cellules spléniques ou les cellules productrices d'anticorps de l'étape a) ont été préalablement obtenues chez un hôte non humain après une étape de sacrifice dudit animal non humain.

Si nécessaire, les anticorps monoclonaux ou polyclonaux produits par les procédés de préparation d'anticorps de la demande ou l'invention peuvent être "humanisés", c'est-à-dire modifiés par génie génétique de façon remplacer au maximum les fragments constants Fc de l'espèce d'origine par des fragments humains.

La demande ainsi que l'invention ont également pour objet l'utilisation d'un polypeptide chimérique de la demande ou l'invention, d'un polypeptide de la demande ou l'invention, d'une ou plusieurs vésicule(s) membranaire(s) de la demande ou l'invention ou d'une composition immunogène de la demande ou l'invention, pour la détection (en particulier *in vitro*) de partenaires spécifiques capables d'interagir avec ledit peptide ou un polypeptide d'intérêt ou avec un fragment dudit peptide ou un polypeptide d'intérêt.

La demande ainsi que l'invention ont également pour objet un procédé de criblage *in vitro* de molécules ou un procédé de sélection de cellules productrices de molécules interagissant avec un peptide ou un polypeptide d'intérêt ou avec un fragment dudit peptide ou un polypeptide d'intérêt, ledit procédé comprenant :
a) la mise en contact de vésicules membranaires de la demande ou l'invention avec une ou plusieurs molécules susceptibles d'interagir avec ledit peptide ou polypeptide d'intérêt ;
b) la détection d'une éventuelle interaction entre ledit peptide ou polypeptide d'intérêt ou un fragment dudit peptide ou polypeptide d'intérêt et ladite ou lesdites molécules.

Les interactions entre ledit peptide ou polypeptide d'intérêt et un éventuel partenaire peuvent être mises en évidence *in vitro* par toute technique permettant de démontrer des interactions protéiques et en particulier entre une protéine et son ligand, par exemple, par des expériences de coimmunoprécipitation, par exemple par ELISA, par exemple par cytofluorimétrie en flux, par exemple par électrophorèse PAGE-SDS ou par transfert de type «Western» (Western Blot), ainsi que par toute technique de criblage à haut débit permettant de démontrer des interactions protéiques et en particulier entre une protéine et son ligand, par exemple des techniques mesurant des modifications en inositol phosphates, ou en cAMP, ou en calcium, ou des transferts d'énergie (par exemple technique FRET ou BRET) entre molécules ou entre deux domaines de molécules.

Selon un mode de réalisation particulier, les vésicules membranaires utilisées à l'étape a) du procédé de criblage de la demande ou l'invention sont telles que au moins un peptide ou polypeptide d'intérêt ou au moins un de leurs fragments est exposé, en partie ou en totalité, à l'extérieur de ladite vésicule membranaire. Ledit peptide ou polypeptide d'intérêt peut être en particulier un récepteur à multiples domaines transmembranaires, par exemple le récepteur CXCR4 ou un récepteur comportant un seul domaine transmembranaire, par exemple le récepteur CD4 ou EGF-R1.

Enfin, la demande ainsi que l'invention décrivent un kit comprenant un polynucléotide de la demande ou l'invention et une notice d'utilisation.

D'autres caractéristiques et avantages de la demande ou invention apparaissent dans les exemples et les figures qui suivent.

### DESCRIPTION DES DESSINS

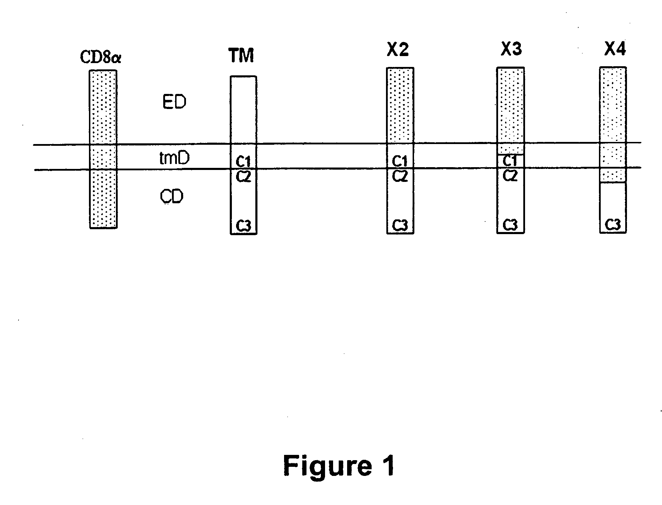

**Figure 1** **:** Représentation des différents types de constructions CD8-CD™ étudiées. **Construction X2** (séquence SEQ ID NO : 79 et SEQ ID NO : 80): l'ectodomaine (ED) de CD8 est fusionné avec les domaines transmembranaires (tmD) et cytoplasmique (CD) de la protéine TM du BLV. Cette construction conserve les deux sites de palmitoylation Cys 1 et Cys 2 ainsi que le résidu cystéine régulateur non palmitoylable (Cys 3) de la protéine TM du BLV. **Construction X3** (séquence SEQ ID NO: 81 et SEQ ID NO: 82): ED et une portion de tmD de CD8 sont fusionnés avec une portion tmD du BLV et la totalité de CD™ BLV. TmD du BLV est alors amputée de ses 15 premiers résidus. Cette construction conserve les deux sites de palmitoylation (les résidus cystéine en positions 153 et 158) ainsi que le résidu cystéine régulateur non palmitoylable (le résidu cystéine en position C-terminale ; Cys 3) de la protéine TM du BLV. **Construction X4** (séquence SEQ ID NO : 83 à SEQ ID NO : 86): La majeure partie de CD™ BLV est conservée. Dans cette construction, le domaine transmembranaire du CD8 alpha de souris est lié par un linker de séquence "RSR" au domaine CD de la protéine TM du BLV. Cette construction ne comporte que le résidu cystéine régulateur non palmitoylable (Cys 3) de la protéine TM du BLV.

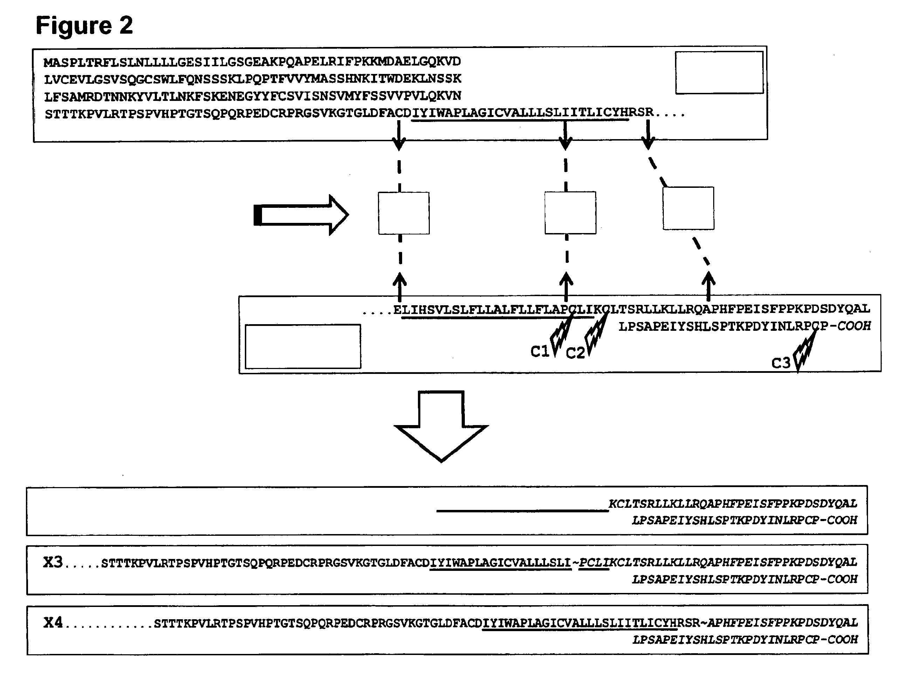

**Figure 2** **:** Récapitulatif des séquences des protéines chimères obtenues à partir de la protéine CD8 alpha de souris et de la protéine TM du BLV. Les résidus soulignés correspondent aux résidus des hélices transmembranaires des protéines CD8 alpha et TM. Les trois résidus cystéine de la protéine TM du BLV qui ont fait l'objet d'une mutation (substitution par un résidu alanine) sont indiqués par C1 C2 et C3. Les protéines chimères crées correspondent aux constructions X2 (séquence SEQ ID NO : 79 et SEQ ID NO : 80), X3 (séquence SEQ ID NO : 81 et SEQ ID NO : 82) et X4 (séquence SEQ ID NO : 83 à SEQ ID NO : 86).
**Figure 3** **:** Préparations des ADN par la méthode STET. Les chiffres 1 à 7 correspondent aux numéros des échantillons des produits STET. « M » représente le marqueur de taille. Les bandes correspondant à l'ADN plasmidique super enroulé sont encadrées.
**Figure 4** **:** Contrôle de la présence des ADN dans les produits de purification sur colonne. Les bandes correspondant à l'ADN plasmidique super enroulé sont encadrées. **A :** ADN obtenu par la méthode STET et déposé sur colonne. **E** et **E'** : Fractions retenues sur la colonne puis éluées. **FT :** Fraction non retenue. **M :** Marqueur de taille.
**Figure 5** **:** Dosages spectrométriques des aliquotes et ajustement des concentrations des ADN après digestion enzymatique. La double flèche indique que les concentrations ont été réajustées.
**Figure 6** **:** Contrôle de l'identité des ADN après digestion enzymatique.-Discriminations des mutants de CD8-CD™. A: discrimination des mutants pX2 AAC et pX2 CCA. B : discrimination des mutants X2 entre eux.
**Figure 7** **:** Analyse par Western Blot de l'expression des différentes chimères CD8-CD™. La chimère CD8-CD™ est indiquée par une flèche.
**Figure 8** : Analyse par Western Blot de l'expression des différentes chimères CD8-CD™ après immunoprécipitation. La chimère CD8-CD™ est indiquée par une flèche.
**Figure 9** **:** Analyse par Western Blot de l'expression de CD8-CD™ dans les exosomes isolés par ultracentrifugation. Le signal à 55 kDa correspond à la présence de CD8-CD™.
**Figure 10** **:** Analyse par Western Blot du contenu en CD8-CD™ des vésicules isolées après sédimentation sur gradient de densité de sucrose, pour les mutants pX4 --C et pX4 --A.
**Figure 11** **:** Analyse par Western Blot de l'expression de CD8-CD™ après lyse cellulaire, selon la présence ou non d'inhibiteurs de transport vésiculaire. La chimère CD8-CD™ est indiquée par une flèche.
**Figure 12** **:** Analyse par Western Blot de l'expression de CD8-CD™ au sein des exosomes, selon la présence ou non d'inhibiteurs de transport vésiculaire. La chimère CD8-CD™ est indiquée par une flèche.
**Figure 13** **:** Analyse par imagerie d'immunofluorescence confocale des phénotypes généraux - **Phénotype A -** Cellules HEK293 transfectées avec pX2 CAC. Ce phénotype est retrouvé chez les mutants pX2 conservant la Cys 3 : pX2 CCC, pX2 ACC, pX2 CAC et pX2 AAC. (Voir partie résultats, « localisation par immunofluorescence)
**Figure 14** **:** Analyse par imagerie d'immunofluorescence confocale des phénotypes généraux - **Phénotype B -** Cellules transfectées avec pX2 CAA. Ce phénotype est retrouvé chez les mutants pX2 ne conservant pas la Cys 3 : pX2 CCA, pX2 ACA, pX2 CAA et pX2 AAA. (Voir partie résultats, « localisation par immunofluorescence)
**Figure 15** **:** Analyse par imagerie d'immunofluorescence confocale des phénotypes généraux - **Phénotype C -** Cellules transfectées avec pX3 CCC. Ce phénotype est retrouvé chez les deux mutants pX3 étudiés : pX3 CCC et pX3 ACC. (Voir partie résultats, « localisation par immunofluorescence)
**Figure 16** **:** Analyse par imagerie d'immunofluorescence confocale des phénotypes généraux - **Phénotype D -** Cellules transfectées avec pX4 --C. Ce phénotype est retrouvé chez les deux mutants pX4 étudiés : pX4 --C et pX4 --A. (Voir partie résultats, « localisation par immunofluorescence)
**Figure 17****:** Analyse par imagerie d'immunofluorescence confocale des phénotypes généraux - **Phénotype E -** Cellules transfectées avec pX4 stp. Ce phénotype est retrouvé uniquement chez pX4 stp. (Voir partie résultats, « localisation par immunofluorescence)
**Figure 18****:** Analyse par imagerie d'immunofluorescence confocale des zones périnucléaires présentant un fort signal FITC. Cellules transfectées avec pX3 CCC.
**Figure 19** **:** Représentation du panel de mutants CD™.
**Figure 20** **:** Schéma du plasmide Topo^{®} (pCR-Blunt II-TOPO) utilisé pour cloner les différents produits PCR. Le vecteur Topo fournit dans le kit Topo-blunt cloning (Invitrogen) est linéarisé et possède, à chacune de ses extrémités 3'-phosphate, la Topoisomérase I du virus de la vaccine, qui permet de liguer les produits de PCR avec le vecteur Topo linéarisé.
**Figure 21** **:** Vecteur d'expression pBluescript II KS (+).
**Figure 22****:** Schéma du plasmide pKSII-CD8α.
**Figure 23** **:** Schéma du plasmide rétroviral pLPCX utilisé pour l'expression des gènes chimères. Ces gènes sont introduits au niveau du site multiple de clonage.
**Figure 24** **:** Schéma des constructions chimériques finales clonées dans le vecteur d'expression rétroviral pLPCX.
**Figure 25** **:** Visualisation de l'expression et ciblage exosomal des protéines chimères. Western blot anti-CD™ et anti-CD8α pour les lysats cellulaires et exosomaux de l'ensemble des protéines mutantes et sauvage CD8α-CD™ (taille variant de 31 kDa à 27kDa) ainsi que du témoin négatif (vecteur d'expression pLPCX contenant le CD8α seul). Les sérums de lapin anti-CD™ et anti-CD8α sont dilués au 1/200^{ème}. L'anticorps secondaire anti-IgG de lapin couplé à la peroxydase est dilué au 1/5000^{ème}.
**Figure 26** **:** Résultats comparés des expériences de détection de CD8α associé aux exosomes par cytofluorimétrie de flux et Western Blot. Tableau donnant les résultats de l'expression et le ciblage vers les exosomes des différentes protéines chimères analysées. Les mutants signalés par une étoile sont significatifs pour le ciblage exosomal. La présence de CD8α sur les exosomes est repérée par cytofluorimétrie de flux au moyen d'un anticorps monoclonal de souris fluorescent spécifique d'un épitope conformationnel de la protéine CD8α (anticorps 53-6.7 de chez Pharmingen).

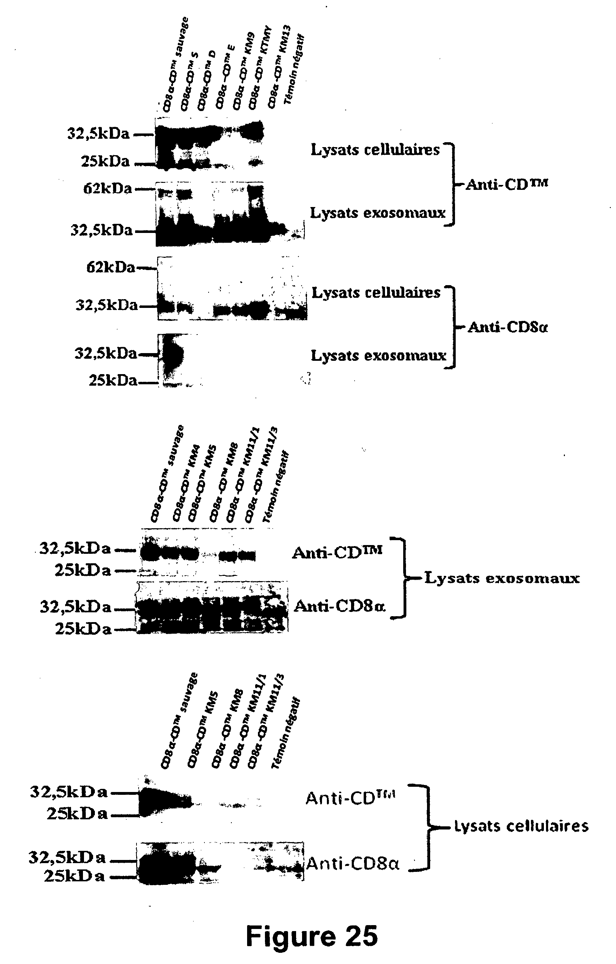

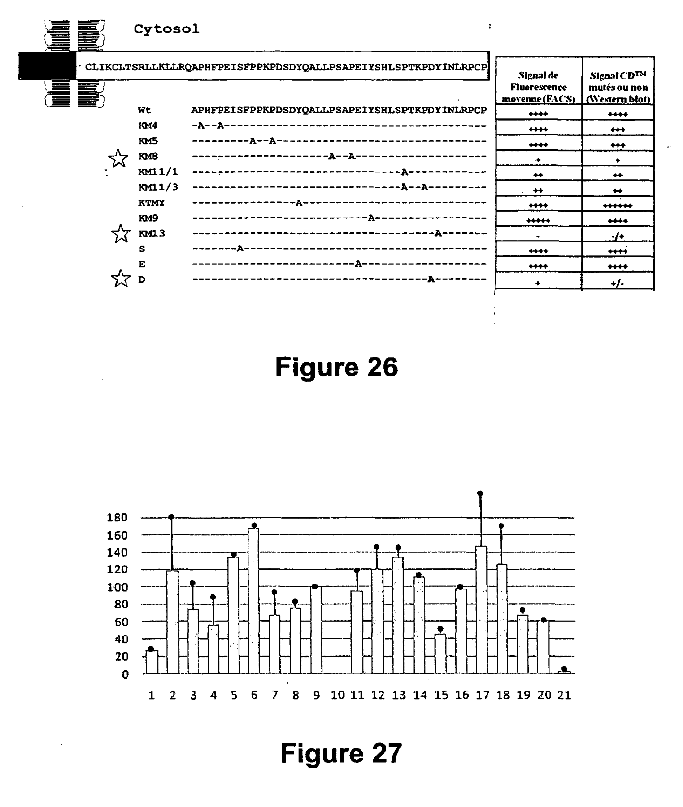

**Figure 27****: Expression du CD8 à la surface des exosomes.** Histogramme représentant la moyenne des mesures de l'exposition de chaque protéine chimère à la surface des exosomes. Ces mesures ont été effectuées par cytofluorimétrie de flux. L'exposition de chaque protéine chimère est exprimée en pourcentage par rapport à l'exposition de la protéine chimère CD8a-CDTM sauvage (construction n°9 sur l'histogramme = 100 %). L'écart type est indiqué. Les protéines chimères analysées sont : **1 :** témoin négatif (vecteur d'expression pLPCX contenant le CD8α seul) ; **2 :** KS5 ; **3 :** KS6 ; **4 :** KS8 ; **5 :** KS9 ; **6 :** KS10 ; 7 : KS12 ; **8 :** KS14 ; **9 :** séquence sauvage ; 10 : aucune construction ; **11 :** KM4 ; **12 :** KM5 ; **13 :** S ; **14 :** KTMY ; **15 :** KM8 ; **16** : E ; **17 :** KM9 ; **18 :** KM11/1 ; **19** : KM11/3 ; **20 :** D et **21 :** KM13. Les résultats observés confirment les résultats présentés en figures 25 et 26.
**Figure 28** **:** Représentation de différents plasmides d'expression pLPCX obtenus après clonage de gènes chimères codant pour des protéines à simple ou multiples domaines transmembranaires.
**Figure 29****:** A. Analyse par Western Blot anti-CD™-BLV réalisée sur les **extraits de protéines cellulaires** de cellules HEK293T non transfectées (N-T) ou transfectées avec les vecteurs d'expression pLPCX contenant les trois constructions codant pour les trois protéines chimères. L'anticorps primaire de lapin anti-CD™-BLV est utilisé dilué au 1/200. L'anticorps secondaire anti-IgG de lapin couplé à la peroxydase est utilisé au 1/2000. B. Les tailles des bandes observées correspondent aux tailles attendues et sont notées à droite.
**Figure 30** **:** A. Analyse Western Blot anti-CD™-BLV réalisée sur les **extraits de protéines exosomales** produites par des cellules HEK293T non transfectées (N-T) ou transfectées avec les vecteurs d'expression pLPCX contenant les trois constructions codant pour les trois protéines chimères. L'anticorps primaire de lapin anti-CD™-BLV est utilisé dilué au 1/200. L'anticorps secondaire anti-IgG de lapin couplé à la peroxydase est utilisé au 1/2000. B. Les tailles des bandes observées correspondent aux tailles attendues et sont notées à droite.
**Figure 31** **:** Révélation au Bleu de Coomassie des empreintes protéiques des différents extraits cellulaires (A) et exosomaux (B).

### EXEMPLES

### EXEMPLE 1

### MATERIEL ET METHODES

### I. Préparations des ADN plasmidiques :

### A. Transformation de bactéries :

Des bactéries compétentes DH5α (200µl) sont transformées par choc thermique avec 12,5 ng de chacun des 13 ADN étudiés codant pour les CD™ sauvages ou mutants du virus BLV, ainsi que par un plasmide dépourvu d'insert (pLPCX) faisant office de témoin négatif.

Les bactéries sont ensuite étalées sur un milieu LB/Agar contenant 50µg/ml d'ampicilline, à 37°C pendant 16h. Elles sont ensuite conservées à 4°C.

### B. Pré-culture et culture de bactéries :

Une colonie de chaque type de bactéries est ensuite mise en pré-culture dans 3 ml de milieu LB contenant 100 µg/ml d'ampicilline, à 37 °C sous agitation, pendant environ 8 h.

Chaque pré-culture servira à inoculer, au 1/200, deux flacons contenant chacun 250 ml de LB/Amp (100 µg/ml). L'incubation se fait à 37 °C, sous agitation, pendant 12 à 16 h.

### C. Maxipréparation STET :

Les cultures obtenues sont centrifugées (GR 412, Jouan) 20 min à une vitesse de 3600 rpm et à une température de 4 °C. Les culots sont repris dans 25 ml de tampon STET (*Sucrose 8* %, *Triton X100 5%, Tris HCl pH 8 50 mM, EDTA 50 mM)* auquel on ajoute 500µl de lysozyme (10 mg/ml ; Sigma) et 250 µl de RNase (2 mg/ml ; Sigma). Les tubes sont alors incubés 10 min à 100 °C puis centrifugés à 16000 rpm pendant 30 min. Les surnageants obtenus sont incubés 45 min à 65 °C en présence de 1mg/ml de protéinase K (Amresco). Les surnageants sont prélevés et l'ADN est précipité avec 0,15 volume d'acétate de sodium (AcNa) 3 M pH 6 et 0,6 volume d'isopropanol. Les solutions sont centrifugées (Aventi 30, Beckman) à 4 °C pendant 15 min à 15000 rpm. Les culots obtenus sont lavés avec 10 ml d'Ethanol avant d'être de nouveau centrifugés selon les paramètres précédents. Les acides nucléiques obtenus sont ensuite séchés et repris dans 2 ml de TE 1X et conservés à 4 °C.

La présence d'ADN super-enroulé est vérifiée, pour chaque produit de Maxipréparation, par électrophorèse de 0,2 µl et 1 µl des produits par électrophorèse sur gel d'agarose 0,8 %.

### D. Purification sur colonne :

Les maxipréparations STET permettent l'obtention de quantités importantes de plasmides, mais dont le degré de pureté peut être amélioré. Pour cela nous avons purifié chacun des 14 plasmides en réalisant un double passage sur colonnes AX100 (Kit Nucleobond PC 100, Macherey Nagel). Après précipitation à l'isopropanol, les plasmides purifiés obtenus sont repris dans 500 µl de TE1X et conservé à 4 °C.

La présence d'ADN super-enroulé est ensuite vérifiée par électrophorèse sur gel d'agarose (0,8 %) pour chaque éluat obtenu ainsi que pour la fraction non-retenue sur les colonnes (FT=flow-trough).

La concentration en ADN des solutions obtenues est évaluée par spectrophotométrie, à une longueur d'onde de 260 nm.

### E. Aliquotage et précipitation à l'EtOH :

On aliquote chaque type d'ADN par tubes de 50 ou 100 µg, puis on réalise une précipitation avec de l'ethanol (EtOH) et NaCl, sous hotte a flux laminaire, afin de stériliser les plasmides. Les culots obtenus sont repris à raison de 200 µl de TE1X pour 100 µg de plasmides, soit une concentration de 500 ng/µl. La présence d'ADN dans les aliquotes est vérifiée par électrophorèse sur gel d'agarose 0,8 %.

### F. Dosages spectrophotométriques des aliquotes obtenus :

Les aliquotes sont dosés par spectrophotométrie à une longueur d'onde de 260 nm. Les échantillons sont dilués au 1/50 et sont dosés dans un volume final de 500 µl.

### G. Digestions enzymatiques :

L'identité de chaque plasmide est contrôlée par digestion de 20 ng de chacun d'eux par des enzymes de restriction (New England Biolabs): le couple Hind III/Not I, Xba I, Aat II, Pac I, Sfo I. Les plasmides sont discriminés selon le nombre de bandes obtenus et leur poids moléculaire, après électrophorèse sur gel d'agarose (0,8 %) de chaque produit de digestions.

### II. Analyse de l'expression des chimères

### A. Culture cellulaire :

Les cellules HEK293 (Human Embryonic Kidney cells, cellules embryonnaires de rein humain) sont cultivées dans un milieu DMEM (Dulbecco's modified Eagle's médium), complémenté par 10 % de sérum de veau foetal (SVF) et 20 µg/ml de gentamicine, à 37 °C sous 5 % de CO₂.

### B. Transfection :

En vue des transfections, on ensemence 5.10⁵ cellules dans 2 ml de milieu par puits de 9,6 cm² (plaques 6 puits). Après incubation une nuit à 37 °C sous 5% de CO₂,le milieu est remplacé par du DMEM complet sans antibiotiques.

Les cellules sont alors transfectées par un polyplexe formé par la complexation, dans un tampon NaCl (0,15 M), de 6 µl de Jet PEI (Qbiogen) et 3 µg de chaque acide nucléique à tester; Un plasmide exprimant LacZ fait office de témoin positif pour la transfection. Après 24 h d'incubation à 37 °C sous 5 % de CO₂, le milieu est éliminé et remplacé par du DMEM complet avec 20 µg/ml de gentamicine. L'expression optimum des plasmides est obtenue 48 h après le début de la transfection.

Dans certains cas, afin d'analyser l'importance du transport vésiculaire dans la dégradation et le ciblage de CD™, nous avons utilisé des inhibiteurs de transport vésiculaire qui sont la bafilomycine et Ly 294002. 32 h après transfection, chaque inhibiteur est ajouté au milieu de culture à une concentration de 0,5 µM pour la bafilomycine et 10 µM pour Ly 294002.

### C. Extraction protéique :

48 h après transfection les cellules sont lysées par un tampon TNE-NP40 0,5% auquel on ajoute 0,1 mM de PMSF. Après clarification par centrifugation (14000 rpm, 15 min, 4 °C ; Eppendorf 5417R), les lysats sont dosés par spectrophotométrie (à 595 nm) selon la technique de Bradford.

Pour chaque échantillon, on prélève 200 µg de protéines que l'on complète par du tampon de lyse pour un volume finale de 30 µl auquel on rajoute 10 µl de tampon d'échantillon 4X (CB 4X : *NaOH 200 mM, EDTA 20 mM, SDS* 2%, *Vert de bromocrésol 0,05%, Glycérol 10%*).

### D. Préparation des exosomes :

Avant lyse des cellules, les milieux de cultures sont récupérés et précentrifugés à 10000 rpm pendant 20 min à une température de 4 °C (Aventi 30, Beckman). Les surnageants obtenus sont ensuite ultracentrifugés (Optima LE-80K, rotor Ti 50, Beckman) à 100000 g pendant 2 h à une température de 4 °C. Les culots obtenus sont repris dans 100 µl de CB 1X.

Nous avons aussi analysé les exosomes par sédimentation sur gradient de densité de sucrose. Les culots obtenus après ultracentrifugation des milieux de culture sont alors repris dans 100 µl d'une solution de sucrose à 0,25 M.
Les vésicules sont ensuite déposées sur un gradient de sucrose préparé avec 8 couches (de 1,2 ml) de densités différentes (en molarité) : 0,5 / 0,75 / 1 / 1,25 / 1,5 / 1,75 / 2 / 2,5. Les tubes sont alors centrifugés (Optima LE-80K, rotor SW 41, Beckman) à 39 000 rpm pendant 16 h, à une température de 4 °C.

Après centrifugation, on prélève le gradient par fraction de 700 µl. Les protéines sont ensuite précipitées par addition d'un même volume de TCA 30 %. Les tubes sont conservés 2h à 4°C puis centrifugés (Eppendorf, 5417R) à une température de 4 °C pendant 20 min à 13000 rpm. Les culots sont repris dans 500 µl d'acétone puis de nouveaux centrifugés comme précédemment.

Les culots alors obtenus sont repris dans 80 µl de CB1X puis analysés par migration sur gel d'acrylamide-SDS et Western Blot.

### E. Western Blot et Immunoprécipitation :

Les échantillons protéiques obtenus sont analysées par western blot : après migration et séparation sur gel d'acrylamide (12,5 %), les protéines sont transférées sur membrane hydrophobe PVDF (Immobillon-P, Millipore).

La présence des mutants de la protéine TM est révélée par immunomarquage à l'aide des anti-corps suivant :
- *Anticorps primaire :* antisérum de lapin Anti-CD™ BLV (Dilution 1/200).
- *Anticorps secondaire :* Anti-IgG de lapin marqué à la péroxydase (Dilution 1/5000 ; Jackson Immuno Research, JIR)

La présence de récepteurs à la transferrine est révélée par immunomarquage à l'aide des anti-corps suivant :
- *Anticorps primaire :* IgG de souris anti-TFr humain (Dilution 1/200 ; Zymed)
- *Anticorps secondaire :* Anti-IgG de souris (Dilution 1/5000 ; JIR)

Afin d'accroître sélectivement la concentration de la protéine étudiée, nous avons aussi réalisé des immunoprécipitations sur les lysats protéiques, préalablement à la migration sur gel. Après normalisation des quantités de protéines, l'adsorption du lysat se fait sur de la sepharose 6B, afin d'éliminer les réactions non spécifiques et l'immunoprécipitation est réalisée avec de la protéine sepharose 6A, en présence de 2,5 µg des anticorps suivant :
- *Anticorps primaires :*
   IgG de souris Anti-CD8 de souris (19/178) (JIR)
   IgG de rat Anti-CD8 de souris (53/672) (JIR)
- *Anticorps secondaires :*
   Anti-IgG de souris (JIR)
   Anti-IgG de rat (JIR)

Les culots obtenus sont alors repris dans 70 µl de CB 1,5X.

### III. Localisation par immunofluorescence

### A. Préparation des lamelles.

Les lamelles type coverslips sont stérilisées à l'EtOH absolu, sous hotte à flux laminaire, puis placées dans des puits de 1,9/cm² (plaques 24 puits) avant d'être « coatées » à la poly-L-Lysine (25 µg/ml, Sigma) pendant 1 h à 37 °C. Après lavage au PBS, les lamelles sont conservées à 4 °C, dans 1 ml de PBS.

### B. Culture cellulaire et transfection.

Les cellules HEK293 sont cultivées et transfectées selon la méthode décrite dans la partie « Analyses de l'expression des chimères ». 24 h après transfection, les cellules sont reprises dans 35 ml de DMEM complet, puis distribuées dans des puits de 1,9 cm² (plaques 24 puits), contenant les lamelles préalablement stérilisées et « coatées », à raison de 1 ml de dilution cellulaire par puits. Les cellules ainsi mises en culture sont incubées 24 h à 37 °C, sous 5 % de CO₂.

### C. Fixation et perméabilisation.

Les cellules sont ensuite fixées pendant 30 min avec une solution de formaldéhyde (4 %) puis perméabilisées au Triton X-100 (0,2 % final). Après 3 rinçages de 10 min au PBS, les lamelles sont conservées avec 1 ml de PBS, à 4 °C.

### D. Marquages pour l'immunofluorescence.

Les différents anticorps à disposition nous ont permis de tester plusieurs marquages pour la localisation par immunofluorescence:

### Marquages CD8 :

N°1 : IgG de souris / anti-CD8 de souris (19/178) (JIR) + Anti-IgG de souris FITC (JIR)
N°2 : Ascites de souris / anti-CD8 de souris (19/178) (JIR) + Anti-IgG de souris FITC (JIR)
N°3 : IgG de rat / anti-CD8 de souris (53/672) (JIR) + Anti-IgG de rat FITC (JIR)
N°4 : IgG de rat (53/6.7) / anti-CD8 de souris FITC (Pharmingen)

### Marquages des compartiments intra-cellulaires :

N°5 : IgG de souris / anti-CD63 (Lamp3) humain (Zymed) + Anti-IgG de souris Cy3 (Sigma)
N°6 : IgG de souris / anti-Lamp1 humain + Anti-IgG de souris Cy3 (Sigma)
N°7 : IgG de souris / anti-Tf2 humain (Zymed) + Anti-IgG de souris Cy3 (Sigma)
N°8 : IgG de lapin / anti-caveolin (BD) + Anti-IgG de lapin TRITC (JIR)

### RESULTATS

Les connaissances sur le processus de formation des exosomes sont partielles. De même, les fonctions associées au domaine cytoplasmique de Env sont mal caractérisées. La présente étude repose sur l'hypothèse que le modèle du BLV est un bon outil pour étudier les phénomènes de formations des exosomes et des particules virales et que le domaine cytoplasmique de la protéine TM (CD™) du BLV est un outil potentiellement intéressant pour le développement de la vaccination par « exosome display ».

Pour évaluer ces potentialités et afin de caractériser les fonctions responsables du ciblage de la protéine TM du BLV, et plus particulièrement le rôle des résidus cystéine palmitoylés ou non, nous avons développé plusieurs vecteurs permettant l'expression, au sein de cellules eucaryotes, de protéines transmembranaires chimères comprenant l'ectodomaine de la protéine CD8 de souris et le domaine CD™ (protéines chimères CD8-CD™). Des chimères CD8-CD™ sauvages ainsi que des chimères CD8-CD™ mutées ont été exprimées.

L'ectodomaine CD8 de souris est, dans une cellule humaine, un élément neutre n'interférant pas avec les récepteurs cellulaires. Les chimères utilisées nous assurent donc de l'absence d'interactions entre l'ectodomaine des protéines et les structures cellulaires.

En conséquences, ces chimères permettent d'étudier spécifiquement les domaines cytoplasmique et transmembranaire de la protéine TM, en évitant les phénomènes engendrés par l'ectodomaine de la protéine TM et par la protéine SU qui lui est associée.

Trois types de constructions ont été utilisés (voir figures 1 et 2). A partir de ces constructions, nous avons développé différents vecteurs d'expression pLPCX permettant l'expression de CD8-CD™ sauvages ou mutées au niveau des résidus cystéine 1, 2 et 3 (schématisé par le sigle : CCC). Les résidus cystéine sont alors remplacés, ou non, par des résidus alanine, qui ne peuvent pas être palmitoylés.

Les protéines CD8-CD™ sauvages ou mutés étudiées sont :
- 1 : pX2 CCC (phénotype sauvage)
- 2 : pX2 ACC
- 3 : pX2 CAC
- 4 : pX2 AAC
- 5 : pX2 CCA
- 6 : pX2 ACA
- 7 : pX2 CAA
- 8 : pX2 AAA
- 9 : pX3 CCC (phénotype sauvage)
- 10 : pX3 CAC
- 11 : pX4 - - C (phénotype sauvage)
- 12 : pX4 - - A
- 13 : pX4 stp (*pX4 stp est composé uniquement par ED, tmD et une petite partie du CD™ de CD8).*

Ces constructions nous ont permis d'étudier, au niveau du ciblage de la protéine TM : les conséquences de la mutation des résidus cystéine, l'importance de l'intégrité du domaine trans-membranaire de la protéine TM, et l'importance du domaine cytoplasmique de la protéine TM (CD™).

### I. Préparation des ADN plasmidiques :

Dans un premier temps, nous avons produit, en grande quantité, chacun de nos 13 ADN ainsi qu'un vecteur sans insert (pLPCX) faisant office de témoin négatif. Après culture de bactéries transformées par nos différents plasmides, les ADN obtenus ont été purifiés successivement par la méthode STET puis sur colonne. L'identité des plasmides obtenus a ensuite été contrôlée par digestion enzymatique.

### A. Préparations des ADN par la méthode STET :

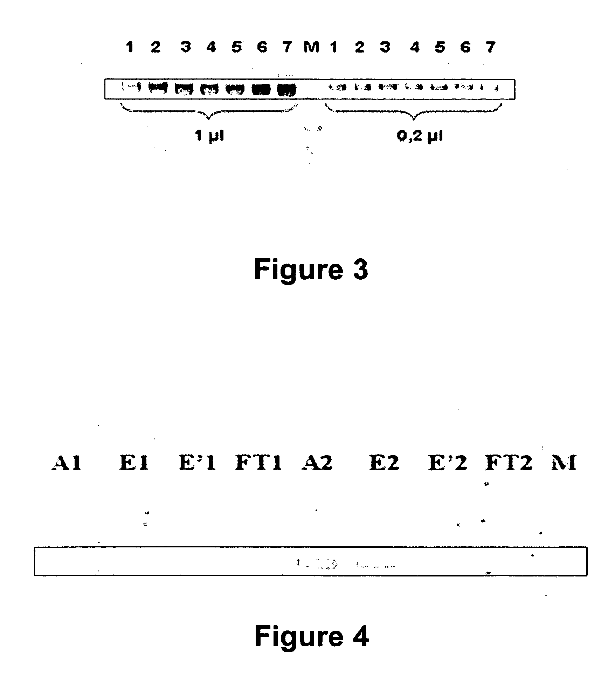

Afin de vérifier l'intégrité des ADN plasmidiques et le rendement des préparations STET, nous avons réalisé une migration de 1µl et 0,20µl de chacun d'eux, sur gel d'agarose (0,8% ; voir figure 3). La non-dégradation des ADN est mise en évidence par la présence de bandes distinctes d'ADN super enroulés de tailles homogènes.

### B. Contrôle de la présence des ADN dans les produits de purification sur colonne :

Les ADN obtenus par la méthode STET sont purifiés sur colonnes, les fractions retenues puis éluées ainsi que les fractions non retenues sont ensuite analysées par électrophorèse sur gel afin de vérifier l'intégrité des ADN purifiés obtenus. Le gel présenté en figure 4 nous indique que l'ADN super-enroulé est bien présent dans les fractions pures éluées des colonnes (E et E') mais indétectable dans les fractions non retenues (FT).

### C. Dosages spectrométriques des aliquotes et ajustement des concentrations des ADN après digestion enzymatique :

Les ADN purifiés sont ensuite dosés au spectrophotomètre. Les concentrations alors obtenues varient, en fonction des aliquotes, entre 149 µg/ml et 584 µg/ml. Après précipitation à l'EtOH, les ADN des aliquotes sont repris dans du TE1X et ajustés à une même concentration.

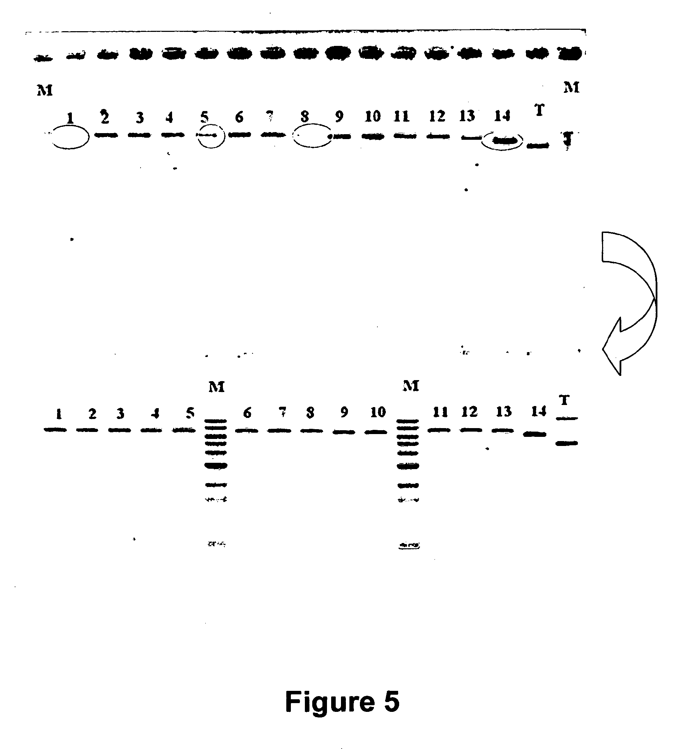

Afin de vérifier les concentrations d'ADN, nous avons linéarisé les plasmides par digestion enzymatique, à l'aide du couple Hind III/Not I, puis nous les avons analysé sur gel d'électrophorèse (voir figure 5, profil supérieur). Il est apparu que l'intensité des bandes obtenues était encore variable. Nous avons donc réajusté les concentrations en fonction des dosages spectrométriques et de l'intensité des bandes obtenues après la digestion (voir figure 5 - profil inférieur). L'obtention de bandes d'intensités égales, en plus d'assurer l'exactitude des concentrations de nos ADN avant leurs transfections dans des cellules, permettra une meilleure lisibilité des électrophorèses après digestions enzymatiques, lors du contrôle de l'identité des plasmides.

### D. Contrôle de l'identité des ADN après digestion enzymatique.

### 1) Principe général :

Afin de s'assurer de l'identité des 14 ADN préparés, tous sont contrôlés par digestion à l'aide des enzymes de restriction Hind III et Not I, Xba I, Aat II, Pac I ainsi que Sfo I qui doivent résulter en des combinaisons différentes de profils sur gels pour chacun des ADN. La migration sur gel d'agarose (0,8%) des produits de digestions permet la discrimination des ADN en fonction du nombre et de la taille des bandes obtenues, comme indiqué dans le tableau 1 (remarque : les bandes d'une taille inférieure à une centaine de paires de bases ne sont pas visibles sur nos gels d'électrophorèse) :

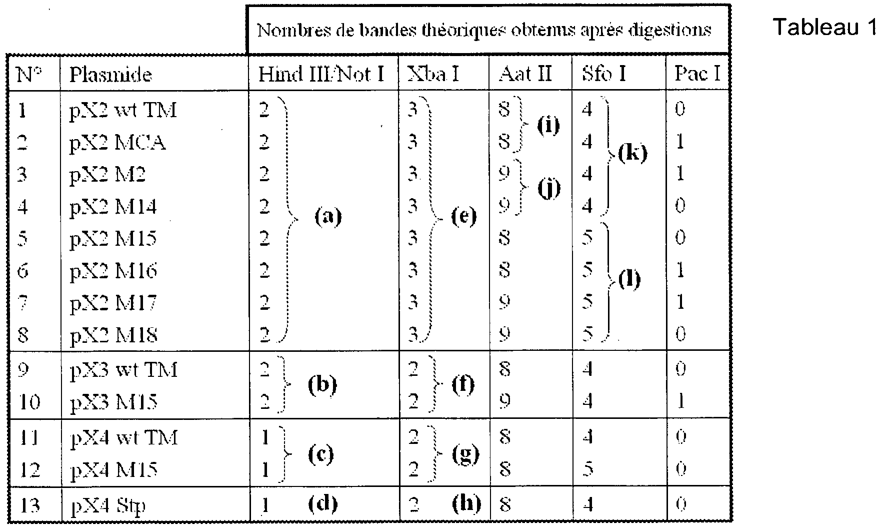

La digestion des constructions étudiées permet de s'assurer de l'identité de chaque plasmide. La discrimination des grands types de construction CD8-CD™ se fait par l'analyse du nombre et de la taille des bandes obtenus après digestion avec les enzymes Hind III/Not I et Xba I. La discrimination entre les différents mutants des résidus cystéine se fait après digestion par les enzymes Aat II, Pac I et Sfo I. Le nombre de bandes alors obtenus est plus important qu'avec les digestions par Hind III/Not I et Xba I, et la discrimination est réalisée en se basant principalement sur la présence ou non de bandes spécifiques (en gras).
Tailles des bandes attendues (en bp) :

| | |
|---|---|
| **a)** : 7206 / 49 | **(g)** : 6552/610 |
| (**b) :** 6868 / 311 | **(h)** : 6552 / 385 |
| **(c)** : 7162 | **(i)** : **4285** / 1055 / 801 / 498 / 294 / 186 / 83 / 53 |
| **(d)** : 6937 | **(j)** : **3423** / 1055 / **862** / 801 / 294 / 186 / 83 / 53 |
| **(e)** : 6822 / 367 / 66 | **(k)** : 4276 / **2538** / 310 / 131 |
| **(f)** : 6812/367 | **(l)** :4276 / **1839** / **699** / 310 / 131 |

### 2) Exemple:

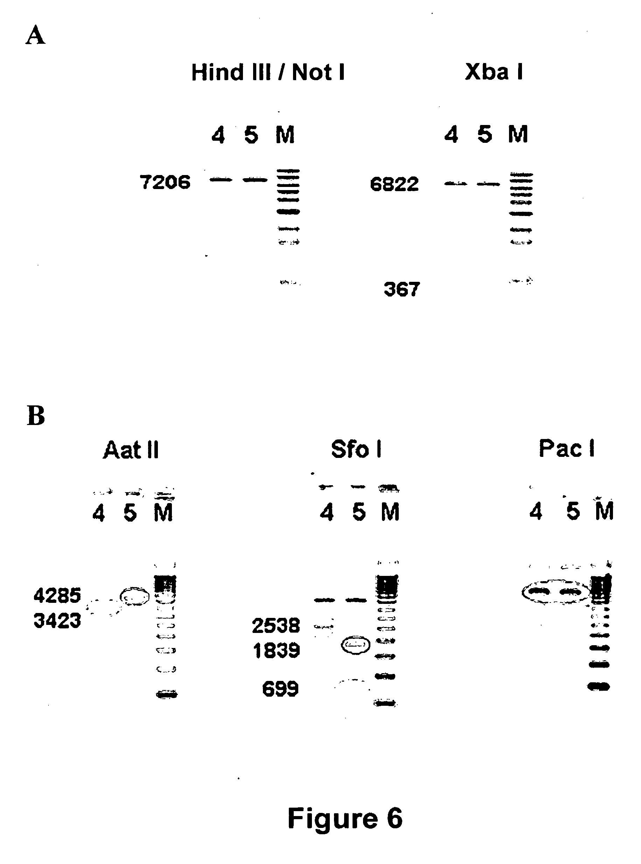

Pour la discrimination des mutants pX2 AAC et pX2 CCA, les gels obtenons sont représentés en figure 6A.

### Interprétation :

- Digestion par Hind III/ Not I : l'obtention d'une bande unique, à environ 7206 pb, correspond à la présence de constructions X2 ou X4 dans chacun des deux échantillons.
- Digestion par Xbal : l'obtention de deux bandes à environ 6822 (ou 6812) et 367 bp correspondent à la présence de constructions X2 ou X3 dans chacun des deux échantillons.

En recoupant ces deux résultats nous pouvons affirmer que nous sommes donc bien en présence de ***deux constructions de type X2.***
On cherche ensuite à discriminer les mutants X2 entre eux (voir figure 6B):

### Interprétation :

- Digestion par AatII :
   - **Echantillon 4 :** l'obtention d'une 1 bande spécifique avoisinant 3423 pb (rouge) correspond à la présence d'un des plasmides X2 suivant : ***pX2 CAC, pX2 AAC, pX2 CAA*** ou ***pX2 AAA.***
   - Echantillon 5 : l'obtention d'une 1 bande spécifique avoisinant 4285 pb (bleu) correspond à la présence d'un des plasmides X2 suivant : ***pX2 CCC, pX2 ACC, pX2 CCA*** ou ***pX2 ACA.***
- Digestion par Sfo I :
   - **Echantillon 4 :** l'obtention d'une bande spécifique avoisinant 2538 pb (rouge) correspond à la présence d'un des plasmides X2 suivant : ***pX2 CCC, pX2 ACC, pX2 CAC, pX2 AAC.*** En recoupant ce résultat avec celui de la digestion par Aat II, nous pouvons alors en déduire que nous sommes en présence d'un des plasmides suivant : ***pX2 AAC*** ou ***pX2 CAC.***
   - **Echantillon 5 :** l'obtention de 2 bandes spécifiques avoisinant 1839 pb (noir) et 699 (vert) pb correspond à la présence d'un des plasmides X2 suivant : ***pX2 CCA, pX2 ACA, pX2 CAA, pX2 AAA.*** En recoupant ce résultat avec celui de la digestion par Aat II, nous pouvons alors en déduire que nous sommes en présence d'un des plasmides suivant : ***pX2 CCA*** ou ***pX2 ACA.***
- Digestion par Pac I :
   Nous obtenons pour chaque échantillon, 1 bande de faible intensité correspondant à de l'ADNp non super enroulé ainsi qu'une bande de forte intensité correspondant à nos ADNp super enroulé (bleu) mettant en évidence l'absence de digestion par Pac I, pour ces échantillons.

Les échantillons 4 et 5 correspondent respectivement aux mutants ***pX2 AAC*** et ***pX2 CCA.***

Ce processus a été appliqué à l'identification de tous les plasmides.

### 3) Conclusion :

Lors de cette étape de préparation des ADN, nous avons obtenus plusieurs milligrammes de chacun des 14 plasmides purifiés et leurs identités ont toutes été confirmées après contrôle par digestions enzymatiques et analyses sur gels. De plus, tous les ADN ont été ajustés à une même concentration.

### II. Analyse de l'expression des chimères :

Afin d'évaluer l'expression des ADN, une même quantité de chacun des plasmides a été transfecté au sein de cellules HEK293. Les protéines ainsi exprimées sont analysées par migration sur gel suivie d'un transfert sur membrane PVDF. Les membranes sont ensuite révélées à l'aide d'un sérum de lapin Anti-CD™ et d'un anticorps secondaire Anti-Lapin marqué à la péroxydase.

***A. Analyse de la présence de CD8*-*CD*™ *au sein des lysats cellulaires :***

### 1) Analyses des lysats bruts :

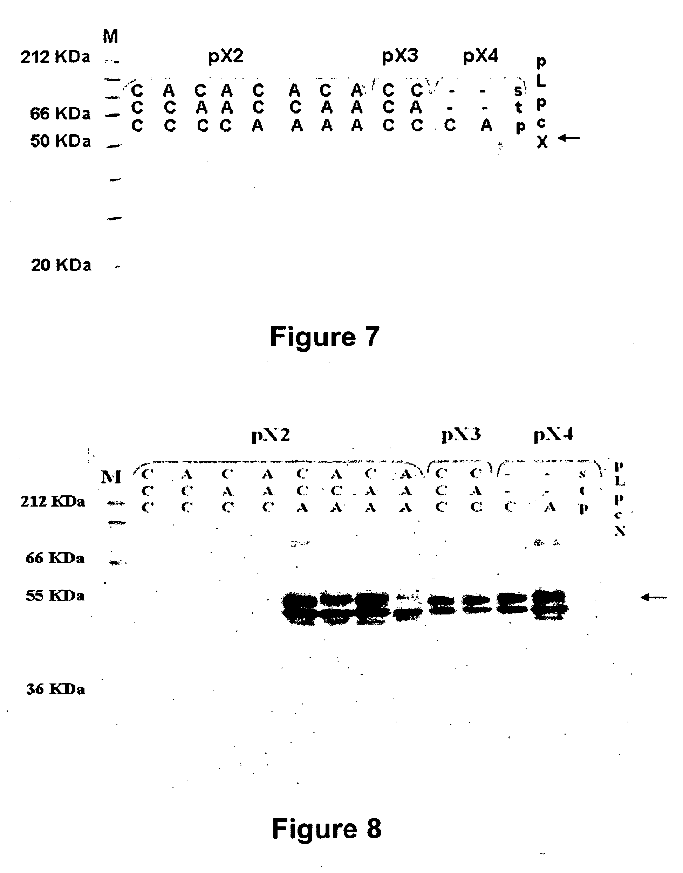

Après 48h de transfection, les cellules sont lysées et les extraits obtenus sont dosés selon la technique de Bradford. Nous avons analysé 200µg de protéines brutes, issues de ces lysats, par migration sur gel et western blot révélé avec un anticorps anti-CD™ (voir figure 7).

Différents signaux spécifiques sont alors observables selon les échantillons :
- Une double bande à 50 kDa (bleu) correspondant à la présence de CD8-CD™. Les deux bandes correspondent à deux niveaux de glycosylation de CD8.
- Un signal à environ 20 kDa (rouge) d'origine indéterminée.

Les échantillons pX2 CCC, pX2 ACC, pX2 CAC, pX2 AAC, pX4 stp et pLPCX ne présentent pas ces bandes.

### 2) Analyse des lysats après immunoprécipitation par des anticorps anti-CD8 :

Afin d'abaisser le seuil de détection de CD8-CD™ au sein des lysats cellulaires, nous avons utilisé une grande quantité d'extrait et concentré les chimères par immunoprécipitation avec des anticorps monoclonaux spécifique d'un épitope conformationnel de l'ectodomaine du CD8.
Après 48h de transfection, les cellules sont lysées et les extraits obtenus sont dosés selon la technique de Bradford. Après normalisation des concentrations en protéines, les extrait sont immunoprécipités en présence des anticorps Anti-CD8 et de protéine A sepharose. Les produits obtenus sont analysés par migration sur gel et Western Blot révélés avec un anticorps anti-CD™ (voir figure 8). Différents signaux sont alors observables selon les échantillons :
- Une double bande à 55 kDa (bleu) correspondant à la présence de CD8-CD™.
- Un signal non identifié, retrouvé pour chaque échantillon, à environ 66 kDa.
- Un ou plusieurs signaux d'un poids moléculaire supérieur à 66 KDa correspondant probablement à la présence de protéines CD8-CD™ multimérisées.

Ici, l'échantillon pX2 AAC présente un signal détectable à 50KDa contrairement à l'analyse effectuée sans immunoprécipitation. En revanche aucun signal n'est détectable pour les échantillons pX2 CCC, pX2 ACC, pX2 CAC, pX4 stp et pLPCX.

### 3) Récapitulatifs de l'analyse de l'expression de CD8-CD™ au sein de lysats cellulaires :

En corrélant les données obtenues par western blot sur les lysats cellulaires avec les caractéristiques de chaque chimère (intégrité de tmD BLV, présence du troisième résidu de cystéine et nombre de résidus cystéine), nous pouvons établir le tableau 2 ci-dessous.

Il apparaît que, en plus du CD™ du BLV, deux facteurs puissent jouer sur la présence des chimères CD8-CD™. Ces facteurs sont : la présence ou non de tmD BLV, et la présence ou non du troisième résidu de cystéine (Cys 213).

**Tableau 2**

| Construction | tmD BLV | Nbre de Cystéines | Signal CD™ BLV |
|---|---|---|---|
| p×2 CC**C** | Complet | 3 | Nul |
| p×2 AC**C** | Complet | 2 | Nul |
| p×2 CA**C** | Complet | 2 | Nul |
| p×2 AA**C** | Complet | 1 | + |
| p×2 CC**A** | Complet | 2 | + + + + |
| p×2 AC**A** | Complet | 1 | + + + + |
| p×2 CA**A** | Complet | | + + + + |
| p×2 AA**A** | Complet | 0 | + + |
| p×3 CC**C** | - 15 aa | 3 | + + + |
| p×3 CA**C** | -15 aa | 2 | + + + |
| p×4 - - **C** | Absent | 1 | + + + + |
| p×4 - - **A** | Absent | 0 | + + + + + |
| p×4 stp | Absent | 0 | Nul |
| pLpo× | Absent | 0 | Nul |

### B. Analyse de l'association de CD8-CD™ avec les exosomes :

Les différents ADN utilisés ne varient que de quelques nucléotides. Il est donc probable que les quantités de CD8-CD™ synthétisées soient équivalentes. Cependant il est apparu, lors de l'analyse de l'expression de CD™ au sein de lysats cellulaires, que certaines chimères ne sont pas détectables 48h après transfection. Cette absence de signal serait le reflet de la disparition de certaines de nos protéines chimériques. Cette disparition pourrait être due soit à une dégradation rapide de ces chimères, soit à leur sécrétion sous forme d'exosomes. Nous avons donc cherché à détecter la présence de CD8-CD™ au sein d'exosomes.

### 1) Contenu en CD8-CD™ des vésicules isolées par centrifugation :

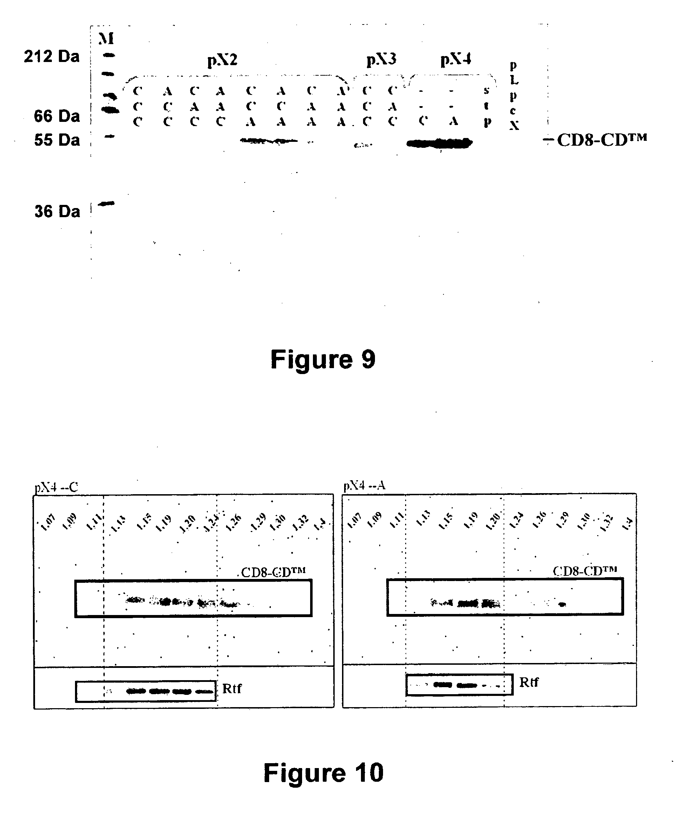

Après 48h de transfection, les milieux de cultures sont récupérés et centrifugés en vue d'isoler les exosomes. Les culots obtenus sont alors analysés par migration sur gel et Western Blot révélé avec un anticorps anti-CD™ (voir figure 9).

On peut visualiser, selon les échantillons, un signal à 55 kDa correspondant à la présence de CD8-CD™. Ce signal est non détectable pour les échantillons pX2 CCC, pX2 ACC, pX2 CAC, pX2 AAC, pX4 stp et pLPCX.

### 2) Contenu en CD8-CD™ après sédimentation des vésicules sur gradient de densité de sucrose :

Afin de nous assurer que le signal obtenu après ultracentrifugation des milieux de culture est bien du à la présence des chimères au sein d'exosomes et non pas à la présence de débris cellulaires contenant CD8-CD™, nous avons analysé les culots d'exosomes obtenus selon la méthode décrite précédemment par sédimentation sur un gradient de densité de sucrose. Les exosomes flottent alors à une densité allant de 1,13 à 1,20 g/ml en fonction des cellules utilisées et de la composition des vésicules. Après la sédimentation par ultracentrifugation, le gradient est prélevé par fractions de 700µl. Les protéines sont précipitées à l'aide de TCA et analysées par migration sur gel puis Western Blots révélés avec un anticorps anti-CD™ et un anticorps anti-récepteur de la transferrine (RTf) permettant de détecter la présence de vésicules cellulaires (endosomes ou exosomes). Cette analyse a été réalisée pour les mutants pX4 -C et pX4 --A.(voir figure 10).

Chez pX4 --C et pX4 --A, on détecte un signal correspondant à CD8-CD™ dans les fractions de densité allant de 1,13 à 1,25 g/ml. Pour ces mêmes densités, nous avons également pu détecter un signal correspondant à RTf. Ces résultats révèlent la présence de CD8-CD™ dans les fractions contenant les exosomes.

### 3) Récapitulatif de l'analyse de l'association des exosomes avec CD8-CD™ :

L'association de CD8-CD™ avec les exosomes a été détectée pour tous les mutants contenant CD™ exceptés pour les pX2 possédant Cys 213. Les mutants pX4 --C et pX4 --A apparaissent comme très efficacement ciblés dans les exosomes. Les mutants pX3 et pX2 ne possédant pas Cys 213 sont eux aussi retrouvés dans les exosomes mais dans des proportions moindre que pour les mutants pX4 --C et pX4--A.

Il apparaît donc que, en plus de la présence du CD™ viral, deux facteurs puissent jouer sur le ciblage des chimères CD8-CD™ dans les exosomes.
Ces facteurs sont :
- La présence ou non du domaine transmembranaire du BLV.
- La présence ou non de la Cys 3.

### C. Influence de l'inhibition du transport vésiculaire sur l'expression des chimères CD8-CD™:

L'absence de détection des chimères pX2 CCC, pX2 ACC, pX2 CAC et pX2 AAC n'étant pas due à une sécrétion exosomale accrue, nous avons cherché à savoir si elle pouvait être due à une dégradation par la voie de tri des protéines des MVBs vers les lysosomes.

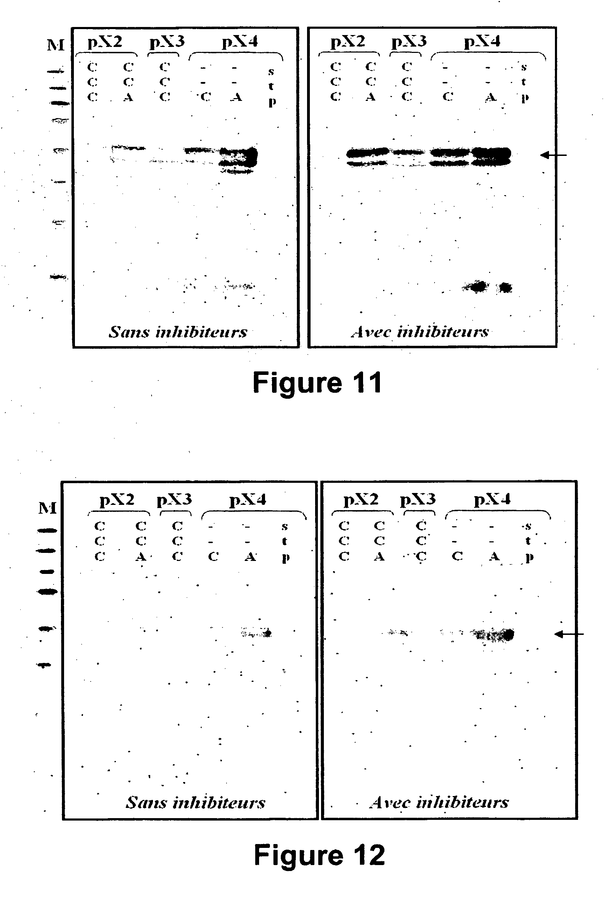

Pour cela, nous avons utilisé les inhibiteurs de transport vésiculaire que sont la bafilomycine et Ly294002. Ces inhibiteurs permettent de bloquer la voie de dégradation lysosomale, favorisant ainsi la sécrétion de protéines par les exosomes. Les inhibiteurs sont ajoutés aux milieux de cultures 32h après la transfection ; 16h plus tard, les cellules sont lysées et les milieux de cultures sont récupérés puis centrifugés en vue d'isoler les exosomes. Les échantillons obtenus sont alors analysés par migration sur gel et Western Blot révélé avec un anticorps anti-CD™. Nous avons ainsi analysé l'expression des chimères pX2 CCC, pX2 CCA, pX3 CCC, pX4 -- C, pX4 -- A et pX4 stp .(voir figures 11 et 12).

Il apparaît que, pour ces mutants, le profil des Western Blots des protéines obtenues en présence d'inhibiteurs est le même que lors de l'analyse sans inhibiteur, que cela soit pour les lysats cellulaires ou pour les exosomes.

L'absence des chimères pX2 CCC, pX2 ACC, pX2 CAC et pX2 AAC n'est donc apparemment due ni à une sortie exosomale accélérée, ni à une dégradation dans les lysosomes. Il est probable qu'elle soit la conséquence d'une dégradation très précoce au niveau du système de contrôle du repliement au sein du réticulum endoplasmique (RE) et du TGN.

### III. Localisation par immunofluorescence :

Afin d'apprécier le ciblage membranaire de CD8-CD™ ainsi que sa localisation dans les compartiments cellulaires, nous avons réalisé des observations en microscopie d'immunofluorescence confocale. 48h après transfection, les cellules ont été fixées puis marquées à l'aide de différents anticorps.

### A. Choix des anticorps :

Dans un premier temps, chaque type de marquage (voir Matériels et Méthodes) est testé, avec des dilutions d'anticorps variables, sur des cellules fixées exprimant pX4 -- C ou pLPCX. Après observation sur un microscope à immunofluorescence classique (ZEISS Axiovert 200 M), nous avons testé les différents anticorps à disposition (voir partie « Matériels et Méthodes ») et déterminé leur efficacité ainsi que leurs dilutions optimales. Il est apparu que plusieurs anticorps anti-compartiments intra-cellulaires présentaient un signal nul ou aspécifique.

Pour l'observation en imagerie confocale, nous n'avons donc pu utiliser que deux types de marquages :
- Marquage CD8-CD™ :
   IgG de rat (53/6.7) Anti-CD8 de souris FITC (Pharmingen, dilution 1/50)
- Marquage Lamp3 :
   IgG de souris Anti-CD63 (Lamp3) humain (Zymed, dilution 1/50) + Anti-IgG de souris Cy3 (Sigma, dilution 1/500)

### B. Observations de la répartition de CD8-CD™ :

Après fixation et marquage des cellules à l'aide de nos différents anticorps, nous avons observé la répartition du marquage issu de CD8-CD™ ainsi que sa colocalisation avec Lamp3 à l'aide d'un microscope confocal (ZEISS LSM 510).

Les cellules exprimant pLPCX (témoin négatif) ne présentent pas de signal FITC. Ce témoin nous permet donc de s'assurer que la fluorescence FITC visualisée pour les autres mutants est bien issue de la présence de CD8-CD™.

De façon surprenante, malgré une absence de détection par Western Blot, les constructions pX2 comprenant la Cys 3 sont visibles, bien que faiblement, en immunofluorescence.

Analyse des phénotypes généraux :
Les cellules HEK293 sont transfectées pendant 48h puis fixées. L'observation est réalisée à l'aide d'un microscope confocale ZEISS LSM 510 (objectif X63 à immersion).
**CD8** : Marquage FITC (vert) révélant la présence des chimères contenant le CD8. **Lamp3 :** Marquage Cy3 (rouge) révélant de la présence de la protéine Lamp3 caractéristique des endosomes tardifs.
**CD8-CD™ / Lamp3 :** Superposition des images FITC et Cy3. La nuance jaune obtenu met en évidence la colocalisation de CD8-CD™ avec Lamp3.

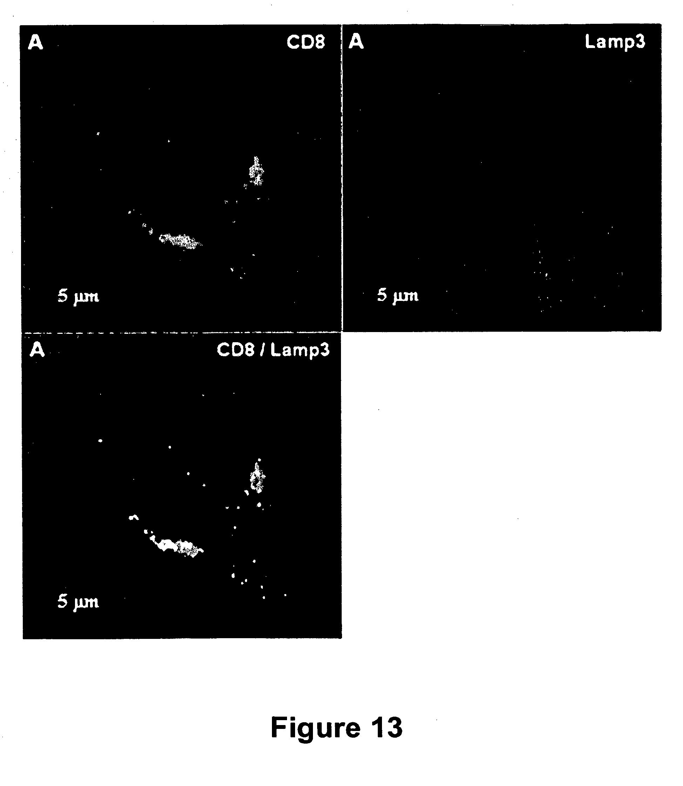

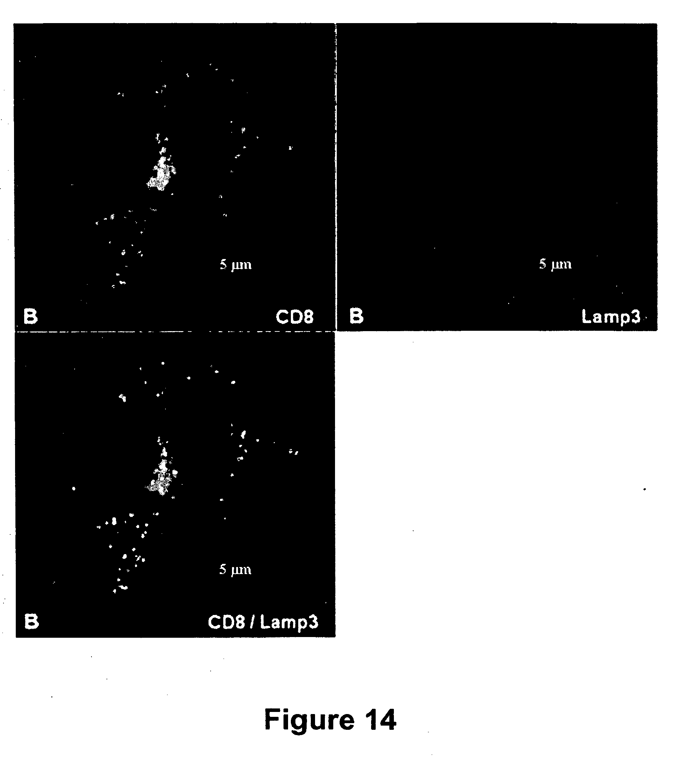

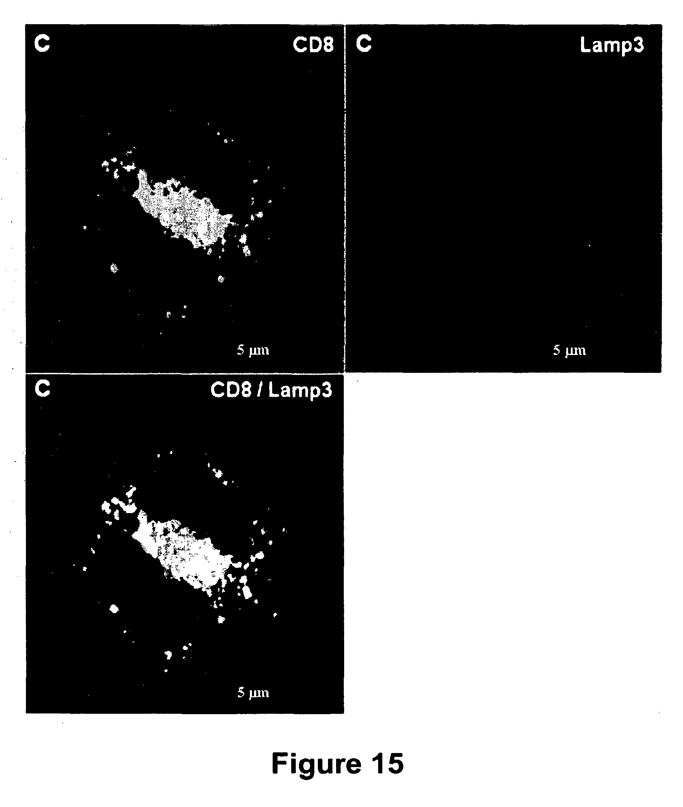

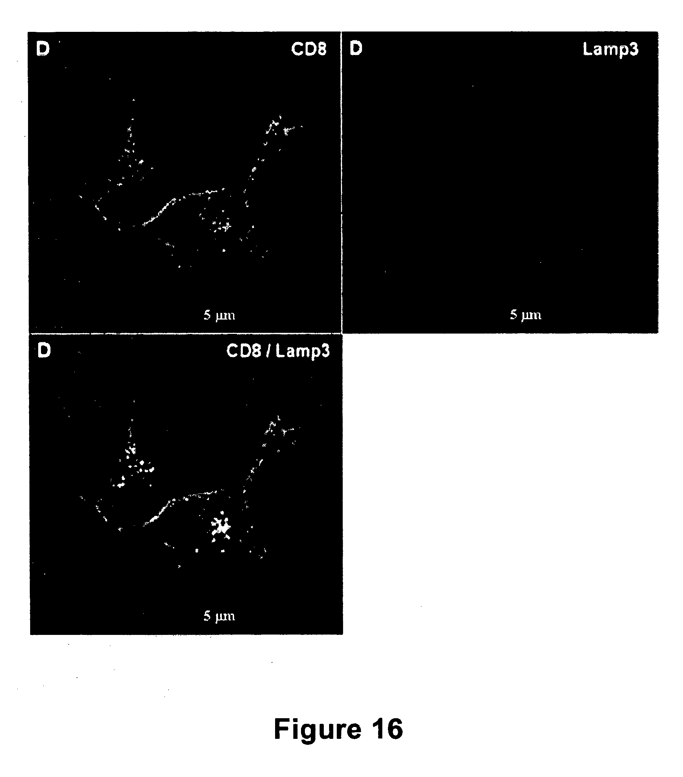

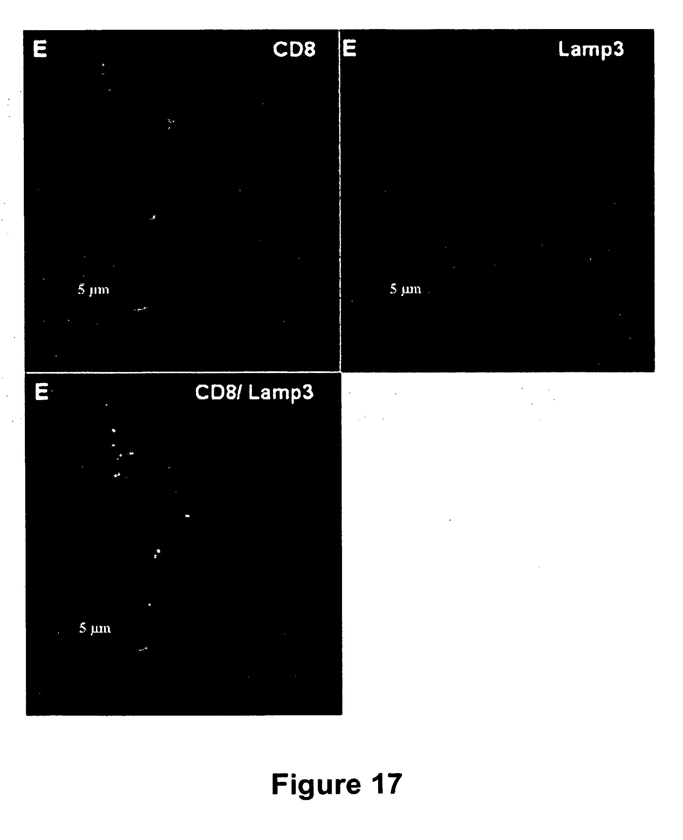

Après observations, nous avons pu distinguer 5 phénotypes généraux (voir figures 13 à 17) en se basant sur l'aspect (vésiculaire, membranaire ou périnucléaire) et l'intensité du marquage FITC. La colocalisation du marquage FITC d'aspect vésiculaire avec Lamp3 ne rentre pas en ligne de compte pour la détermination de ces phénotypes car elle est avérée chez tous les mutants. De plus cette co-localisation est toujours partielle.

Analyse des zones périnucléaires présentant un fort signal FITC :

Pour tous les échantillons, excepté pX4 stp, on retrouve de manière plus ou moins constante un signal périnucléaire CD8 homogène important à la périphérie du noyau. Les paramètres d'intensité requis pour la visualisation de la majeure partie de la fluorescence FITC présente dans les cellules entraînent la saturation de cette zone. Un signal ainsi saturé étant peu exploitable, nous avons réalisé des acquisitions de paramètres d'intensité plus faible, en nous concentrant sur l'analyse de ces zones, notamment pour déterminer la pertinence des colocalisations qu'elles présentent avec Lamp3.
**CD8-CD™ / Lamp3 [a] :** Colocalisation de CD8-CD™ (vert) avec Lamp3 (rouge). Les paramètres d'intensité pour l'acquisition, mêmes faibles, entraînent une saturation du signal périnucléaire et l'apparition de nuances jaunes à ce niveau, mettant en évidence une colocalisation partielle entre ces deux marquages. En raison de la saturation du signal, il est impossible de dire si la colocalisation mise en évidence est réelle ou artéfactuelle.
**CD8-CD™ [d]** : Signal FITC issu de CD8-CD™. Les paramètres d'intensité ont été abaissés sensiblement afin d'éliminer tout phénomène de saturation du signal. Le signal FITC apparaît diffus, homogène et non ponctué.
**CD8-CD™ / Lamp3 [d] :** Colocalisation de CD8-CD™ (vert) avec Lamp3 (rouge). Les paramètres d'intensité ont été abaissés sensiblement afin d'éliminer tout phénomène de saturation du signal. Il en résulte une absence de nuances jaunes mettant en évidence l'absence de colocalisation entre FITC et Lamp3.

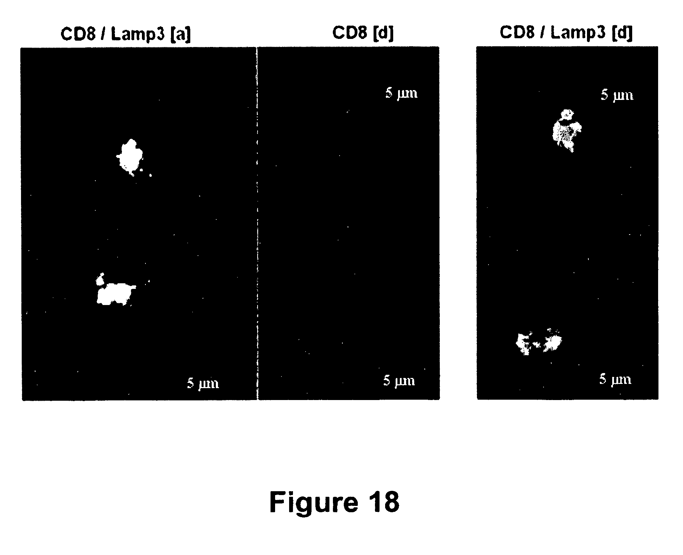

D'après ces acquisitions, le marquage issu de Lamp3 apparaît en fait comme étant situé à l'intérieur de cette zone périnucléaire mais n'est en tout cas jamais colocalisé avec le marquage FITC (voir figure 18).

De plus le marquage issu de CD8-CD™ de ces zones périnucléaires apparaît diffus, homogène et non ponctué, et semble mettre en évidence la présence de CD8-CD™ au sein d'une structure cellulaire. La localisation et l'aspect de ce marquage, ainsi que l'absence de colocalisation réelle avec Lamp3 suggèrent la présence de CD8-CD™ dans l'appareil de Golgi.

Ce phénotype est retrouvé, de façon plus ou moins intense, chez tous les mutants, hormis pX4 stp. Chez les mutants pX4 --C et pX4 --A, ce phénotype n'est visible que dans une minorité de cellules, contrairement aux mutants pX2 et pX3 chez qui il est présent de manière constante.

Ainsi, l'observation spécifique des zones périnucléaires fortement marquées par FITC nous a permis de mettre en évidence la présence probable de CD8-CD™ au sein du TGN.

D'après les données obtenues, nous pouvons établir le tableau récapitulatif ci-dessous (voir tableau 3).

Ces observations confirment que, outre le CD™ viral, deux facteurs jouent un rôle sur la stabilité et le ciblage de nos chimères.

Ces facteurs sont :
- La présence ou non de tmD BLV.
- La présence ou non des résidus N-terminaux du CD™ du BLV
- La présence ou non de la Cys 3.

**Tableau 3**

| | | | Localisation du marquage FITC | | |
|---|---|---|---|---|---|
| Phénotype | Mutants | Intensité FITC | Vésiculaire | Membranaire | TGN |
| **A** | pX2 CC**C** | + | +++ | - | ++ |
| | pX2 AC**C** | + | +++ | - | ++ |
| | pX2 CA**C** | + | +++ | - | ++ |
| | pX2 AA**C** | + | +++ | - | ++ |
| **B** | pX2 CC**A** | +++ | ++++ | - | ++ |
| | pX2 AC**A** | +++ | ++++ | - | ++ |
| | pX2 CA**A** | +++ | ++++ | - | ++ |
| | pX2 AA**A** | +++ | ++++ | - | ++ |
| **C** | pX3 CC**C** | ++++++ | + | +++ | +++++ |
| | pX3 CA**C** | ++++++ | + | +++ | +++++ |
| **D** | pX4 - - **C** | ++++++ | ++ | ++++ | + |
| | pX4 - - **A** | ++++++ | ++ | ++++ | + |
| **E** | pX4 stp | ++++ | + | +++ | - |

### CONCLUSION :

Au cours de nos analyses, il est apparu que la chimère pX4 stp, composée uniquement de l'ectodomaine et du tmD de CD8, s'accumule dans les cellules HEK293 en étant efficacement ciblée vers la membrane plasmique.

De la même façon, les chimères pX4 --C et pX4 --A s'accumulent et se retrouvent dans la membrane plasmique mais dans des proportions plus importantes que pX4 stp. Ces chimères sont très efficacement sécrétées au sein d'exosomes.
Les chimères pX3 quant à elles sont très présentes au sein de l'appareil de Golgi, contrairement aux chimères pX4. Leur ciblage au sein de la membrane plasmique et dans les exosomes parait moins efficace que chez les chimères pX4 mais demeure important.

Les chimères pX2 apparaissent peu stables et sont retrouvées au sein du TGN mais pas dans la membrane plasmique. Les constructions pX2 comportant Cys 3 sont très peu stables dans les cellules et ne sont pas détectées dans les exosomes. La substitution de la Cys terminale dans ce type de constructions semble contribuer à un gain de stabilité des protéines chimériques. En effet, les constructions pX2 sans Cys 3 sont détectables dans les cellules et dans les exosomes.

Toutes les chimères étudiées présentent, en immunofluorescence, un marquage vésiculaire qui est partiellement colocalisé avec un marqueur des endosomes tardifs..

Cette étude a permis de mettre en évidence l'importance de la présence, ou non, de Cys 3 dans la stabilité et dans le ciblage des chimères CD8-CD™. Il semble que l'absence de ce résidu cystéine favorise la stabilité et le ciblage membranaire des chimères étudiées ainsi que leur présence dans les exosomes. Ces phénomènes pourraient être indépendants de l'hyperpalmitoylation associée à l'absence de Cys 3. En effet, la construction pX2 AAC, qui est non palmitoylée, est plus stable que les trois mutants pX2 possédant Cys 3 ainsi que Cys 1 et/ou Cys 2, qui sont palmitoylables. Cependant, une implication différentielle de la palmitoylation dans la stabilité et le ciblage de nos chimères ne doit pas être exclue.

Cette étude a permis de découvrir l'importance fondamentale de tmD du BLV. En effet, la délétion de tout ou partie de tmD BLV semble accroître sensiblement la stabilité des chimères ainsi que leur ciblage au niveau de la membrane plasmique. Ainsi de façon étonnante, la présence de tmD BLV favoriserait la dégradation précoce des chimères. Cette dégradation pourrait intervenir au niveau du système du contrôle du repliement au sein des compartiments cellulaires que sont le RE et le TGN puisque la voie de dégradation lysosomale ne semble pas en cause. L'absence d'effets d'inhibiteurs de transport vésiculaire sur la stabilité et le ciblage exosomal des chimères CD8-CD™ ainsi que leur colocalisation très partielle avec les endosomes tardifs, même pour les chimères les moins stables, vient soutenir cette hypothèse.

L'attrait principal de nos travaux réside dans la découverte d'outils potentiellement efficaces pour le développement d'un mode de vaccination basé sur le concept « exosome display ». En effet, les chimères pX4 --C et surtout pX4 --A semblent disposer d'un « moteur » moléculaire permettant un ciblage très efficace d'antigène peptidique (ici le CD8) vers les exosomes. Ce « moteur » serait donc situé dans le domaine cytoplasmique de la protéine TM du BLV.

### Exemple 2 : Identification d'acides aminés impliqués dans le ciblage exosomal du peptide CD™

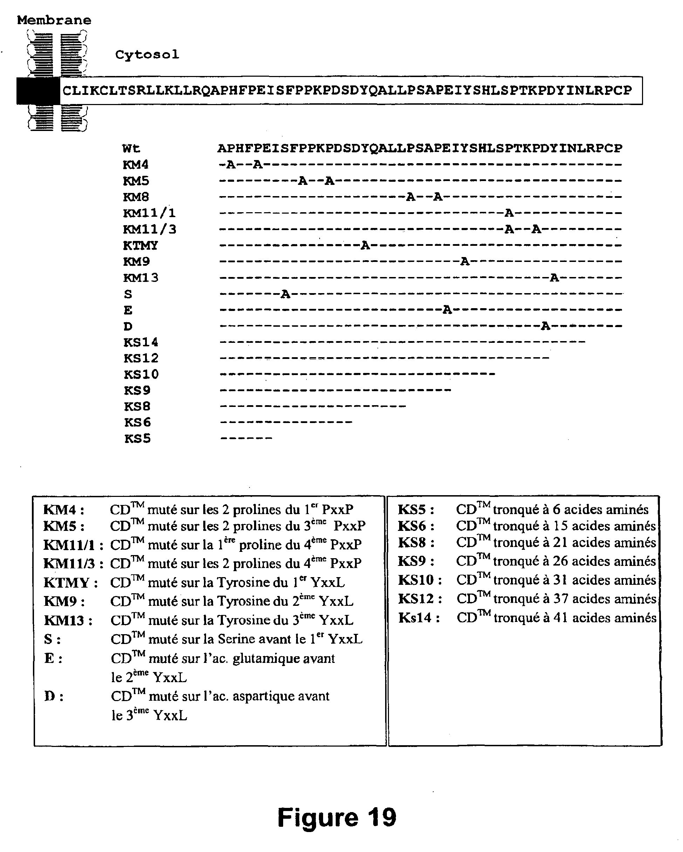

Afin de préciser la nature exacte du « moteur » situé dans le domaine cytoplasmique de la protéine TM du BLV avant de procéder à de premiers essais d'immunisation, nous avons étudié l'effet de 18 types de mutations dans le domaine cytoplasmique de la protéine TM du BLV (voir figure 19):
- délétion des 13 résidus N-terminaux et substitution des 2 résidus proline du premier motif PxxP (SEQ ID NO.:13 et SEQ ID NO. :14 ; mutation KM4) ;
- délétion des 13 résidus N-terminaux et substitution des 2 résidus proline du deuxième motif PxxP (SEQ ID NO.:15 et SEQ ID NO.:16; mutation KM5) ;
- délétion des 13 résidus N-terminaux et substitution des 2 résidus proline du troisième motif PxxP (SEQ ID NO.:17 et SEQ ID NO. :18; mutation KM8) ;
- délétion des 13 résidus N-terminaux et substitution du premier résidu proline du quatrième motif PxxP (SEQ ID NO.:19 et SEQ ID NO.:20; mutation KM11/1);
- substitution des 2 résidus proline du quatrième motif PxxP (SEQ ID NO.:21 et SEQ ID NO.:22; mutation KM11/3) ;
- délétion des 13 résidus N-terminaux et substitution du résidu tyrosine du premier motif YxxL (SEQ ID NO.:23 et SEQ ID NO. :24; mutation KTMY) ;
- délétion des 13 résidus N-terminaux et substitution du résidu tyrosine du deuxième motif YxxL (SEQ ID NO.:25 et SEQ ID NO. :26; mutation KM9) ;
- délétion des 13 résidus N-terminaux et substitution du résidu tyrosine du troisième motif YxxL (SEQ ID NO.:27 et SEQ ID NO.:28; mutation KM13) ;
- délétion des 13 résidus N-terminaux et substitution du résidu sérine se trouvant avant le premier motif YxxL (SEQ ID NO.:29 et SEQ ID NO. :30; mutation S);
- délétion des 13 résidus N-terminaux et substitution du résidu acide glutamique se trouvant avant le deuxième motif YxxL (SEQ ID NO.:31 et SEQ ID NO. :32 ; mutation E) ;
- délétion des 13 résidus N-terminaux et substitution du résidu acide aspartique se trouvant avant le troisième motif YxxL (SEQ ID NO.:33 et ; SEQ ID NO. :34; mutation D) ;
- séquence tronquée à 6 résidus - délétion des 13 résidus N-terminaux et des 39 résidus C-terminaux (SEQ ID NO. : 35 et SEQ ID NO. :36 ; mutation KS5) ;
- séquence tronquée à 15 résidus - délétion des 13 résidus N-terminaux et des 30 résidus C-terminaux (SEQ ID NO. :37 et SEQ ID NO. :38; mutation KS6) ;
- séquence tronquée à 21 résidus - délétion des 13 résidus N-terminaux et des 24 résidus C-terminaux (SEQ ID NO. : 39 et SEQ ID NO.:40; mutation KS8) ;
- séquence tronquée à 26 résidus - délétion des 13 résidus N-terminaux et des 19 résidus C-terminaux (SEQ ID NO.:41 et SEQ ID NO. :42; mutation KS9) ;
- séquence tronquée à 31 résidus - délétion des 13 résidus N-terminaux et des 14 résidus C-terminaux (SEQ ID NO.:43 et SEQ ID NO. :44; mutation KS10) ;
- séquence tronquée à 37 résidus - délétion des 13 résidus N-terminaux et des 8 résidus C-terminaux (SEQ ID NO. :45 et SEQ ID NO. :46; mutation KS12) ;
- séquence tronquée à 41 résidus - délétion des 13 résidus N-terminaux et des 4 résidus C-terminaux (SEQ ID NO. :47 et SEQ ID NO.48; mutation KS14).

Les mutants de substitution et de délétion ont été obtenus par mutagénèse dirigée ; les résidus substitués ont été remplacés par un résidu alanine. L'arrêt de traduction des mutants de délétion a été obtenu par addition d'un codon stop (codon TGA, TAG ou TAA).

Les séquences ADN codant pour les 18 mutants, ainsi que la séquence ADN CD™ sauvage dans laquelle seuls les 13 résidus N-terminaux ont été délétés (SEQ D NO. : 7 ; séquence appelée « séquence sauvage » ci-après) ont été sous-clonées en aval d'une séquence codant pour l'ectodomaine du CD8α murin. Les 19 gènes chimères obtenus ont ensuite été clonés dans un vecteur d'expression viral. Les vecteurs recombinants ainsi obtenus ont été transfectés dans des cellules eucaryotes (cellules HEK) afin d'analyser le ciblage vers les exosomes des protéines chimères résultantes. 48 heures après, l'expression protéique dans les cellules a été examinée par Western Blot. Parallèlement, les exosomes ont été purifiés par ultracentrifugation permettant d'évaluer ainsi le tri de la protéine chimère dans les exosomes par FACS et western blot.

Les résultats présentés ci-après mettent en évidence la nécessité de deux motifs peptidiques individuellement reconnus dans la littérature pour leurs interactions avec des protéines associées à la machinerie ESCRT et à la machinerie de transfert intermembranaire (en particulier les adaptines dont AP3). C'est la première fois que l'on apporte des évidences expérimentales que ces deux motifs peptidiques jouent, en synergie, un rôle déterminant dans le ciblage exosomal.

Ces résultats apportent des informations inédites et intéressantes en termes de signalisation inter-cellulaires utilisant les exosomes. Ils permettent ainsi d'avoir en main un outil bien défini pour cibler des protéines avec les exosomes. D'un point de vue industriel, ils faciliteront l'élaboration d'une nouvelle génération de vaccin et d'un outil unique de criblage de molécules thérapeutiques ou d'anticorps par exemple.

### 1- Obtention des constructions moléculaires

### A- Préparation des inserts par PCR:

Les trois mutants de substitutions S (Ser→Ala), D (Ac. Asp→Ala) et E (Ac.Glut→Ala) ont été obtenus par mutagenèse dirigée par une double PCR utilisant les amorces de mutations suivantes :
- Amorce S vers A, sens:
   5' CCCTAAACCCGATGCTGATTATCAGGCGTTGCTACCATCC 3'
- Amorce S vers A, anti-sens:
   5' CGCGGATGGTAGCAACGCCTGATAATCAGCATCGGGTTTA 3'
- Amorce D vers A, sens:
   5' CCACCAAGCCGGCATACATCAACCT 3'
- Amorce D vers A, anti-sens:
   5' TCGAAGGTTGATGTATGCCGGCTTGGT 3'
- Amorce E vers A, sens:
   5' GCTACCATCCGCGCCAGCGATCTAC 3'
- Amorce E vers A, anti-sens:
   5' GTAGATCGCTGGCGCGGATGGTA 3'.

Les PCR ont été réalisées à l'aide du kit Expand High Fidelity PCR system (Roche®) qui possède un mélange enzymatique contenant de l'ADN polymérase *Taq* thermostable et d'une ADN polymérase *Tgo* thermostable pourvue d'une activité correctrice (activité exonucléasique 3'-5') qui permet de limiter les erreurs lors de la polymérisation et d'obtenir des fragments à bouts francs. Deux mélanges de réactifs ont été préparés à 4°C :

A (25µL):10ng d'ADN à amplifier, 1µL de dNTP 10mM (200µM final chacun), 1,5µL de chacune des deux amorces sens et anti-sens à 10µM (300nM final), eau stérile (qsp 25µL) ; et

B (25µL) :5µL de tampon « Expand High Fidelity » 10X à 15mM en MgCl₂ (1,5mM final), 0,75µL de mélange enzymatique « High Fidelity » (2,6U final), eau stérile (qsp 25µL).

A et B ont été mélangés à 4°C puis les cycles d'amplication suivants ont été réalisés :
- dénaturation de l'ADN double brin 2 minutes à 94°C ;
- 4 cycles de dénaturation (94°C, 10 secondes), hybridation (50°C, 15 secondes) et élongation (72°C, 20 secondes) ;
- 25 cycles : 10 secondes à 94°C, 15 secondes à 64°C puis 20 secondes à 72°C;
- élongation finale de 7 minutes à 72°C.

Le produit PCR obtenu a été maintenu à 4°C.

Les séquences ADN codant pour les 18 mutants, ainsi que la séquence ADN sauvage ont été modifiées par PCR (mutagénèse dirigée) afin qu'elles soient encadrées par des sites de restriction particuliers ; le protocole indiqué ci-dessus a été mis en oeuvre, en utilisant deux amorces possédant les sites de restrictions Xbal et Notl.

### B- Clonage des produits PCRs dans TOPO-bluntll et contrôles

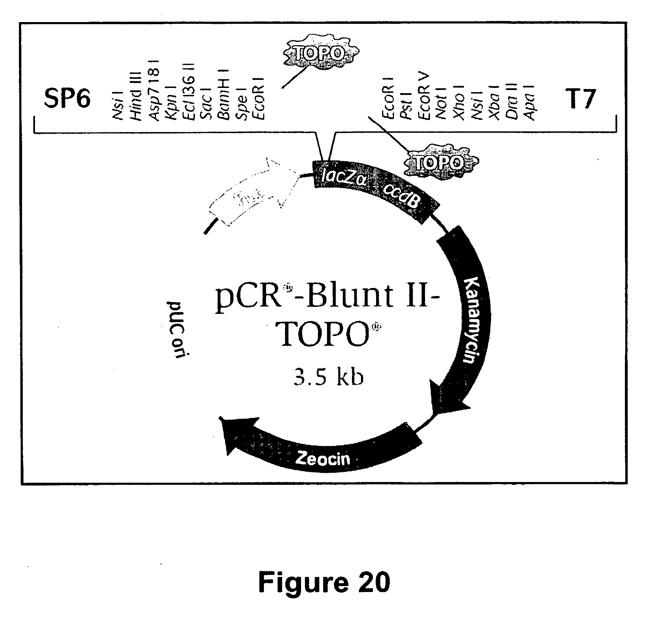

Chacun des 19 ADNs a été ligué dans un vecteur de clonage TOPO (voir figure 20) du kit Topo-blunt cloning (Invitrogen) pour être introduit dans des bactéries chimiocompétentes. Les clones transformés ont été sélectionnés et toutes les séquences d'ADNs vérifiées par séquençage.

### a) Ligation dans le plasmide et Transformation dans Top10

Chacun des différents produits de PCR a été intégré dans un plasmide TOPO-Bluntll déjà ouvert et à bouts francs portant le gène de résistance à la kanamycine. Les bactéries chimiocompétentes Top10 ont été transformées par ces plasmides et mises en culture sur boîte de LB/agar (100µg/mL de kanamycine). Le plasmide TOPO-Bluntll sans insert a servi de témoin négatif et un autre témoin (1ng du plasmide PUC19) a été utilisé comme témoin positif de transformation. Après culture, seules les bactéries transformées par un plasmide contenant l'insert ou PUC19 (témoin positif) se sont développées en présence de l'agent de sélection (antibiotique).

### b) Criblage des bons clones et séquençage:

Suivant les résultats des clonages, 2 à 10 colonies ont été amplifiées pour réaliser l'extraction de l'ADN plasmidique. Pour chaque construction et chaque clone, nous avons obtenu un volume d'ADN plasmidique de 100µL à environ 150ng/µL. Le rapport absorption à 260nm / absorption à 280nm donne pour chaque purification une valeur comprise entre 1,8 et 2 ce qui témoigne de la pureté de la préparation (une valeur inférieure à 1,8 témoignerait d'une contamination protéique).

Pour s'assurer de la présence de l'insert dans le plasmide, les ADNs plasmidiques ont été digérés par l'enzyme de restriction EcoRI qui encadre les séquences d'intérêt. La visualisation des ces produits de digestion a été faite sur gel d'agarose 2% où la présence d'un fragment (d'environ 300pb) constitue la preuve d'un ADN recombinant.

Une fois les bons clones identifiés, 2 à 4 µg d'ADN plasmidique de chaque mutant ont été séquencés afin de vérifier l'intégrité de chaque séquence d'intérêt et donc du cadre ouvert de lecture. Les mutations et les sites de restrictions rajoutés ont été contrôlés par la même occasion.

### C- Obtention des gènes chimères dans un vecteur de clonage pKSII

Chacune des 19 séquences (1 sauvage et 18 mutées) a été placée en aval d'une séquence codant pour l'ectodomaine CD8α de souris, pour donner 19 constructions.

### a) Préparation d'un vecteur de clonage pKSII-CD8

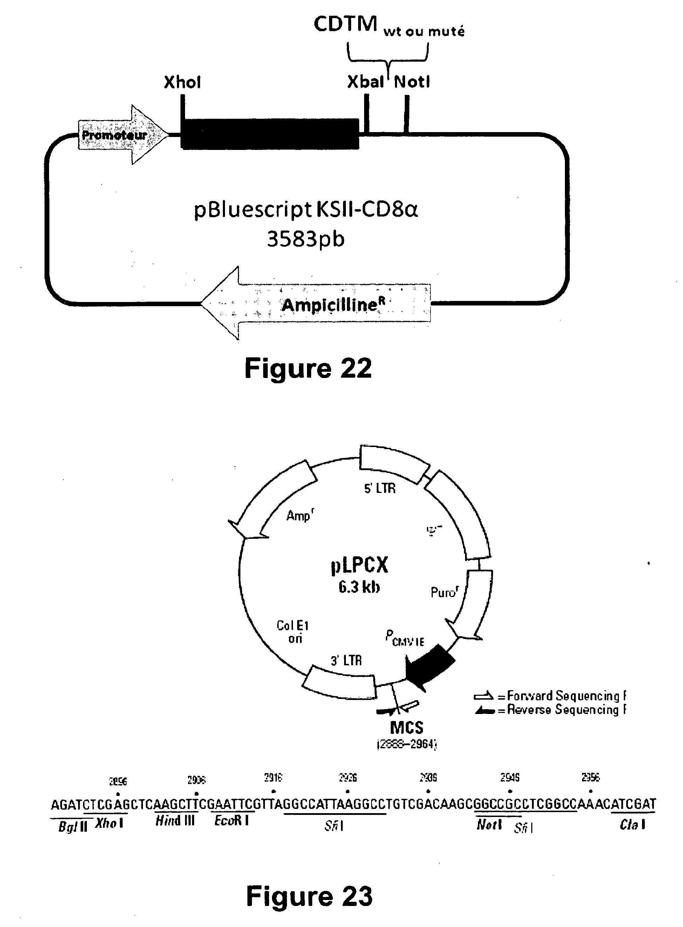

Le vecteur pKSII-CD8α (voir figure 22) a été digéré successivement avec les enzymes de restrictions XbaI et Notl pour pouvoir accueillir les inserts. Les digestions effectuées, le plasmide a été déphosphorylé, précipité à l'éthanol et purifié sur gel d'agarose 0,8%.

### b) Préparation des inserts

Les différents inserts encadrés par les sites de restrictions Xbal-Notl ont été digérés par les mêmes enzymes de restrictions que le plasmide afin que celui-ci puisse les intégrer.

### c) Obtention des plasmides chimères par clonage

Les ADNs ont été insérés dans le plasmide pKSII-CD8α linéarisé :

Les inserts digérés ont été purifiés sur gel d'agarose 2,5%. Leur réinsertion dans le vecteur pKSII-CD8α a été réalisée par la technique de ligation dans le gel. Un fragment de gel vierge a servi de témoin négatif de ligation.

Ayant utilisé les mêmes enzymes de restrictions pour le vecteur et les inserts, ils possèdent donc des sites cohésifs Xbal et Notl complémentaires : plasmide et inserts devraient pouvoir établir des liaisons entre eux. C'est une enzyme, la ligase, qui catalyse la formation d'une liaison phosphodiester entre une extrémité 3'-OH et une extrémité 5'-Phosphate de deux acides nucléiques.

Une fois l'étape de ligation achevée, des bactéries DH5α sont transformées par ces plasmides portant le gène de résistance à l'ampicilline. Après culture des différentes bactéries transformées à 37°C sur LB/agar (50 µg/mL d'ampicilline) et comparaison avec les témoins négatifs, on constate le développement de colonies uniquement chez les cellules transformées par les produits de ligation en présence d'insert CD™. Cela suggère que les colonies obtenues ont bien été transformées par un vecteur contenant un insert entre les sites Xbal et Notl.

### d) Criblage :

Pour confirmer l'obtention des plasmides chimères, différents clones de chaque mutant ont été criblés en digérant l'ADN plasmidique par les deux enzymes Xhol/ Notl et après migration des produits de digestion sur gel d'agarose 0,8%. Cette double digestion excise l'ensemble du gène chimère créé, c'est-à-dire avec le CD8α en phase avec le CD™.

Pour chaque clone et chaque construction (pKSII-CD8α-CD™ muté ou sauvage), il a été observé une première bande à 2,9 kpb correspondant à la taille du plasmide pKSII linéarisé. Une deuxième bande a été repérée à environ 950pb ; elle correspond au gène codant pour les chimères CD8α- CD™ mutés ou non.

### D- Obtention des gènes chimères dans le vecteur d'expression rétroviral pLPCX :

Le vecteur d'expression pKSII, s'il est facile à manipuler de par sa taille et ses sites de restrictions, ne permet pas l'expression protéique dans les cellules eucaryotes. Nous avons donc choisi pLPCX (Clontech Laboratories Inc., voir figure 23), qui permet d'introduire un gène autant par transfection que par transduction au moyen d'un vecteur rétroviral.

Chaque gène chimère a été excisé du plasmide pKSII entre les sites Xhol-Notl pour être purifié par extraction sur gel d'agarose 2% en utilisant le kit Nucleospin Extract II® (Macherey-Nagel).

Le vecteur d'expression pLPCX a lui aussi été préalablement digéré par le couple d'enzymes Xhol-Notl, déphosphorylé puis précipité à l'isopropanol (cette étape permet d'éliminer le court fragment d'ADN (inférieur à 100pb) situé entre Xhol et Notl libéré lors de la digestion).

La ligation des gènes chimères avec pLPCX a été réalisée en utilisant la T4 DNA ligase (Biolabs) (rapport insert/vecteur d'environ 3/1 molécule à molécule). Des bactéries chimio-compétentes Stbl2 (MAX Efficiency® Stbl2™ Competent Cells, Invitrogen) ont été transformées par ces produits de ligation. Un témoin positif (1 ng du plasmide pUC19) et un témoin négatif (pLPCX « ligué » sans insert) ont été préparés en parallèle. Après culture bactérienne à 30°C sur milieu gélosé contenant 50µg/mL d'ampicilline, seules les bactéries transformées par le témoin positif ou par les produits de ligation avec inserts se sont développées.

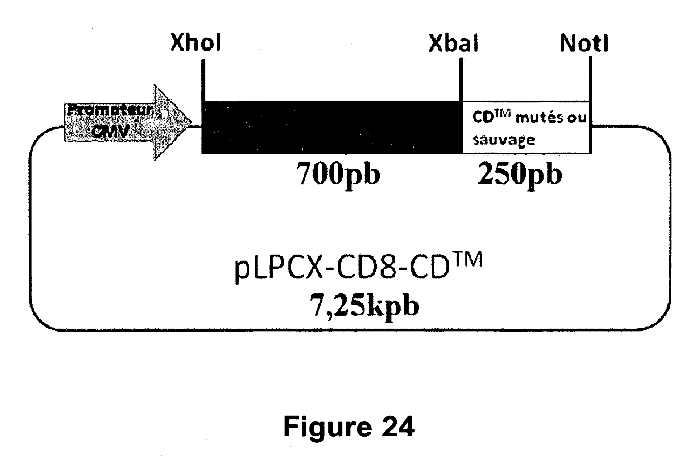

Pour pouvoir procéder au criblage et avoir une quantité conséquente d'ADN plasmidique nécessaire pour les transfections dans cellules eucaryotes, des Midipreprations ont été effectuées. La présence des inserts dans le plasmide a été vérifiée sur gel d'agarose 2% après digestion par Xhol puis Notl. Pour chaque construction (pLPCX-CD8α-CD™ mutées et sauvage ; voir figure 24) on observe une bande à 6,3kpb correspondant au pLPCX linéarisé et une bande à 950pb correspondant aux gènes chimères excisés.

### 2- Expression et analyse du ciblage vers les exosomes :

### A- Transfections dans les cellules HEH 293T :

Pour s'assurer de la transfection des cellules eucaryotes HEK 293T et estimer le pourcentage de cellules transduites, les cellules sont transfectées par le plasmide contenant le gène *LacZ* et incubées dans une solution de X-Gal.

D'abord à l'oeil et après observation au microscope optique (grossissement X40) nous avons constaté que plus de 50% des cellules étaient colorées en bleu ce qui prouve que la grande majorité ont été transduites par le plasmide LacZ. Les mêmes conditions ayant été respectées pour la transfection de nos gènes chimères, il est probable que plus de 50% des cellules aient été transduites par les gènes chimères.

En parallèle, vingt transfections simultanées dans des cellules HEK 293T ont été effectuées. Elles correspondent à chacun des plasmides ainsi qu'à un témoin négatif (pLPCX-CD8 sans CD™).

### B- Expression des protéines chimères dans la cellule et ciblage vers les exosomes

### a) Analyse par western blot :

Les lysats cellulaires et exosomaux issus des transfections ont été analysés par migration sur gel SDS-PAGE à 10%, suivie d'un transfert sur membrane PVDF (polyvinylidène difluoride, Immobilon-P, Millipore). Ces membranes ont ensuite été révélées à l'aide d'un sérum primaire de lapin anti-CD™ suivi d'un anticorps secondaire anti-IgG de lapin couplés à la peroxydase. Après révélation, ces anticorps sont éliminés et les membranes de transfert révélées de la même façon mais en utilisant un sérum de lapin anti-CD8α.

Les résultats sont présentés sur les figures 25 et 26.

### • Comparaison des niveaux d'expression des chimères dans les cellules et les exosomes :

On constate que les lysats cellulaires ne révèlent qu'une faible expression des protéines chimères. Par contre, la présence de certaines protéines chimères dans les exosomes est parfois très forte.

Outre les niveaux d'expression, la différence majeure que l'on remarque entre protéines cellulaires et protéines exosomales est la présence de 2 à 3 bandes pour les cellules et d'une seule pour les exosomes. Ceci vient du fait que la cellule contient des formes non-glycosylées et plus ou moins glycosylées. Seule la forme correctement glycosylée se retrouve dans les exosomes.

### • Analyse par western blot du ciblage exosomal des témoins positifs (CD8α-CD™) et négatif (CD8α seul) :

Avec le sérum anti-CD™, une bande migrant à 31 kDa est présente dans le lysat exosomal du témoin CD8α-CD™ sauvage alors qu'elle est absente dans le lysat des cellules transfectées par le témoin négatif. Cette bande est caractéristique de la protéine chimère attendue.

Avec le sérum anti-CD8α, le témoin négatif CD8α seul présente une bande vers 27kDa qui correspond à l'expression et au ciblage exosomal de CD8α dépourvu de CD™. Comme précédemment, une bande migrant à 31kDa est présente dans le lysat exsomal du témoin CD8α-CD™ sauvage. La différence d'intensité entre les bandes 31kDa et 27kDa indique que le CD8α est bien plus ciblé sur les exosomes quand il est fusionné au CD™.

### • Analyse par western blot des variations de ciblage des CD8α-CD™ mutés :

Seuls les résultats obtenus avec le sérum anti-CD8a sont comparables entre eux. Les résultats obtenus avec le sérum anti-CD™ sont uniquement utilisés pour confirmer les précédents. Suivant la mutation de la séquence codant pour le CD™, ces résultats montrent une variation dans l'expression et le ciblage des protéines chimères vers les exosomes. Ceci est particulièrement clair pour les mutations qui inhibent fortement le ciblage des protéines sur les exosomes. Les mutants concernés sont les mutants KM8, KM13, D et KS8.

De ces observations on peut conclure que le motif PSAP (mutant KM8) et le motif DY (au niveau du dernier motif YxxL (mutants KM13 et D)) sont importants pour le ciblage exosomal.

### b) Quantification des protéines chimères sur les exosomes par FACs :

La présence des protéines chimères a été aussi recherchée par une analyse en cytofluorométrie (FACscan) utilisant un anticorps monoclonal anti-CD8 fluorescent.
Après fixation des exosomes sur billes de latex (billes IDC (Interfacial Dynamics Corporation) ultraclean aldehyde/sulfate Latex), les protéines chimères présentes à la surface des exosomes ont été marquées à l'aide d'un anticorps monoclonal de souris anti-CD8α couplé à la fluorescéine (anticorps 53-6.7 de chez Pharmingen) et analysées au cytofluorimètre (FACScan).

Les résultats obtenus sont particulièrement clairs pour les mutants KM8, KM13 et D, qui révèlent l'importance des acides aminés mutés pour le ciblage des protéines chimères sur les exosomes (voir Figures 26 et 27). Ces résultats confirment l'impact des motifs PSAP, D et Y (du motif DYxxL)dans le ciblage des protéines vers les exosomes déjà révélés par les résultats des westerns blots.

### Conclusion :

Au cours de cette étude, nous avons construit des gènes chimères permettant d'exprimer le peptide pilote CD™ muté ou sauvage fusionné avec le CD8α de souris. Ces mutations sur des acides aminés ou des motifs remarquables du CD™ avaient pour but d'identifier les aminoacides et motifs consensus importants dans le ciblage exosomal.

Les différents gènes chimères ont été intégrés dans un vecteur d'expression rétroviral pour transfecter des cellules eucaryotes HEK 293T afin d'obtenir l'expression de ces protéines chimères. Les bandes observées en western blot suggèrent que, comme la protéine CD8α native, ces protéines sont différemment glycosylées lors de leur passage dans l'appareil de Golgi. Seules les protéines correctement glycosylées se retrouveraient dans les exosomes. Ces protéines ont subi les modifications post-traductionnelles adéquates, condition indispensable à l'expression d'épitopes conformationnels essentiels à la future élaboration d'une immunité vaccinale ou au criblage de molécules thérapeutiques. Cependant, ces glycosylations, qui sont plus ou moins présentes, conduisent à de multiples bandes diffuses qui nous ont gênés lors de la quantification comparée des protéines. Pour palier ce problème, il faudra traiter les lysats avec une endoglycosylase afin d'observer une seule bande sur gel.

D'après ces résultats, les motifs PSAP et DY (du dernier YxxL) sont indispensables à l'expression et au ciblage des protéines chimères vers les exosomes. Ces résultats sont inédits et sont intéressants aussi bien du point de vue fondamental que du point de vue application industrielle.

Il est probable que le motif PSAP soit responsable d'une interaction avec la protéine Tsg101 du complexe ESCRT. Quant au motif DYxxL, il pourrait être impliqué dans l'interaction avec la protéine ALIX du complexe ESCRT. Ainsi, pour la première fois, des données expérimentales suggèrent que le complexe ESCRT serait impliqué dans la formation des exosomes.

### EXEMPLE 3 : ciblage de récepteurs à domaines transmembranaires vers les exosomes.

Les récepteurs membranaires sont des cibles majeures pour le développement de molécules thérapeutiques. En général, le criblage à haut débit de drogues s'effectue en particulier avec des récepteurs à multiples domaines membranaires exprimés sur des cellules en culture. Outre les difficultés pour obtenir une forte expression de récepteurs à la surface des cellules, cette technique entraîne des difficultés de robotisation. C'est pourtant actuellement la seule solution, puisque l'utilisation de récepteurs recombinants purifiés est pour l'instant techniquement inenvisageable.

Dans ce contexte, des exosomes porteurs de récepteurs en particulier de récepteurs à multiples domaines seraient un matériel simple d'emploi et bien adapté au criblage en raison de leur stabilité et de leur facilité de manipulation.

La présente étude visait à produire des exosomes porteurs de récepteurs à simple ou multi- domaines transmembranaires, en particulier le récepteur CxCR4 (récepteur de la chimiokine SDS-1 (CXCL-12) et du HIV) et le récepteur CD4 (récepteur du HIV).

Trois gènes chimères ont été synthétisés. Ils comportent, à l'extrémité 3', le peptide CD™-BLV de séquence SEQ ID. NO. : 8, et à l'extrémité 5', un ADN codant pour le récepteur humain CxCR4, pour une version du récepteur CxCR4 tronquée en partie C-terminale comportant 307 acides aminés (CxCR4 (307)) ou pour une version du récepteur CD4 tronquée en partie C-terminale comportant 403 acides aminés (CD4 (403)).

Les récepteurs CD4 et CxCR4 comportent respectivement un et sept domaines transmembranaires.

Les trois gènes chimères ont été clonés dans un vecteur d'expression rétroviral pLPCX. Ces différents plasmides ont été transfectés dans des cellules eucaryotes humaines HEK293T afin d'observer l'expression des différentes protéines chimères dans ces cellules ainsi que leur tri vers les exosomes.

La stratégie de clonage et sous-clonage utilisée est similaire à celle décrite pour l'exemple 2 :

Les ADNs codant pour les récepteurs CxCR4, CxCR4 (307) et CD4 (403) ainsi que celui codant pour le peptide pilote CD™ sont amplifiés par PCR en utilisant des amorces comportant les séquences de sites de restrictions que l'on souhaite intégrer à chaque extrémité des fragments amplifiés (les fragments CxCR4, CxCR4 (307) et CD4 (403) seront flanqués en 5' par le site EcoRI et en 3' par le site Xbal et CD™/BLV sera flanqué en 5' par le site Xbal et en 3' par le site Notl).

Les inserts ainsi produits sont clonés dans des vecteurs d'amplification Topo (voir figure 20). Les plasmides obtenus sont ensuite digérés par des enzymes de restriction et analysés sur gel d'agarose 1,5%, puis séquencés afin de vérifier l'intégrité de leur séquence.

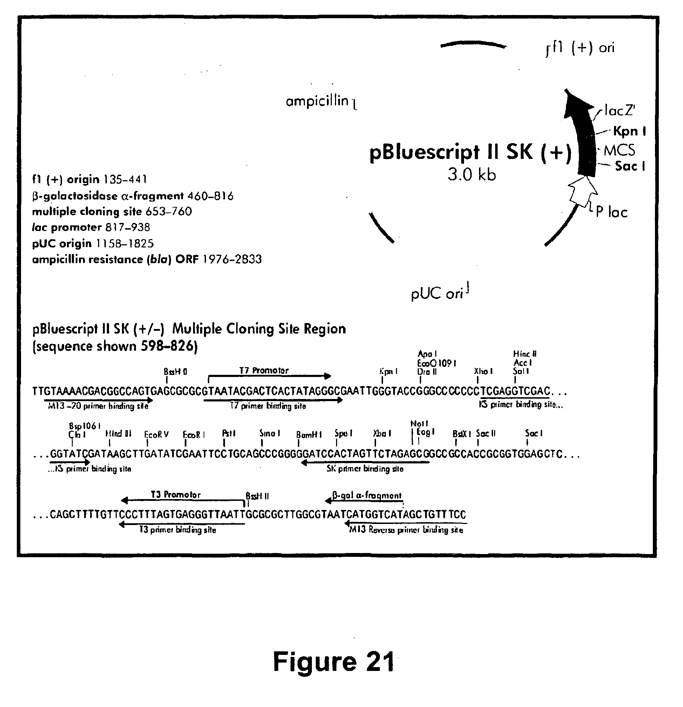

L'insert CD™/BLV est ensuite excisé du vecteur Topo par digestion enzymatique en utilisant le couple Xbal/Notl puis sous-cloné dans le vecteur d'amplification pKS2 (voir figure 21). Le vecteur pKS2 recombinant ainsi que les vecteurs Topo recombinants contenant les inserts CxCR4, CxCR4 (307) et CD4 (403) sont alors digérés avec les enzymes de restriction EcoRI et Xbal, afin de pouvoir sous-cloner les fragments CxCR4, CxCR4 (307) et CD4 (403) dans le vecteur d'amplification pKS2, lesdits fragments étant placés en 5' de la séquence codant pour le peptide CD™.

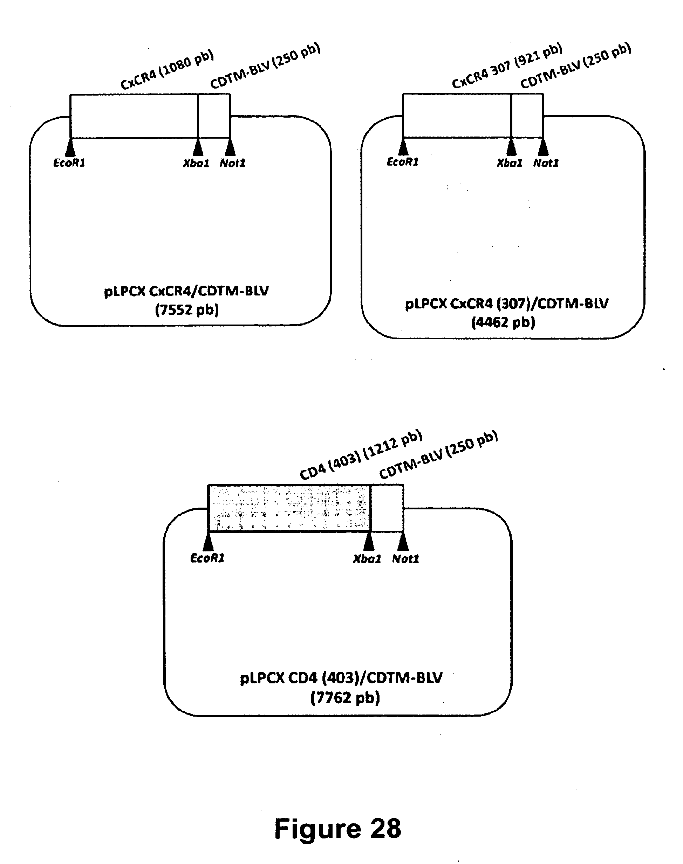

Les différentes constructions ainsi obtenues sont excisées des plasmides pKS2 recombinants par digestion avec le couple d'enzyme EcoRI/Notl puis sous-clonées dans le vecteur d'expression rétroviral pLPCX (voir figure 22). Les vecteurs obtenus (voir figure 28) sont vérifiés par digestion enzymatique en utilisant le couple d'enzymes EcoR1/Xba1.

Les différents plasmides pLPCX sont transfectés dans des cellules eucaryotes humaines HEK293T afin d'exprimer les protéines chimères CxCR4/CD™, CxCR4(307)/CD™ et CD4 (403)/CD™.

Un extrait des protéines cellulaires totales (100 µg) et les protéines d'une suspension d'exosomes produits par chacun des lots de cellules transfectées sont alors soumis à une migration en SDS-PAGE (10%) en présence ou en absence de β-mercaptoéthanol. Les protéines d'intérêt sont révélées par Western Blot grâce à l'utilisation d'un sérum primaire de lapin anti-CD™ et d'anticorps secondaires anti-IgG de lapin couplés à la péroxydase. La révélation des anticorps est réalisée en chambre noire et par utilisation d'une solution ECL. Enfin l'empreinte protéique de chaque échantillon est révélée par coloration au bleu de Coomassie.

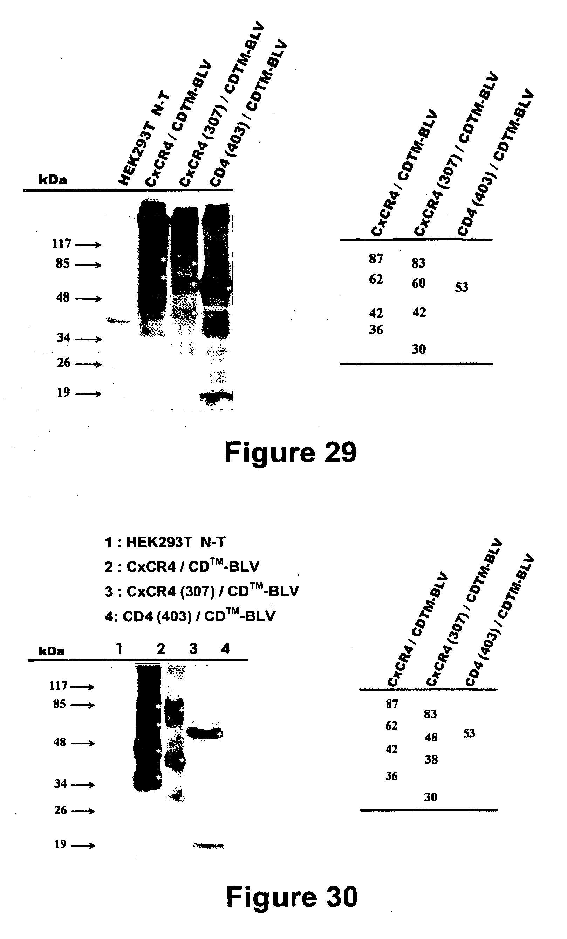

Les résultats sont présentés en figures 29-31.

On observe que les chimères CxCR4/CD™, CxCR4 (307)/CD™ et CD4 (403)/CD™ sont exprimées dans les extraits de protéines cellulaires (voir figure 29).

Plusieurs bandes caractérisent la chimère CxCR4/CD™ : 36, 42, 62 et 87 kDa. L'expression du récepteur CxCR4 sauvage se caractérise en effet par plusieurs isoformes notamment dans les cellules HEK293T ; des bandes 34, 40, 47, 62, 73 et 80 kDa peuvent être identifiées (Sloane JA, *et al*.). Les tailles supérieures des bandes de la chimère CxCR4/CD™ dans les HEK293T sont dues à la présence du peptide pilote (domaine CD™) dans la chimère CxCR4/CD™. De même, les bandes 30, 42, 60 et 83 kDa révèlent la présence de la chimère CxCR4 (307)/CD™. La variation de taille des bandes représentant les différentes isoformes de cette chimère s'explique par le fait que le récepteur CxCR4 est tronqué. La chimère CD4 (403)/CD™, elle, est caractérisée par une bande nettement visible de 53 kDa.

En outre ces chimères sont toutes triées vers les exosomes comme le montre la présence les bandes 36, 42, 62 et 87 kDa (pour CxCR4/CD™) ; 30, 38, 48, et 83 kDa (pour CxCR4 (307)/CD™) et la bande 53 kDa (pour CD4 (403)/CD™) (voir figure 30).

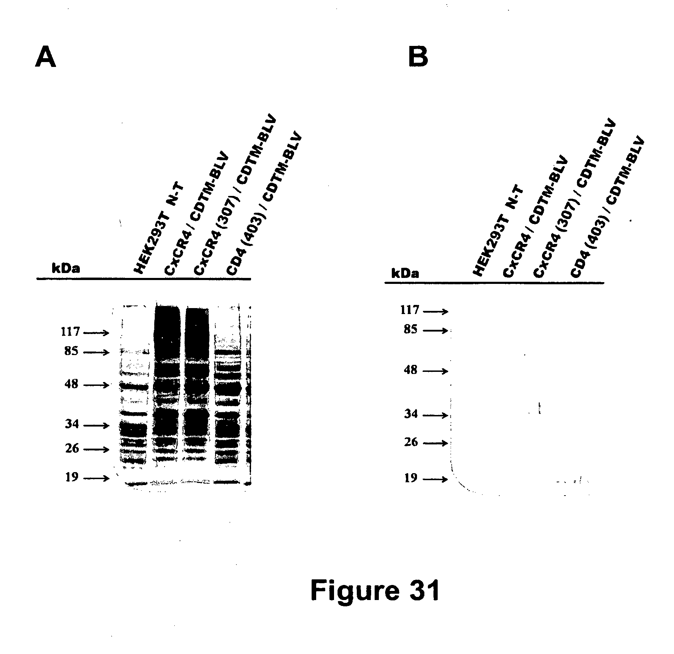

La révélation au bleu de Coomassie des différentes empreintes protéiques montre que les quantités de protéines totales d'origine exosomale (figure 31 B) utilisées au cours de ces expériences sont nettement inférieures aux quantités de protéines totales d'origine cellulaire (figure 31A) alors que le signal en Western blot est équivalent. On constate que toutes les protéines présentes dans le lysat cellulaire témoin ne sont pas triées vers les exosomes. Les chimères CxCR4/CD™ et CD4(403)/CD™ sont adressées très fortement vers les exosomes. En revanche, la chimère CD4 (403)/CD™ est triée presque totalement vers les exosomes.

### Conclusion

La transfection de cellules HEK293T avec différents plasmides pLPCX a permis l'expression des chimères CxCR4/CD™-BLV, CxCR4(403) /CD™-BLV et CD4 (403)/CD™-BLV. L'analyse par Western blot a mis en évidence leur présence dans les lysats cellulaires.

Comme attendu, plusieurs isoformes des chimères CxCR4/CD™-BLV et CxCR4 (307)/CD™-BLV sont exprimées dans les cellules HEK293T transfectées avec les plasmides pLPCX CxCR4/CD™-BLV et pLPCX CxCR4 (307)/CD™-BLV. Les chimères CxCR4/CD™-BLV et CxCR4 (307)/CD™-BLV sont efficacement triées vers les exosomes et il semble que ces protéines de fusion soient partagées à parts égales dans les cellules et les exosomes.

En outre on observe la présence de la chimère CD4 (403)/CD™-BLV dans les cellules HEK293T transfectées avec le plasmide pLPCX CD4 (403)/CD™-BLV. Il semble que le peptide pilote CD™-BLV favorise un tri très important de la chimère CD4 (403)/CD™-BLV vers les exosomes.

Les résultats décrits ci-dessus montrent que le peptide pilote CD™-BLV est capable de trier indifféremment des protéines à simple ou multiples domaines transmembranaires vers les exosomes.

On sait qu'il est très difficile de travailler sur ces récepteurs en solution car s'ils ne sont pas intégrés dans une membrane plasmique, ils ne gardent pas leur structure native. Actuellement les études réalisées sur ces protéines sont souvent faites à partir de lignées cellulaires stables et exprimant les récepteurs d'intérêt. Il est toutefois contraignant en termes de temps et d'économie d'acquérir, de cultiver et d'entretenir ces lignées qui peuvent mourir à tout moment si elles sont mal traitées. C'est pourquoi le fait d'utiliser des exosomes porteurs de récepteurs à multiples domaines transmembranaires pour leurs études représente une solution intéressante car les exosomes possèdent tous les avantages d'une cellule pour ces études sans les inconvénients puisqu'ils ne sont pas vivants.

Des exosomes dans la membrane desquels sont intégrées des protéines recombinantes et en particulier des protéines comportant de multiples domaines transmembranaires pourraient être utilisés en vaccinologie et comme outil de criblage.

### BIBLIOGRAPHIE

Alberts B, Bray D, Lewis J, Raff M, Roberts K, Watson JD (1995). "Molecular Biology of the Cell (3rd Ed.)" Garland, NY.
Cann AJ, Churcher MJ, Boyd M, O'Brien W, Zhao JQ, Zack J, Chen IS (1992). "The région of the envelope gene of human immunodeficiency virus type 1 responsible for détermination of cell tropism." J Virol. 1992 ; 66(1):305-9.
Chaput N, Taïeb J, Schartz N, André F, Angevin E, Zitvogel L. (2004). "Exosomes-based immunotherapy." Cancer Immunol Immunother; 53:234-239.
Colino, J. et Snapper C.M. (2006). Journal of Immunology, 177 :37576
De Gassart A, Geminard C, Hoekstra D, Vidal M. (2004). "Exosome secretion : the art of reutilizing nonrecycled proteins." Traffic. ; 5:896-903**.**
Delamarre L, Rosenberg AR, Pique C, Pham D, Callebaut I, Dokhelar MC. (1996). "The HTLV-I envelope glycoproteins: structure and functions." J Acquir Immune Defic Syndr Hum Retrovirol; 13 Suppl 1:S85-91.
Delcayre, A., et al. 2005, Blood Cells Mol Dis 35 :158 **;** Thery C. et al., (2002); Nature Immunology 3 :1156.
Delcayre A, Le Pecq JB. (2006) "Exosomes as a novel therapeutic nanodevices." Curr Op in Mol Ther.; 8:1464-1471.
Gould SJ, Booth A, Hildret JE. (2003) "The Trojan exosome hypothesis." Proc Natl Acad Sci USA.; 100:10592-10597.
Herbein G, Coaquette A, Perez-Bercoff D, Pancino G. (2002) "Macrophage activation and HIV infection: can the trojan horse turn into a fortress?" Curr Mol Med.; 2:723-738.
Levine AJ. (1992). "Viruses" (Scientific American Library, No. 37). W. H. Freeman/Scientific American Library
Pornillos O, Garrus J, Sundquist WI. (2002). "Mechanism of enveloped RNA virus budding." Trends Cell Biol. 2; 12:569-579.
Raposo G, Moore M, Innes D, Leijenderkker R, Leigh-Brown A, Benaroch P, Geuze H. (2002). "Human macrophage accumulate HIV-1 particles in MHC II compartments." Traffic. ; 3:718-729.
Sloane JA, and al. (2005). Marked structural and functional heterogeneity in CxCR4: Separation of HIV-1 and SDF-1alpha responses. Immunology and Cell Biology; 83: 129-143.
Straub OC, Levy D. (1999). "Bovine immunodeficiency virus and analogies with human immunodeficiency virus." Leukemia.;13 Suppl 1:S106-9.
Zitvogel L, Regnault A, Lozier A, Wolfers J, Tenza D, Raposo G, Amigorena S. (1998). "Dendritic cell-derived exosomes elicit potent antitumour immune responses in vivo." Nat. Med. ; 4:594-600.

## Revendications

1. **Polypeptide** permettant la sécrétion d'un peptide ou polypeptide d'intérêt auquel il est associé, en association avec des vésicules membranaires, en particulier avec des exosomes, lorsqu'il est exprimé dans une cellule eucaryote, **caractérisé en ce qu'**il comprend ou consiste en un dérivé muté du domaine cytoplasmique (CD) de la protéine transmembranaire (TM) du virus de la leucémie bovine (BLV), de séquence SEQ ID NO : 6, où ledit dérivé muté
a) est défini par la substitution, la délétion et/ou l'insertion d'un ou plusieurs résidu(s) dans la séquence du domaine cytoplasmique (CD) de la protéine transmembranaire (TM) du virus de la leucémie bovine (BLV) de séquence SEQ ID NO : 6,
b) comprend au moins un motif PxxP et au moins un motif YxxL, dans lesquels P représente un résidu proline, Y représente un résidu tyrosine, x représente un résidu quelconque, et L représente un résidu leucine ;
c) conserve la capacité du domaine CD de la protéine TM du BLV à adresser le peptide ou polypeptide d'intérêt auquel il est associé vers les vésicules membranaires ;
d) comprend une séquence qui présente au moins 60% d'identité avec la séquence de SEQ ID NO : 8 complète ; et
e) a subi une délétion, ou une substitution par un résidu alanine, du résidu cystéine de la séquence PCP contenue dans la séquence de SEQ ID NO : 6.

2. Polypeptide selon la revendication 1, dans lequel ledit dérivé muté de domaine CD comprend ou consiste en la séquence de SEQ ID NO : 8 délétée de la séquence PCP.

3. Polypeptide selon la revendication 1 ou 2, dans lequel ledit dérivé muté de domaine CD comprend ou consiste en la séquence SEQ ID NO : 10 ou SEQ ID NO : 12.

4. Polypeptide selon l'une quelconque des revendications 1 à 3, dans lequel la séquence dudit dérivé muté de domaine CD est constituée de 30 à 70 résidus.

5. Polypeptide selon l'une quelconque des revendications 1 à 4, dans lequel ledit dérivé muté de domaine CD est dépourvu de la séquence KCLTSRLLKLLRQ.

6. Polypeptide selon l'une quelconque des revendications 1 à 5, dans lequel ladite séquence dudit dérivé muté de domaine CD est identique à au moins 70%, au moins 80%, au moins 90% ou au moins 95% à la séquence de SEQ ID NO:8.

7. Polypeptide selon l'une quelconque des revendications 1 à 6, **caractérisé en ce qu'**en sus de ladite délétion du résidu cystéine de la séquence PCP contenue dans la séquence de SEQ ID NO : 6, ledit dérivé muté a subi une délétion de l'ensemble de ladite séquence PCP.

8. Polypeptide selon l'une quelconque des revendications 1 à 7, qui comprend en outre ledit peptide ou polypeptide d'intérêt et un domaine membranaire ayant la capacité de s'ancrer dans la bicouche lipidique d'une membrane cellulaire.

9. **Polynucléotide** codant pour un polypeptide selon l'une quelconque des revendications 1 à 8.

10. **Composition immunogène** qui comprend un polynucléotide selon la revendication 9, et un support, un diluant ou un véhicule pharmaceutiquement acceptable.

11. Composition immunogène selon la revendication 10, pour utilisation comme médicament.

12. Composition immunogène selon la revendication 10 ou 11, pour utilisation dans la prophylaxie et/ou le traitement d'une infection bactérienne, virale, parasitaire, ou d'une tumeur.

13. **Vésicule membranaire** comportant un polypeptide selon l'une quelconque des revendications 1 à 8.

14. **Vésicule membranaire** comportant un polypeptide chimérique qui comprend ou consiste en les domaines suivants :
(i) un peptide ou polypeptide d'intérêt ;
(ii) un domaine membranaire ayant la capacité de s'ancrer dans la bicouche lipidique d'une membrane cellulaire ; et
(iii) le domaine cytoplasmique (CD) de la protéine membranaire (TM) du virus de la leucémie bovine (BLV) ou un dérivé muté de ce domaine CD,
ledit dérivé muté ayant une capacité d'adressage dudit polypeptide chimérique vers des vésicules membranaires de cellules eucaryotes et/ou vers le(s) compartiment(s) cellulaire(s) impliqué(s) dans la formation de ces vésicules membranaires, et
la séquence dudit dérivé muté :
- étant définie par la substitution, la délétion et/ou l'insertion d'un ou plusieurs résidu(s) dans la séquence dudit domaine CD, et
- étant identique à au moins 60% à la séquence de SEQ ID NO : 8, sous réserve que la séquence dudit dérivé muté comprenne au moins un motif PxxP et au moins un motif YxxL, dans lequel P représente un résidu proline, Y représente un résidu tyrosine, x représente un résidu quelconque et L représente un résidu leucine,
et sous réserve que, lorsque ledit domaine (iii) comprend ou consiste en le domaine CD de la protéine TM du BLV de séquence SEQ ID NO : 6, ledit domaine (ii) ne comprend pas et ne consiste pas en le domaine membranaire de la protéine TM du BLV, qui a pour séquence SEQ ID NO : 4.

15. Vésicule membranaire selon la revendication 14, **caractérisée en ce que** ledit peptide ou polypeptide d'intérêt est exposé, en partie ou en totalité, à l'extérieur de ladite vésicule membranaire.

16. Vésicule membranaire selon la revendication 14, **caractérisée en ce qu'**en sus dudit polypeptide chimérique, elle comprend en outre un fragment dudit peptide ou polypeptide d'intérêt comprenant ou consistant en un ou plusieurs épitope(s) dudit peptide ou polypeptide d'intérêt, ledit fragment étant ancré dans la membrane de ladite vésicule membranaire par l'intermédiaire du domaine membranaire d'une molécule du complexe majeur d'histocompatibilité de type I ou de type II à laquelle il est associé.

17. Vésicule membranaire selon l'une quelconque des revendications 14 à 16, **caractérisée en ce que** lesdits domaines (i) à (iii) sont positionnés successivement dans l'ordre suivant, de l'extrémité N-terminale vers l'extrémité C-terminale dans le polypeptide : peptide ou polypeptide d'intérêt - domaine membranaire - domaine CD ou dérivé muté de domaine CD.

18. Vésicule membranaire selon l'une quelconque des revendications 14 à 17, **caractérisée en ce que** ledit polypeptide chimérique comprend en outre au moins une molécule de liaison liant deux domaines successifs.

19. Vésicule membranaire selon la revendication 18, **caractérisée en ce que** ladite molécule de liaison est un peptide ou un polypeptide.

20. Vésicule membranaire selon la revendication 18 ou 19, **caractérisée en ce que** la séquence nucléotidique codant pour ladite molécule de liaison comprend ou consiste en un site de restriction.

21. Vésicule membranaire selon l'une quelconque des revendications 14 à 20, **caractérisée en ce que** la séquence dudit dérivé muté de domaine CD est dépourvue de la séquence KCLTSRLLKLLRQ et/ou dépourvue de la séquence PCP.

22. Vésicule membranaire selon l'une quelconque des revendications 14 à 21, **caractérisée en ce que** la séquence dudit dérivé muté de domaine CD est identique à au moins 70%, ou au moins 80%, ou au moins 90% ou au moins 95% à la séquence de SEQ ID NO : 8.

23. Vésicule membranaire selon l'une quelconque des revendications 14 à 22, **caractérisée en ce que** la séquence dudit domaine CD comprend, ou la séquence dudit dérivé muté de domaine CD comprend ou consiste en une séquence choisie parmi les séquences suivantes:
PxxPxxxxPxxPxxxYxxLxPxxPExYxxLxPxxPDYxxL ;
PxxPxₙPxxPxₙYxxLxₙPxxPEXₙYxxLxₙPxxPDYxxL ;
PxxPxxxxPxxPxxxYxxLxPxxPExYxxLxPxxPDYxxLxxxx ; et
PxxPxₙPxxPxₙYxxLxₙPxxPEXₙYxxLxₙPxxPDYxxLxxxx,
dans lesquelles x et Xₙ représentent respectivement un résidu quelconque et un ou plusieurs résidu(s) quelconque(s).

24. Vésicule membranaire selon l'une quelconque des revendications 14 à 23, **caractérisée en ce que** ledit domaine CD est de SEQ ID NO : 6.

25. Vésicule membranaire selon l'une quelconque des revendications 14 à 24, **caractérisée en ce que** ledit dérivé muté de domaine CD comprend ou consiste en la séquence SEQ ID NO : 8, SEQ ID NO : 10 ou SEQ ID NO : 12.

26. Vésicule membranaire selon l'une quelconque des revendications 14 à 25, **caractérisée en ce que** ledit domaine membranaire comprend ou consiste en le domaine transmembranaire de la protéine TM du BLV et la séquence dudit dérivé muté de domaine CD est dépourvue de la séquence KCLTSRLLKLLRQ et/ou de la séquence PCP.

27. Vésicule membranaire selon l'une quelconque des revendications 14 à 26, **caractérisée en ce que** ledit domaine (iii) est :
- la séquence de SEQ ID NO : 8, ou
- une séquence qui :
- est identique à au moins 60% avec la séquence de SEQ ID NO : 8,
- est dépourvue de la séquence KCLTSRLLKLLRQ, et qui
- est constituée de :
i. la séquence qui, dans la séquence de SEQ ID NO : 8, s'étend du motif PSAP jusqu'au motif YxxL qui le suit en partie C-terminale, ou
ii. la séquence i. modifiée par substitution ou délétion d'un ou plusieurs acides aminé(s) entre lesdits deux motifs.

28. Vésicule membranaire selon l'une quelconque des revendications 14 à 27, **caractérisée en ce que** ledit peptide ou polypeptide d'intérêt provient d'un organisme pathogène, d'un virus, d'une bactérie, d'un parasite, d'un agent pathogène, d'un antigène tumoral, d'un antigène cytoplasmique, d'un récepteur de ligand, d'un récepteur à multiples domaines membranaires, d'un récepteur à sept domaines transmembranaires, d'un récepteur de cytokines, d'un ligand de récepteur, d'une cytokine ou d'un fragment de ceux-ci.

29. Vésicule membranaire selon l'une quelconque des revendications 14 à 28, **caractérisée en ce que** ledit peptide ou le polypeptide d'intérêt est choisi parmi l'ectodomaine de l'hémagglutinine (HA) d'un virus de la grippe, l'ectodomaine de la protéine HA du virus de la grippe aviaire H5N1, un fragment comprenant ou consistant en un ou plusieurs épitope(s) de cet ectodomaine de HA.

30. Vésicule membranaire selon l'une quelconque des revendications 14 à 29, **caractérisée en ce que** ledit polypeptide chimérique comporte en outre un peptide signal d'importation dans le réticulum endoplasmique.

31. Vésicule membranaire selon l'une quelconque des revendications 14 à 30, **caractérisée en que** ladite cellule eucaryote est une cellule HEK293, un lymphocyte T, un lymphocyte B, un mastocyte, une cellule dendritique, une cellule de Langerhans.

32. Vésicule membranaire selon l'une quelconque des revendications 14 à 31, **caractérisée en ce que** la séquence dudit dérivé muté de domaine CD est dépourvue de la séquence PC et/ou de la séquence CP.

33. Vésicule membranaire selon l'une quelconque des revendications 14 à 32, **caractérisée en ce que** la séquence dudit dérivé muté de domaine CD est constituée de 30 à 70 résidus.

34. Vésicule membranaire selon l'une quelconque des revendications 14 à 33, **caractérisée en ce qu'**elle est un exosome.

35. Une **composition immunogène** dont le principe actif comprend une ou plusieurs vésicule(s) membranaire(s) selon l'une quelconque des revendications 14 à 34, ou un polynucléotide codant pour un polypeptide chimérique tel que défini à l'une quelconque des revendications 14 à 34 et un support, un diluant ou un véhicule pharmaceutiquement acceptable.

36. Composition immunogène selon la revendication 35, pour utilisation comme médicament.

37. Composition immunogène selon la revendication 35 ou 36, pour utilisation pour la prophylaxie et/ou le traitement d'une infection bactérienne, virale, parasitaire, ou d'une tumeur.

38. **Cellule recombinante productrice d'exosomes, caractérisée en ce qu'**elle est recombinée avec un ou plusieurs polynucléotide(s) selon la revendication 9 ou avec un ou plusieurs polynucléotide(s) codant pour un polypeptide chimérique tel que défini à l'une quelconque des revendications 14 à 34, ou qu'elle a absorbé une vésicule membranaire selon l'une quelconque des revendications 13 à 34.

39. **Utilisation** *in vitro* d'un polypeptide selon l'une quelconque des revendications 1 à 8 ou d'un polypeptide chimérique tel que défini à l'une quelconque des revendications 14 à 34, ou d'un polynucléotide codant pour un tel polypeptide, pour adresser un peptide ou polypeptide d'intérêt vers des vésicules membranaires de cellules eucaryotes qui comportent des vésicules internes de sécrétion et qui sont cultivables, capables d'exocytose et modifiables génétiquement, et/ou vers le(s) compartiment(s) cellulaire(s) impliqué(s) dans la formation de ces vésicules membranaires, et pour permettre la sécrétion dudit peptide ou polypeptide d'intérêt en association avec lesdites vésicules membranaires par lesdites cellules eucaryotes.

40. **Utilisation d'une ou plusieurs vésicule(s) membranaire(s)** selon la revendication 15 ou 16, pour la détection *in vitro* de partenaires spécifiques capables d'interagir avec ledit peptide ou polypeptide d'intérêt ou avec un fragment dudit peptide ou polypeptide.

41. **Procédé de production *in vitro* de vésicules membranaires** comportant un peptide ou un polypeptide d'intérêt, ledit procédé comprenant les étapes suivantes :
a) l'introduction, d'un ou plusieurs polynucléotide(s) selon la revendication 9, qui code(nt) pour un polypeptide comprenant ledit peptide ou polypeptide d'intérêt, dans une cellule productrice d'exosomes, ou la mise en contact d'une cellule productrice d'exosomes avec une ou plusieurs vésicule(s) membranaire(s) selon l'une quelconque des revendications 13 à 34, qui comporte(nt) un polypeptide comprenant ledit peptide ou polypeptide d'intérêt,
b) la culture de ladite cellule productrice d'exosomes ;
c) la récupération de vésicules membranaires produites par ladite cellule productrice d'exosomes.

42. **Procédé de préparation d'un sérum polyclonal** dirigé contre un ou plusieurs peptide(s) ou polypeptide(s) antigénique(s) d'intérêt exprimé(s) à la surface de vésicules membranaires, comprenant les étapes suivantes :
a) l'administration, le cas échéant répétée, à un animal non humain, de vésicules membranaires selon l'une quelconque des revendications 14 à 34 ou comportant un polypeptide selon la revendication 8, d'une composition immunogène selon l'une quelconque des revendications 10 à 12 et 35 à 37, d'un polynucléotide selon la revendication 9 ou d'un polynucléotide codant pour un polypeptide chimérique tel que défini à l'une quelconque des revendications 14 à 34, associés ou non à un adjuvant; et
b) la récupération des anticorps formés, capables de reconnaître le ou les peptide(s) ou polypeptide(s) antigénique(s) d'intérêt.

43. **Procédé de préparation d'anticorps monoclonaux** dirigés contre un ou plusieurs peptide(s) ou polypeptide(s) antigénique(s) exprimé(s) à la surface de vésicules membranaires, comprenant les étapes suivantes :
a) la fusion, avec des cellules de myélome, de cellules spléniques préalablement obtenues chez un hôte humain ou non humain auquel on a administré des vésicules membranaires selon l'une quelconque des revendications 14 à 34 ou comportant un polypeptide selon la revendication 8, une composition immunogène selon l'une quelconque des revendications 10 à 12 et 35 à 37, un polynucléotide selon la revendication 9 ou d'un polynucléotide codant pour un polypeptide chimérique tel que défini à l'une quelconque des revendications 14 à 34, le cas échéant en association avec un adjuvant et le cas échéant par administration répétée;
b) la culture et la sélection des hybridomes dans des conditions permettant la production d'anticorps;
c) la récupération des anticorps monoclonaux dirigés contre les peptide(s) ou polypeptide(s) antigénique(s) d'intérêt.

44. **Procédé de préparation d'anticorps monoclonaux** dirigés contre un ou plusieurs peptide(s) ou polypeptide(s) antigénique(s) exprimé(s) à la surface de vésicules membranaires, comprenant les étapes suivantes :
a) l'immortalisation de cellules productrices d'anticorps à partir de cellules hématopoïétiques préalablement obtenues chez un hôte humain ou non humain auquel on a administré des vésicules membranaires selon l'une quelconque des revendications 14 à 34 ou comportant un polypeptide selon la revendication 8, une composition immunogène selon l'une quelconque des revendications 10 à 12 et 35 à 37, un polynucléotide selon la revendication 9 ou un polynucleotide codant pour un polypeptide chimérique tel que défini à l'une quelconque des revendications 14 à 34, le cas échéant en association avec un adjuvant et le cas échéant par administration répétée;
b) la culture et la sélection des cellules immortalisées dans des conditions permettant la production d'anticorps;
c) la récupération des anticorps monoclonaux dirigés contre les peptide(s) ou polypeptide(s) antigénique(s) d'intérêt.

45. **Procédé de criblage *in vitro*** de molécules interagissant avec un peptide ou un polypeptide d'intérêt ou avec un fragment dudit peptide ou un polypeptide d'intérêt, ledit procédé comprenant :
a) la mise en contact de vésicules membranaires selon la revendication 15 ou 16 avec une ou plusieurs molécules susceptibles d'interagir avec ledit peptide ou polypeptide d'intérêt ;
b) la détection d'une éventuelle interaction entre ledit peptide ou polypeptide d'intérêt ou un fragment dudit peptide ou polypeptide d'intérêt et ladite ou lesdites molécules.

46. **Utilisation d'une vésicule membranaire** selon la revendication 15 ou 16 pour produire *in vitro* des anticorps monoclonaux, vaccinants ou non vaccinants, dirigés contre le peptide ou polypeptide d'intérêt ou un fragment dudit peptide ou polypeptide d'intérêt.

## Patentansprüche

1. **Polypeptid,** das die Sekretion eines interessierenden Peptids oder Polypeptids, dem es zugeordnet ist, in Verbindung mit Membranvesikeln, insbesondere mit Exosomen erlaubt, wenn es in einer eukaryotischen Zelle exprimiert wird,
**dadurch gekennzeichnet, dass** es ein mutiertes Derivat der zytoplasmatischen Domäne (CD) des Transmembranproteins (TM) des Rinderleukämievirus (BLV) mit Sequenz SEQ ID NO: 6 umfasst oder daraus besteht, wobei das mutierte Derivat
a) durch Substitution, Deletion und/oder Insertion eines oder mehrerer Reste in der Sequenz der zytoplasmatischen Domäne (CD) des Transmembranproteins (TM) des Rinderleukämievirus (BLV) mit Sequenz SEQ ID NO: 6 definiert ist;
b) wenigstens ein PxxP-Motiv und wenigstens ein YxxL-Motiv aufweist, wobei P einen Prolin-Rest, Y einen Tyrosin-Rest, x einen beliebigen Rest und L einen Leucin-Rest darstellt;
c) die Fähigkeit der CD-Domäne des TM-Proteins des BLV bewahrt, das interessierende Peptid oder Polypeptid, dem es zugeordnet ist, zu Membranvesikeln zu adressieren;
d) eine Sequenz umfasst, die zu wenigstens 60 % mit der vollständigen Sequenz SEQ ID NO: 8 identisch ist; und
e) eine Deletion oder eine Substitution durch einen Alanin-Rest des Cystein-Rests aus der in der Sequenz SEQ ID NO: 6 enthaltenen Sequenz PCP erfahren hat.

2. Polypeptid nach Anspruch 1,
bei dem das mutierte Derivat der CD-Domäne die aus der Sequenz PCP deletierte Sequenz SEQ ID NO: 8 umfasst oder daraus besteht.

3. Polypeptid nach Anspruch 1 oder 2,
bei dem das mutierte Derivat der CD-Domäne die Sequenz SEQ ID NO: 10 oder SEQ ID NO: 12 umfasst oder daraus besteht.

4. Polypeptid nach einem der Ansprüche 1 bis 3,
bei dem die Sequenz des mutierten Derivats der CD-Domäne von 30 bis 70 Resten gebildet ist.

5. Polypeptid nach einem der Ansprüche 1 bis 4,
bei dem das mutierte Derivat der CD-Domäne keine Sequenz KCLTSRLLKLLRQ enthält.

6. Polypeptid nach einem der Ansprüche 1 bis 5,
bei dem die Sequenz des mutierten Derivats der CD-Domäne zu wenigstens 70 %, wenigstens 80 %, wenigstens 90 % oder wenigstens 95 % mit der Sequenz SEQ ID NO: 8 identisch ist.

7. Polypeptid nach einem der Ansprüche 1 bis 6,
**dadurch gekennzeichnet, dass** das mutierte Derivat zusätzlich zur Deletion des Cystein-Rests aus der Sequenz PCP, die in der Sequenz SEQ ID NO: 6 enthalten ist, eine Deletion der gesamten Sequenz PCP erfahren hat.

8. Polypeptid nach einem der Ansprüche 1 bis 7,
das ferner das interessierende Peptid oder Polypeptid und eine Membrandomäne umfasst, die die Fähigkeit zur Verankerung in der Lipid-Doppelschicht einer Zellmembran besitzt.

9. **Polynukleotid,** für ein Polypeptid nach einem der Ansprüche 1 bis 8 kodie-rend.

10. **Immunogene Zusammensetzung,** die ein Polynukleotid nach Anspruch 9 und pharmazeutisch verträglich einen Träger, ein Verdünnungsmittel oder einen Hilfsstoff aufweist.

11. Immunogene Zusammensetzung nach Anspruch 10, zur Verwendung als Medikament.

12. Immunogene Zusammensetzung nach Anspruch 10 oder 11, zur Verwendung bei der Prophylaxe und/oder Behandlung einer bakteriellen, viralen, parasitären Infektion oder eines Tumors.

13. **Membranvesikel,** ein Polypeptid nach einem der Anspräche 1 bis 8 umfassend.

14. **Membranvesikel,** ein chimäres Polypeptid umfassend, das die folgenden Domänen umfasst oder daraus besteht:
(i) ein interessierendes Peptid oder Polypeptid;
(ii) eine Membrandomäne mit der Fähigkeit zur Verankerung in der Lipid-Doppelschicht einer Zellmembran;
(iii) die zytoplasmatische Domäne (CD) des Membranproteins (TM) des Rinderleukämievirus (BLV) oder eines mutierten Derivats dieser CD-Domäne,
wobei das mutierte Derivat eine Fähigkeit zur Adressierung des chimären Polypeptids zu Membranvesikeln von eukaryotischen Zellen und/oder zu einem oder mehreren bei der Bildung dieser Membranvesikeln beteiligten Zellkompartimenten besitzt, und
wobei die Sequenz des mutierten Derivats
- durch die Substitution, die Deletion und/oder die Insertion eines oder mehrerer Reste in der Sequenz dieser CD-Domäne definiert ist, und
- wenigstens zu 60 % mit der Sequenz SEQ ID NO: 8 identisch ist, vorausgesetzt die Sequenz des mutierten Derivats weist wenigstens ein PxxP-Motiv und wenigstens ein YxxL-Motiv auf, wobei P einen Prolin-Rest, Y einen Tyrosin-Rest, x einen beliebigen Rest und L einen Leucin-Rest darstellt,
und vorausgesetzt die Domäne (ii) umfasst nicht die Membrandomäne des TM-Proteins des BLV mit Sequenz SEQ ID NO: 4 und besteht nicht aus dieser, wenn die Domäne (iii) die CD-Domäne des TM-Proteins des BLV mit Sequenz SEQ ID NO: 6 umfasst oder aus dieser besteht.

15. Membranvesikel nach Anspruch 14,
**dadurch gekennzeichnet, dass** das interessierende Peptid oder Polypeptid ganz oder teilweise an der Außenseite des Membranvesikels exponiert ist.

16. Membranvesikel nach Anspruch 14,
**dadurch gekennzeichnet, dass** es neben dem chimären Polypeptid zudem ein Fragment des interessierenden Peptids oder Polypeptids umfasst, das ein oder mehrere Epitope des interessierenden Peptids oder Polypeptids umfasst oder daraus besteht, wobei das Fragment mithilfe der Membrandomäne eines Moleküls des Haupt-Histokompatiblitäts-Komplexes der Klasse I oder Klasse II, dem es zugeordnet ist, in der Membran des Membranvesikels verankert ist.

17. Membranvesikel nach einem der Ansprüche 14 bis 16,
**dadurch gekennzeichnet, dass** die Domänen (i) bis (iii) nacheinander in der folgenden Reihenfolge vom N-Terminus zum C-Terminus in dem Polypeptid positioniert sind: interessierendes Peptid oder Poypeptid - Membrandomäne - CD-Domäne oder mutiertes Derivat der CD-Domäne.

18. Membranvesikel nach einem der Ansprüche 14 bis 17,
**dadurch gekennzeichnet, dass** das chimäre Polypeptid ferner wenigstens ein Bindungsmolekül umfasst, das zwei aufeinanderfolgende Domänen verbindet.

19. Membranvesikel nach Anspruch 18,
**dadurch gekennzeichnet, dass** das Bindungsmolekül ein Peptid oder Polypeptid ist.

20. Membranvesikel nach Anspruch 18 oder 19,
**dadurch gekennzeichnet, dass** die für das Bindungsmolekül codierende Nukleotidsequenz eine Restriktionsstelle umfasst oder daraus besteht.

21. Membranvesikel nach einem der Ansprüche 14 bis 120,
**dadurch gekennzeichnet, dass** die Sequenz des mutierten Derivats der CD-Domäne keine Sequenz KCLTSRLLKLLRQ enthält und/oder keine Sequenz PCP enthält.

22. Membranvesikel nach einem der Ansprüche 14 bis 21,
**dadurch gekennzeichnet, dass** die Sequenz des mutierten Derivats der CD-Domäne zu wenigstens 70 % oder wenigstens 80 % oder wenigstens 90 % oder wenigstens 95 % mit Sequenz SEQ ID NO: 8 identisch ist.

23. Membranvesikel nach einem der Ansprüche 14 bis 22,
**dadurch gekennzeichnet, dass** die Sequenz der CD-Domäne oder die Sequenz des mutierten Derivats der CD-Domäne eine aus den folgenden Sequenzen gewählte Sequenz umfasst oder daraus besteht:
PxxPxxxxPxxPxxxYxxLxPxxPExYxxLxPxxPDYxxL;
PxxPxₙPxxPxₙYxxLxₙPxxPEXₙYxxLxₙPxxPDYxxL;
PxxPxxxxPxxPxxxYxxLxPxxPExYxLxPxxPDYxxLxxxx; und
PxxPxₙPxxPxₙYxxLxₙPxxPEXₙYxxLxₙPxxPDYxxLxxxx,
wobei x und Xₙ einen beliebigen Rest bzw. einen oder mehrere beliebige Reste darstellen.

24. Membranvesikel nach einem der Ansprüche 14 bis 23,
**dadurch gekennzeichnet, dass** die CD-Domäne die Sequenz SEQ ID NO: 6 ist.

25. Membranvesikel nach einem der Ansprüche 14 bis 24,
**dadurch gekennzeichnet, dass** das mutierte Derivat der CD-Domäne die Sequenz SEQ ID NO: 8, SEQ ID NO: 10 oder SEQ ID NO: 12 umfasst oder daraus besteht.

26. Membranvesikel nach einem der Ansprüche 14 bis 25,
**dadurch gekennzeichnet, dass** die Membrandomäne die Transmembrandomäne des TM-Proteins des BLV umfasst oder daraus besteht, und die Sequenz des mutierten Derivats der CD-Domäne nicht die Sequenz KCLTSRLLKLLRQ und/oder die Sequenz PCP enthält.

27. Membranvesikel nach einem der Ansprüche 14 bis 26,
**dadurch gekennzeichnet, dass** die Domäne (iii)
- die Sequenz SEQ ID NO: 8 oder
- eine Sequenz ist, die
- wenigstens zu 60 % mit der Sequenz SEQ ID NO: 8 identische ist,
- keine Sequenz KCLTSRLLKLLRQ enthält und die
- gebildet ist von
i. der Sequenz, die in der Sequenz SEQ ID NO: 8 vom PSAP-Motiv zum Yxxl-Motiv verläuft, das ihm im C-terminalen Bereich folgt, oder
ii. der Sequenz i. modifiziert durch Substitution oder Deletion einer oder mehrerer Aminosäuren zwischen den beiden Motiven.

28. Membranvesikel nach einem der Ansprüche 14 bis 27,
**dadurch gekennzeichnet, dass** das interessierende Peptid oder Polypeptid von einem pathogenen Organismus, einem Virus, einem Bakterium, einem Parasiten, einem Krankheitserreger, einem Tumor-Antigen, einem Cytoplasma-Antigen, einem Ligand-rezeptor, einem Rezeptor mit multiplen Membrandomänen, einem Rezeptor mit sieben Transmembrandomänen, einem Cytokin-Rezeptor, einem Rezeptorligand, einem Cytokin oder einem Fragment von diesen stammt.

29. Membranvesikel nach einem der Ansprüche 14 bis 28,
**dadurch gekennzeichnet, dass** das interessierende Peptid oder Polypeptid gewählt ist aus der Ektodomäne des Hemagglutinins (HA) eines Grippevirus, der Ektodomäne des HA-Proteins des Virus der Vogelgrippe H5N1, einem Fragment, das einen oder mehrere Epitopen dieser Ektodomäne des HA umfasst oder daraus besteht.

30. Membranvesikel nach einem der Ansprüche 14 bis 29,
**dadurch gekennzeichnet, dass** das chimäre Polypeptid ferner ein Import-Signalpeptid in das endoplasmatische Retikulum umfasst.

31. Membranvesikel nach einem der Ansprüche 14 bis 30,
**dadurch gekennzeichnet, dass** die eukaryotische Zelle eine HEK293-Zelle, ein T-Lymphozyt, ein B-Lymphozyt, ein Mastozyt, eine dendritische Zelle, eine Langer-hans-Zelle ist.

32. Membranvesikel nach einem der Ansprüche 14 bis 31,
**dadurch gekennzeichnet, dass** die Sequenz des mutierten Derivats der CD-Domäne keine Sequenz PC und/oder Sequenz CP enthält.

33. Membranvesikel nach einem der Ansprüche 14 bis 32,
**dadurch gekennzeichnet, dass** die Sequenz des mutierten Derivats der CD-Domäne aus 30 bis 70 Resten besteht.

34. Membranvesikel nach einem der Ansprüche 14 bis 33,
**dadurch gekennzeichnet, dass** es ein Exosom ist.

35. **Immunogene Zusammensetzung,** deren Wirkstoff ein oder mehrere Membranvesikel nach einem der Ansprüche 14 bis 34 oder ein für ein chimäres Polypeptid codierendes Polynukleotid wie in einem der Ansprüche 14 bis 34 definiert und pharmazeutisch verträglich einen Träger, ein Verdünnungsmittel oder einen Hilfsstoff umfasst.

36. Immunogene Zusammensetzung nach Anspruch 35, zur Verwendung als Medikament.

37. Immunogene Zusammensetzung nach Anspruch 35 oder 36, zur Verwendung bei der Prophylaxe und/oder Behandlung einer bakteriellen, viralen, parasitären Infektion oder eines Tumors.

38. **Exosome produzierende rekombinante Zelle,**
**dadurch gekennzeichnet, dass** sie mit einem oder mehreren Polynukleotiden nach Anspruch 9 oder mit einem oder mehreren für ein chimäres Polypeptid codierenden Polynukleotiden wie in einem der Ansprüche 14 bis 34 definiert, rekombiniert ist, oder dass sie ein Membranvesikel nach einem der Ansprüche 13 bis 34 absorbiert hat.

39. In-vitro**-Verwendung** eins Polypeptids nach einem der Ansprüche 1 bis 8 oder eines chimären Polypeptids wie in einem der Ansprüche 14 bis 34 definiert oder eines für ein solches Polypeptid codierendes Polynukleotids zur Adressierung eines interessierenden Peptids oder Polypeptids zu Membranvesikeln eukaryotischer Zellen, die innere Sekretionsvesikeln umfassen und die kultivierbar, zur Exotzyose fähig und genetisch modifizierbar sind, und/oder zu dem bzw. den bei der Bildung dieser Membranvesikeln beteiligten Zellkompartimenten, und zum Ermöglichen der Sekretion des interessierenden Peptids oder Polypeptids in Verbindung mit diesen Membranvesikeln durch die eukaryotischen Zellen.

40. **Verwendung eines oder mehrerer Membranvesikel** nach Anspruch 15 oder 16, zum in-vitro-Nachweis von spezifischen Partnern, die zur Interaktion mit dem interessierenden Peptid oder Polypeptid oder mit einem Fragment des Peptids oder Polypeptids fähig sind.

41. **Verfahren zur in-vitro-Produktion von Membranvesikeln,** umfassend ein interessierendes Peptid oder Polypeptid, wobei das Verfahren die folgenden Schritte umfasst:
a) Einschleusen eines oder mehrerer Polynukleotide nach Anspruch 9, das bzw. die für ein das interessierende Peptid oder Polypeptid umfassendes Polypeptid codiert bzw. codieren, in eine Exosome produzierende Zelle, oder Inkontaktbringen einer Exosome produzierenden Zelle mit einem oder mehreren Membranvesikeln nach einem der Ansprüche 13 bis 34, das bzw. die ein das interessierende Peptid oder Polypeptid enthaltendes Polypeptid umfassen;
b) Kultivierung der Exosome produzierenden Zelle;
c) Gewinnung von Membranvesikeln, die von der Exosome produzierenden Zelle produziert wurden.

42. **Verfahren zur Herstellung eines polyklonalen Serums,** das gegen ein oder mehrere interessierende Antigenpeptide oder -polypeptide gerichtet ist, die an der Oberfläche von Membranvesikeln exprimiert sind, das die folgenden Schritte umfasst:
a) Applikation, gegebenenfalls mehrmalig, in ein nicht humanes Tier von Membranvesikeln nach einem der Ansprüche 14 bis 34 oder ein Polypeptid nach Anspruch 8 umfassend, einer immunogenen Zusammensetzung nach einem der Ansprüche 10 bis 12 und 35 bis 37, eines Polynukleotids nach Anspruch 9 oder eines für ein chimäres Polypeptid codierendes Polynukleotids wie in einem der Ansprüche 14 bis 34 definiert, in oder ohne Verbindung eins Adjuvans; und
b) Gewinnung der gebildeten Antikörper, welche das oder die interessierenden Antigenpeptide oder -polypeptide zu erkennen vermögen.

43. **Verfahren zur Herstellung von monoklonalen Antikörpern,** die gegen ein oder mehrere Antigenpeptide oder -polypeptide gerichtet sind, die an der Oberfläche von Membranvesikeln exprimiert sind, das die folgenden Schritte umfasst:
a) Fusion mit Myelomzellen von Splenozyten, die von einem humanen oder nicht humanen Wirt gewonnen wurden, dem Membranvesikeln nach einem der Ansprüche 14 bis 34 oder ein Polypeptid nach Anspruch 8 umfassend, eine immunogene Zusammensetzung nach einem der Ansprüche 10 bis 12 und 35 bis 37, ein Polynukleotid nach Anspruch 9 oder ein für ein chimäres Polypeptid codierendes Polynukleotid wie in einem der Ansprüche 14 bis 34 definiert appliziert wurde, gegebenenfalls in Verbindung mit einem Adjuvans und gegebenenfalls mit mehrmaliger Applizierung;
b) Kultivierung und Selektion der Hybridomzellen unter Bedingungen, die die Antikörperherstellung erlauben;
c) Gewinnung der gegen das interessierende bzw. die interessierenden Antigenpeptide oder -polypeptide gerichteten monoklonalen Antikörper.

44. **Verfahren zur Herstellung von monoklonalen Antikörpern,** die gegen ein oder mehrere Antigenpeptide oder -polypeptide gerichtet sind, die an der Oberfläche von Membranvesikeln exprimiert sind, das die folgenden Schritte umfasst:
a) Immortalisierung von Antikörpern produzierenden Zellen aus hämatopoetischen Zellen, die zuvor bei einem humanen oder nicht humanen Wirt gewonnen wurden, dem Membranvesikeln nach einem der Ansprüche 14 bis 34 oder ein Polypeptid nach Anspruch 8 umfassend, eine immunogene Zusammensetzung nach einem der Ansprüche 10 bis 12 und 35 bis 37, ein Polynukleotid nach Anspruch 9 oder ein für ein chimäres Polpeptid codierendes Polynukleotid wie in einem der Ansprüche 14 bis 34 definiert appliziert wurde, gegebenenfalls in Verbindung mit einem Adjuvans und gegebenenfalls mit mehrmaliger Applizierung;
b) Kultivierung und Selektion der immortalisierten Zellen unter Bedingungen, die die Antikörperherstellung erlauben;
c) Gewinnung der gegen das interessierende bzw. die interessierenden Antigenpeptide oder -polypeptide gerichteten monoklonalen Antikörper.

45. **In-vitro-Screening-Verfahren** von Molekülen, die mit einem interessierenden Peptid oder Polypeptid oder mit einem Fragment des interessierenden Peptids oder Polypeptids interagieren, wobei das Verfahren umfasst:
a) Inkontaktbringung von Membranvesikeln nach Anspruch 15 oder 16 mit einem oder mehreren Molekülen, die zur Interaktion mit dem interessierenden Peptid oder Polypeptid fähig sind;
b) Nachweis einer möglichen Interaktion zwischen dem interessierenden Peptid oder Polypeptid oder einem Fragment des interessierenden Peptids oder Polypeptids und dem oder den Molekülen.

46. **Verwendung eines Membanvesikels** nach Anspruch 15 oder 16 zur in-vitro-Produktion von monoklonalen Antikörpern zur Vakzinierung oder nicht zur Vakzinierung, die gegen interessierende Peptide oder Polypeptide oder ein Fragment des Peptids oder Polypeptids gerichtet sind.

## Claims

1. **Polypeptide** allowing the secretion of a peptide or polypeptide of interest with which it is associated, in association with membrane vesicles, in particular with exosomes, when it is expressed in a eukaryotic cell, **characterized in that** it comprises or consists of a mutated derivative of the cytoplasmic domain (CD) of the transmembrane protein (TM) of bovine leukaemia virus (BLV) with sequence SEQ ID NO: 6, in which said mutated derivative:
a) is defined by substitution, deletion and/or insertion of one or more residue(s) in the sequence for the cytoplasmic domain (CD) of the transmembrane protein (TM) of bovine leukaemia virus (BLV) with sequence SEQ ID NO: 6;
b) comprises at least one motif PxxP and at least one motif YxxL, in which P represents a proline residue, Y represents a tyrosine residue, x represents any residue and L represents a leucine residue;
c) conserves the capacity of the CD domain of the TM protein of BLV to address the peptide or polypeptide of interest with which it is associated to membrane vesicles;
d) comprises a sequence which has at least 60% identity with the complete sequence SEQ ID NO: 8; and
e) has undergone a deletion or a substitution with an alanine residue, of the cysteine residue of the sequence PCP contained in the sequence SEQ ID NO: 6.

2. The polypeptide as claimed in claim 1, in which said mutated derivative of the CD domain comprises or consists of the sequence SEQ ID NO: 8 deleted from the sequence PCP.

3. The polypeptide as claimed in claim 1 or claim 2, in which said mutated derivative of the CD domain comprises or consists of the sequence SEQ ID NO: 10 or SEQ ID NO: 12.

4. The polypeptide as claimed in any one of claims 1 to 3, in which the sequence of said mutated derivative of the CD domain is constituted by 30 to 70 residues.

5. The polypeptide as claimed in any one of claims 1 to 4, in which said mutated derivative of the CD domain is free of the sequence KCLTSRLLKLLRQ.

6. The polypeptide as claimed in any one of claims 1 to 5, in which said sequence of said mutated derivative of the CD domain has at least 70%, at least 80%, at least 90% or at least 95% identity with the sequence SEQ ID NO: 8.

7. The polypeptide as claimed in any one of claims 1 to 6, **characterized in that** in addition to said deletion of the cysteine residue of the PCP sequence contained in the sequence SEQ ID NO: 6, said mutated derivative has undergone a deletion of the whole of said sequence PCP.

8. The polypeptide as claimed in any one of claims 1 to 7, which further comprises said peptide or polypeptide of interest and a membrane domain having the capacity to become anchored in the lipid bilayer of a cell membrane.

9. A polynucleotide coding for a polypeptide as claimed in any one of claims 1 to 8.

10. An immunogenic composition which comprises a polynucleotide as claimed in claim 9 and a pharmaceutically acceptable support, diluent or vehicle.

11. The immunogenic composition as claimed in claim 10, for use as a drug.

12. The immunogenic composition as claimed in claim 10 or claim 11, for use in prophylaxis and/or treatment of a bacterial, viral or parasitic infection or a tumour.

13. A membrane vesicle comprising a polypeptide as claimed in any one of claims 1 to 8.

14. A membrane vesicle comprising a chimeric polypeptide which comprises or consists of the following domains:
(i) a peptide or polypeptide of interest;
(ii) a membrane domain having the capacity to become anchored in the lipid bilayer of a cell membrane; and
(iii) the cytoplasmic domain (CD) of the membrane protein (TM) of bovine leukaemia virus (BLV) or a mutated derivative of this CD domain;
said mutated derivative having the capacity to address said chimeric polypeptide to the membrane vesicles of eukaryotic cells and/or to the cell compartment(s) involved in the formation of said membrane vesicles; and
the sequence of said mutated derivative:
• being defined by substitution, deletion and/or insertion of one or more residue(s) in the sequence for said CD domain; and
• having at least 60% identity with the sequence SEQ ID NO: 8, provided that the sequence for said mutated derivative comprises at least one motif PxxP and at least one motif YxxL, in which P represents a proline residue, Y represents a tyrosine residue, x represents any residue and L represents a leucine residue;
and provided that, when said domain (iii) comprises or consists of the CD domain of the TM protein of BLV of SEQ ID NO: 6, said domain (ii) does not comprise or consist of the membrane domain of the TM protein of BLV which has the sequence SEQ ID NO: 4.

15. The membrane vesicle as claimed in claim 14, **characterized in that** said peptide or polypeptide of interest is partially or completely exposed on the outside of said membrane vesicle.

16. The membrane vesicle as claimed in claim 14, **characterized in that** in addition to said chimeric polypeptide, it further comprises a fragment of said peptide or polypeptide of interest comprising or consisting of one or more epitope(s) of said peptide or polypeptide of interest, said fragment being anchored in the membrane of said membrane vesicle via the membrane domain of a molecule of the major histocompatibility complex type I or type II with which it is associated.

17. The membrane vesicle as claimed in any one of claims 14 to 16, **characterized in that** said domains (i) to (iii) are positioned in succession in the following order from the N-terminal end to the C-terminal end in the polypeptide: peptide or polypeptide of interest - membrane domain - CD domain or mutated derivative of CD domain.

18. The membrane vesicle as claimed in any one of claims 14 to 17, **characterized in that** said chimeric polypeptide further comprises at least one linker linking two successive domains.

19. The membrane vesicle as claimed in claim 18, **characterized in that** said linker is a peptide or polypeptide.

20. The membrane vesicle as claimed in claim 18 or claim 19, **characterized in that** the nucleotide sequence coding for said linker comprises or consists of a restriction site.

21. The membrane vesicle as claimed in any one of claims 14 to 20, **characterized in that** the sequence for said mutated derivative of the CD domain is free of the sequence KCLTSRLLKLLRQ and/or is free of the sequence PCP.

22. The membrane vesicle as claimed in any one of claims 14 to 21, **characterized in that** the sequence for said mutated derivative of the CD domain has at least 70% or at least 80% or at least 90% or at least 95% identity with the sequence SEQ ID NO: 8.

23. The membrane vesicle as claimed in any one of claims 14 to 22, **characterized in that** the sequence of said CD domain comprises or the sequence of said mutated derivative of the CD domain comprises or consists of a sequence selected from the following sequences:
PxxPxxxxPxxPxxxYxxLxPxxPExYxxLxPxxPDYxxL;
PxxPxₙPxxPxₙYxxLxₙPxxPEXₙYxxLxₙPxxPDYxxL;
PxxPxxxxPxxPxxxYxxLxPxxPExYxxLxPxxPDYxxLxxxx; and
PxxPxₙPxxPxₙYxxLxₙPxxPEXₙYxxLxₙPxxPDYxxLxxxx,
in which x and xₙ respectively represent any residue and any one or more residue(s).

24. The membrane vesicle as claimed in any one of claims 14 to 23, **characterized in that** said CD domain has the sequence SEQ ID NO: 6.

25. The membrane vesicle as claimed in any one of claims 14 to 24, **characterized in that** said mutated derivative of the CD domain comprises or consists of the sequence SEQ ID NO: 8, SEQ ID NO: 10 or SEQ ID NO: 12.

26. The membrane vesicle as claimed in any one of claims 14 to 25, **characterized in that** said membrane domain comprises or consists of the transmembrane domain of the TM protein of BLV and the sequence of said mutated derivative of the CD domain is free of the sequence KCLTSRLLKLLRQ and/or is free of the sequence PCP.

27. The membrane vesicle as claimed in any one of claims 14 to 26, **characterized in that** said domain (iii) is:
• the sequence SEQ ID NO: 8; or
• a sequence which:
o has at least 60% identity with the sequence SEQ ID NO: 8;
o is free of the sequence KCLTSRLLKLLRQ ; and which
o is constituted by:
i. the sequence which, in the sequence SEQ ID NO: 8, extends from the motif PSAP to the motif YxxL which follows it in the C-terminal portion; or
ii. the sequence i. modified by substitution or deletion of one or more amino acid(s) between said two motifs.

28. The membrane vesicle as claimed in any one of claims 14 to 27, **characterized in that** said peptide or polypeptide of interest derives from a pathogenic organism, a virus, a bacterium, a parasite, a pathogenic agent, a tumour antigen, a cytoplasmic antigen, a ligand receptor, a receptor with multiple membrane domains, a seven-transmembrane domain receptor, a cytokine receptor, a receptor ligand, a cytokine or a fragment thereof.

29. The membrane vesicle as claimed in any one of claims 14 to 28, **characterized in that** said peptide or polypeptide of interest is selected from the ectodomain of the haemagglutinin (HA) of an influenza virus, the ectodomain of the HA protein of H5N1 avian influenza virus, and a fragment comprising or consisting of one or more epitope(s) of said ectodomain of HA.

30. The membrane vesicle as claimed in any one of claims 14 to 29, **characterized in that** said chimeric polypeptide further comprises a signal peptide for importation into the endoplasmic reticulum.

31. The membrane vesicle as claimed in any one of claims 14 to 30, **characterized in that** said eukaryotic cell is a HEK293 cell, a T lymphocyte, a B lymphocyte, a mastocyte, a dendritic cell or a Langerhans cell.

32. The membrane vesicle as claimed in any one of claims 14 to 31, **characterized in that** the sequence of said mutated derivative of the CD domain is free of the sequence PC and/or the sequence CP.

33. The membrane vesicle as claimed in any one of claims 14 to 32, **characterized in that** the sequence of said mutated derivative of the CD domain is constituted by 30 to 70 residues.

34. The membrane vesicle as claimed in any one of claims 14 to 33, **characterized in that** it is an exosome.

35. An immunogenic composition wherein the active principle comprises one or more membrane vesicle(s) as claimed in any one of claims 14 to 34, or a polynucleotide coding for a chimeric polypeptide as defined in any one of claims 14 to 34 and a pharmaceutically acceptable support, diluent or vehicle.

36. The immunogenic composition as claimed in claim 35, for use as a drug.

37. The immunogenic composition as claimed in claim 35 or 36, for use in the prophylaxis and/or treatment of a bacterial, viral or parasitic infection, or of a tumour.

38. A recombinant exosome-producing cell, **characterized in that** it is recombined with one or more polynucleotide(s) as claimed in claim 9 or with one or more polynucleotide(s) coding for a chimeric polypeptide as defined in any one of claims 14 to 34, or **in that** it has absorbed a membrane vesicle as claimed in any one of claims 13 to 34.

39. *In vitro* **use** of a polypeptide as claimed in any one of claims 1 to 8 or of a chimeric polypeptide as defined in any one of claims 14 to 34, or of a polynucleotide coding for a polypeptide of this type, to address a peptide or polypeptide of interest to the membrane vesicles of eukaryotic cells which comprise internal secretion vesicles and which are capable of being cultured, are capable of exocytosis and capable of being genetically modified, and/or to the cell compartment(s) involved in the formation of said membrane vesicles, and to allow the secretion of said peptide or polypeptide of interest in association with said membrane vesicles by said eukaryotic cells.

40. **Use of one or more membrane vesicle(s)** as claimed in claim 15 or claim 16, for the *in vitro* detection of specific partners capable of interacting with said peptide or polypeptide of interest or with a fragment of said peptide or polypeptide.

41. **Method for the *in vitro* production of membrane vesicles** comprising a peptide or a polypeptide of interest, said method comprising the following steps:
a) introducing one or more polynucleotide(s) as claimed in claim 9, which code(s) for a polypeptide comprising said peptide or polypeptide of interest, into an exosome-producing cell, or bringing an exosome-producing cell into contact with one or more membrane vesicle(s) as claimed in any one of claims 13 to 34, which comprise(s) a polypeptide comprising said peptide or polypeptide of interest;
b) culturing said exosome-producing cell;
c) recovering the membrane vesicles produced by said exosome-producing cell.

42. **Method for preparing a polyclonal serum** directed against one or more antigenic peptide(s) or polypeptide(s) of interest expressed on the surface of membrane vesicles, comprising the following steps:
a) administration, repeating if necessary, to a non-human animal, of membrane vesicles as claimed in any one of claims 14 to 34 or comprising a polypeptide as claimed in claim 8, an immunogenic composition as claimed in any one of claims 10 to 12 and 35 to 37, of a polynucleotide as claimed in claim 9 or a polynucleotide coding for a chimeric polypeptide as defined in any one of claims 14 to 34, associated or otherwise with an adjuvant; and
b) recovering the antibodies formed which are capable of recognizing the antigenic peptide(s) or polypeptide(s) of interest.

43. **Method for preparing monoclonal antibodies** directed against one or more antigenic peptide(s) or polypeptide(s) expressed on the surface of membrane vesicles, comprising the following steps:
a) fusing, with myeloma cells, spleen cells obtained in a human or non-human host to which membrane vesicles as claimed in any one of claims 14 to 34 or comprising a polypeptide as claimed in claim 8, an immunogenic composition as claimed in any one of claims 10 to 12 and 35 to 37, a polynucleotide as claimed in claim 9 or a polynucleotide coding for a chimeric polypeptide as defined in any one of claims 14 to 34 have been administered, if necessary in association with an adjuvant and if necessary by repeated administration;
b) culturing and selecting hybridomas under conditions allowing the production of antibodies;
c) recovering monoclonal antibodies directed against the antigenic peptide(s) or polypeptide(s) of interest.

44. **Method for preparing monoclonal antibodies** directed against one or more antigenic peptide(s) or polypeptide(s) expressed on the surface of membrane vesicles, comprising the following steps:
a) immortalizing antibody-producing cells from haematopoïetic cells which have previously been obtained from a human or non-human host to which membrane vesicles as claimed in any one of claims 14 to 34 or comprising a polypeptide as claimed in claim 8, an immunogenic composition as claimed in any one of claims 10 to 12 and 35 to 37, a polynucleotide as claimed in claim 9 or a polynucleotide coding for a chimeric polypeptide as defined in any one of claims 14 to 34 have been administered, if necessary in association with an adjuvant and if necessary by repeated administration;
b) culturing and selecting immortalized cells under conditions allowing the production of antibodies;
c) recovering monoclonal antibodies directed against the antigenic peptide(s) or polypeptide(s) of interest.

45. ***In vitro* method for screening** molecules interacting with a peptide or a polypeptide of interest or with a fragment of said peptide or a polypeptide of interest, said method comprising:
a) bringing membrane vesicles as claimed in claim 15 or claim 16 into contact with one or more molecules susceptible of interacting with said peptide or polypeptide of interest;
b) detecting any interaction between said peptide or polypeptide of interest or a fragment of said peptide or polypeptide of interest and said molecules.

46. **Use of a membrane vesicle** as claimed in claim 15 or claim 16 for the *in vitro* production of monoclonal antibodies, vaccinating or non-vaccinating, directed against the peptide or polypeptide of interest or a fragment of said peptide or polypeptide of interest.
